(19) Europäisches Patentamt
European Patent Office
Office européen des brevets

(11) **EP 3 395 939 B1**

(12) **EUROPEAN PATENT SPECIFICATION**

(45) Date of publication and mention
of the grant of the patent:
**09.02.2022 Bulletin 2022/06**

(21) Application number: **16879034.3**

(22) Date of filing: **24.12.2016**

(51) International Patent Classification (IPC):
**C12M 3/00** *(2006.01)*      **C12M 1/28** *(2006.01)*
**C12M 1/12** *(2006.01)*      **C12M 1/00** *(2006.01)*
**C12M 1/32** *(2006.01)*

(52) Cooperative Patent Classification (CPC):
**C12M 23/04; C12M 23/12; C12M 23/38**

(86) International application number:
**PCT/JP2016/088597**

(87) International publication number:
**WO 2017/111148 (29.06.2017 Gazette 2017/26)**

(54) **CELL SORTING, CULTURE, AND GROWTH VESSEL; AND CELL SORTING, CULTURE, AND GROWTH METHOD**

ZELLSORTIERUNG, KULTUR UND WACHSTUMSGEFÄSS SOWIE ZELLSORTIERUNG, KULTUR UND WACHSTUMSVERFAHREN

CUVE DE TRI, DE CULTURE ET DE CROISSANCE CELLULAIRES ; ET PROCÉDÉ DE TRI, DE CULTURE ET DE CROISSANCE CELLULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.12.2015 JP 2015255511**

(43) Date of publication of application:
**31.10.2018 Bulletin 2018/44**

(73) Proprietors:
• **Nepa Gene Co., Ltd.**
**Ichikawa-shi, Chiba 272-0114 (JP)**
• **National Center for Geriatrics and Gerontology**
**Aichi 4748511 (JP)**
• **Minowa, Noboru**
**Edogawa-ku**
**Tokyo 133-0065 (JP)**

(72) Inventors:
• **SHIMAGAKI Masaaki**
**Tokyo 103-0023 (JP)**
• **SUZUKI Kosho**
**Ichikawa-shi**
**Chiba 272-0114 (JP)**
• **ATSUMI Yusuke**
**Ichikawa-shi**
**Chiba 272-0114 (JP)**
• **NAKASHIMA Misako**
**Obu-shi**
**Aichi 474-8511 (JP)**

(74) Representative: **Vossius & Partner**
**Patentanwälte Rechtsanwälte mbB**
**Siebertstrasse 3**
**81675 München (DE)**

(56) References cited:
**WO-A1-2012/133803      WO-A1-2015/121302**
**US-A1- 2012 100 110      US-A1- 2014 178 992**

• **HIROSHI HORIBE ET AL: "Isolation of a Stable Subpopulation of Mobilized Dental Pulp Stem Cells (MDPSCs) with High Proliferation, Migration, and Regeneration Potential Is Independent of Age", PLOS ONE, vol. 9, no. 5, 28 May 2014 (2014-05-28), page e98553, XP055603427, DOI: 10.1371/journal.pone.0098553**

- **MURAKAMI MASASHI ET AL: "The use of granulocyte-colony stimulating factor induced mobilization for isolation of dental pulp stem cells with high regenerative potential", BIOMATERIALS, ELSEVIER SCIENCE PUBLISHERS BV., BARKING, GB, vol. 34, no. 36, 27 August 2013 (2013-08-27), pages 9036-9047, XP028714757, ISSN: 0142-9612, DOI: 10.1016/J.BIOMATERIALS.2013.08.011**
- **AKON HIGUCHI ET AL: "A hybrid-membrane migration method to isolate high-purity adipose-derived stem cells from fat tissues", SCIENTIFIC REPORTS, vol. 5, no. 1, 13 May 2015 (2015-05-13), pages 1-11, XP055430261, DOI: 10.1038/srep10217**

**Description**

Technical Field

The present invention relates to a sealable vessel for cell isolation, culture and proliferation. In particular, it relates to a technique for cell isolation, culture and proliferation that can realize high-efficiency isolation of cells of interest from a tissue or a cell population and their high-efficiency proliferation and that can be stably performed with simple operation and at a low cost.

Background Art

The technique for isolation of cells of interest from a cell population such as a tissue or the like and their proliferation is crucial in the field of life science. Especially, techniques for efficiently performing the isolation and proliferation of stem cells are highly demanded in the medical field.

Flow cytometry, magnetic antibody beads and the like are used in conventional cell isolation methods. However, the methods utilizing flow cytometry or magnetic antibody beads have a problem in that it is difficult to carry them out at a low cost because of the instruments, equipment and principles involved. Moreover, it is pointed out that they have a fundamental safety problem in that they may not fulfill the requirements of the Ministerial Ordinance on Standards for Manufacturing Control and Quality Control for Drugs and Quasi-drugs (GMP).

For example, in the isolation of target stem cells using flow cytometry, SP cells (pulp CD31⁻ SP cells) are stained with DNA specific dyes and the fraction showing higher efflux of the dyes is collected. However, it is pointed out that the safety of the cells may be compromised in the method using flow cytometry.

In the magnetic bead-based method, the target cells are isolated using magnetic beads coated with antibodies recognizing CD34 or CD133 surface antigens of bone marrow cells. However, the isolation efficiency is low because the content of stem cells in the tissue to be subjected to cell isolation is not high. Moreover, it is admitted that this technique cannot be achieved at a low cost, because some kinds of antibody beads are made to order for each experiment.

As a technique for isolating stem cells without using flow cytometry or magnetic antibody beads, the research group of the present inventors conceived a technique utilizing chemotactic factors and a membrane separation culture device (Patent Document 1). The membrane separation culture device disclosed in Patent Document 1 can also be used as an upper structure of a commercially available product such as the Falcon Cell Culture Insert by being inserted thereto.

However, upon using the membrane separation culture device of Patent Document 1, a large number of cells are lost because they are exposed to stimuli or suffer damage when they are transferred to another dish for cell culture, and the cell quality and yield are greatly reduced especially in the isolation and proliferation of precious stem cells, which are highly sensitive to stimuli and whose pluripotency is easily lost. Moreover, since the isolation and proliferation processes are performed separately, skills are required for handling the cells, and the isolation efficiency strongly depends on the skills of the operator.

Furthermore, since the membrane separation culture device of Patent Document 1 is used in an open environment, there are such risks as infection with mycoplasmas or viruses and contamination from other cell sources. Accordingly, it is difficult to achieve a stable production efficiency or obtain consistent experimental results.

Citation List

Patent Documents

Patent document 1: WO 2012/133803

US 2014/178992 A1 relates to a membrane-separation-type culture device, a corresponding kit, a stem cell separation method and a separation membrane.

Horibe et al. (2014) Isolation of a Stable Subpopulation of Mobilized Dental Pulp Stem Cells (MDPSCs) with High Proliferation, Migration, and Regeneration Potential Is Independent of Age, PLoS ONE 9(5), relates to insights into the understanding of the influence of the age of MSCs on their cellular responses and regenerative potential are critical for stem cell therapy in the clinic. Murakami et al., The use of granulocyte-colony stimulating factor induced mobilization for isolation of dental pulp stem cells with high regenerative potential, Biomaterials, Volume 34, Issue 36, December 2013, Pages 9036-9047, relates to a method for isolation of DPSCs subsets based on their migratory response to optimized concentration of 100 ng/ml of granulocyte-colony stimulating factor (G-CSF). Higuchi et al., A hybrid-membrane migration method to isolate high-purity adipose-derived stem cells from fat tissues, Sci Rep 5, 10217 (2015) relates to a hybrid-membrane migration method that purifies hADSCs from a fat tissue solution. US 2012/100110 A1 relates to physiological methods for isolation of high purity cell populations. WO 2015/121302 A1 discloses dividable surfaces for cell culturing. WO 2012/133803 A1 discloses a membrane-separation-type culture device.

Summary of Invention

Technical Problem

The present invention was achieved in light of the aforementioned circumstances of the conventional techniques, and it is an object thereof to provide a technique for high-efficiency isolation of cells of interest from a tissue or a cell population and their high-efficiency proliferation that can be stably performed with simple operation and at a low cost.

Solution to Problem

As a result of intensive study conducted to solve the aforementioned problems, the inventors of the present invention have conceived a technique related to sealable vessel for cell isolation, culture and proliferation comprising a cell separation unit, a primary culture unit and a main vessel unit and have accomplished the present invention.

Specifically, the inventors have conceived a composition of members in which the cell separation unit is a structural body comprising a cell separation membrane having fine pores capable of isolating cells.

Also, the inventors have conceived a composition of members in which the primary culture unit is a container-like structural body that has a culture surface for primary culture on which cells that have passed through the cell separation membrane can be cultured and a releasing structure through which cells and a liquid that have been held inside the primary culture unit can be freely released toward a unit having a culture surface for a subsequent step.

Moreover, the inventors have conceived a composition of members in which the main vessel unit is a container-like structural body that has a culture surface for cell proliferation on which cells that have been released from a culture unit for a preceding step can be cultured, an area of the culture surface for cell proliferation being larger than that of a culture surface of the culture unit for the preceding step, the container-like structural body of the main vessel unit having a structure in which the cell separation unit and all of the culture unit/units for preceding step/steps can be stored.

The inventors of the present invention have produced a sealable vessel for cell isolation, culture and proliferation having the structures above. Using this vessel, the inventors have performed the cell isolation process for selectively isolating the target cells, the primary culture process, the cell detachment process and the cell proliferation process, and have found that the isolation and proliferation of target cells of interest can be performed inside the same vessel with an easy operation. These operations can be performed without requiring such handling steps as the transfer of cells to a different vessel for proliferation culture, and therefore, the risk of infection with mycoplasmas or viruses and contamination from other cell sources is extremely reduced.

The excellent functions of this vessel can only be achieved by the interaction of the special structures and features of the components of this vessel.

The present inventors have further conceived an idea of employing material with light transmitting properties for the members located at certain positions, to realize real-time observation of the progress in the cell culture process over time under a microscope or the like without opening the main vessel unit.

Also, the present inventors have conceived an idea that the transition from the cell isolation process to the primary culture process can be performed without opening the main vessel unit, as a structure through which a hollow tubular member can be inserted or taken out is provided at the main vessel unit as a member of the vessel.

Moreover, the present inventors have conceived an idea that the transition from the cell isolation process to the primary culture process can be performed without opening the main vessel unit, as the members of the vessel include a structure in which the position of the cell separation membrane of the cell separation unit can be adjusted in response to the operation of the main lid portion of the main vessel unit.

Furthermore, the present inventors have conceived an idea that the transition from the primary culture process to the cell proliferation process can be performed without opening the main vessel unit, as the members of the vessel include a structure with which the releasing structure provided at the primary culture unit can be adjusted to open in response to the operation from outside the main vessel unit.

The present inventors have found that the risk of infection and contamination can be further reduced by employing one or more of the aforementioned means for avoiding the opening of the main vessel unit.

The present inventors have further found that the cells can be easily detached from the culture surface and can be transferred even more efficiently, as a base material whose surface is modified with molecules which exhibit cell detachment properties in response to stimuli is employed for the culture surface of the culture unit in the sealable vessel for cell isolation, culture and proliferation having the aforementioned structures.

In addition, the present inventors have found that the number of target cells lost due to adhesion to the cell separation membrane can be greatly reduced and the yield of the target cells can be improved, as a base material whose surface is modified with hydrophilic polymers is employed for the cell separation membrane of the cell separation unit.

The invention is defined in the independent claim. Dependent claims describe preferred embodiments. The present invention specifically relates to aspects of the invention described below.

[1] A sealable vessel for cell isolation, culture and proliferation, the vessel being a container-like structural body in which isolation, culture and proliferation of target cells of interest can be performed,

wherein (A) the vessel includes a cell separation unit, a primary culture unit and a main vessel unit;
(B) the cell separation unit is a structural body including a cell separation membrane having fine pores capable of isolating cells by attracting or activating movement of the target cells by utilizing chemotactic factors;
(C) the primary culture unit is a container-like structural body that has a culture surface for primary culture on which cells that have passed through the cell separation membrane can be cultured and a releasing structure through which cells and a liquid that have been held inside the primary culture unit can be freely released toward a unit having a culture surface for a subsequent step, the culture surface for primary culture having a size of 10 to 400 cm$^2$; and
(D) the main vessel unit is a container-like structural body that has a culture surface for cell proliferation on which cells that have been released from a culture unit for a preceding step can be cultured, the culture surface for cell proliferation being a culture surface different from the culture surface for primary culture of the primary culture unit, an area of the culture surface for cell proliferation being larger than that of a culture surface of the culture unit for the preceding step, the container-like structural body of the main vessel unit having a structure in which the cell separation unit and all of the culture unit/units for preceding step/steps can be stored.

[2] The vessel for cell isolation, culture and proliferation according to aspect 1,
wherein (b-1) the cell separation unit recited in (B) is a container-like structural body;

(b-2) the cell separation unit includes the cell separation membrane having fine pores capable of isolating cells, the cell separation membrane being provided on at least a part of surfaces of the container-like structural body of the cell separation unit;
(c-1) the primary culture unit recited in (C) is a container-like structural body;
(c-2) the primary culture unit has a structure with which the cell separation unit can be disposed in a state in which, when a liquid is filled in the container-like structural body of the primary culture unit, a surface of the cell separation membrane and a surface of the liquid filled are in contact with each other;
(c-3) the primary culture unit has the culture surface for primary culture on which the cells that have passed through the cell separation membrane can be cultured, the culture surface for primary culture being provided on at least a part of surfaces of the container-like structural body of the primary culture unit;
(c-4) the primary culture unit has the releasing structure through which the cells and the liquid that have been held inside the container-like structural body of the primary culture unit can be freely released toward the unit having the culture surface for the subsequent step, the releasing structure being provided at a surface of the container-like structural body of the primary culture unit;
(d-1) the main vessel unit recited in (D) is a container-like structural body mainly composed of a main vessel and a main lid portion;
(d-2) the main vessel unit has the culture surface for cell proliferation on which the cells that have been released from the culture unit for the preceding step can be cultured, the area of the culture surface for cell proliferation being larger than that of the culture surface of the culture unit for the preceding step;
(d-3) the main vessel unit has a structure with which the cell separation unit and all of the culture unit/units for the preceding step/steps can be stored in the container-like structural body of the main vessel unit; and
(d-4) the main lid portion is a lid-like structural body with which the entire vessel can be sealed.

[3] The vessel for cell isolation, culture and proliferation according to aspect 1 or 2, wherein the cell separation unit is a unit for isolating the target cells of interest into the container-like structural body of the primary culture unit through the cell separation membrane.
[4] The vessel for cell isolation, culture and proliferation according to any one of aspects 1 to 3, wherein, when an area of the culture surface of the culture unit for the preceding step is taken as 1, a ratio of an area of the culture surface for cell proliferation is within a range of more than 1 to not more than 20.
[5] The vessel for cell isolation, culture and proliferation according to any one of aspects 1 to 4, wherein, in the vessel for cell isolation, culture and proliferation,

(E)(e-1) at least a part of all the culture surfaces included in the vessel for cell isolation, culture and proliferation and at least a part of vessel structures opposed to or substantially opposed to the culture surface for cell proliferation of the main vessel unit are made of light-transmitting material; and

(e-2) the culture surfaces inside the vessel can be observed through portions having light-transmitting properties without opening the main vessel unit.

[6] The vessel for cell isolation, culture and proliferation according to any one of aspects 1 to 5, wherein the primary culture unit is a container-like structural body composed of two or more members, and has a structure with which the releasing structure can be adjusted to open as the members of the container-like structural body of the primary culture unit are detached or their positions are changed.

[7] The vessel for cell isolation, culture and proliferation according to any one of aspects 1 to 6, wherein the primary culture unit has a structure with which the releasing structure can be adjusted to open in response to operation from outside the main vessel unit.

[8] The vessel for cell isolation, culture and proliferation according to any one of aspects 1 to 7, wherein the primary culture unit has a structure with which the releasing structure can be adjusted to open in response to operation of the main lid portion.

[9] The vessel for cell isolation, culture and proliferation according to any one of aspects 1 to 8, wherein, in the vessel for cell isolation, culture and proliferation,

(F)(f-1) a structure through which a hollow tubular member can be inserted into or taken out of the vessel without opening the main vessel unit is provided at a wall surface of the main vessel or the main lid portion of the main vessel unit;

(f-2) the structure is disposed at a position at which the liquid filled in the primary culture unit can be suctioned out through the hollow member inserted; and

(f-3) the structure is mainly composed of an elastic member which can secure airtightness both in a state in which the hollow tubular member is not inserted and in a state in which the hollow tubular member is inserted.

[10] The vessel for cell isolation, culture and proliferation according to any one of aspects 1 to 9, wherein the cell separation unit has a structure with which a position of the cell separation membrane can be adjusted in response to operation of the main lid portion.

[11] The vessel for cell isolation, culture and proliferation according to any one of aspects 1 to 10, wherein the main vessel unit is provided with a gas exchange port.

[12] The vessel for cell isolation, culture and proliferation according to any one of aspects 1 to 11, wherein the main vessel unit is provided with a medium exchange port through which a medium can be introduced into or discharged from the main vessel.

[13] The vessel for cell isolation, culture and proliferation according to any one of aspects 1 to 12, wherein the cell separation unit is a container-like structural body that is mainly composed of the cell separation membrane and a membrane holding structure, and a peripheral portion of a bottom surface of an opening portion of the membrane holding structure covered by the cell separation membrane has a shape having an upward inclination toward the opening portion.

[14] The vessel for cell isolation, culture and proliferation according to any one of aspects 1 to 13, including:

(G) one or more cell proliferation units with which a plurality of cell proliferation processes can be performed after a primary culture process;

wherein (g-1) the cell proliferation unit is a container-like structural body;

(g-2) the cell proliferation unit includes a culture surface for cell proliferation on which cells that have been released from a culture unit for a preceding step can be cultured, an area of the culture surface for cell proliferation being larger than that of a culture surface of the culture unit for the preceding step;

(g-3) the cell proliferation unit has a structure with which the cell separation unit and all of the culture unit/units for the preceding step/steps can be stacked or stored in the container-like structural body of the cell proliferation unit; and

(g-4) the cell proliferation unit has a releasing structure through which cells and a liquid that have been held inside the container-like structural body of the cell proliferation unit can be freely released toward a unit having the culture surface for the subsequent step, the releasing structure being provided at a surface of the container-like structural body of the cell proliferation unit.

[15] The vessel for cell isolation, culture and proliferation according to any one of aspects 1 to 14, wherein the fine pores formed in the cell separation membrane have an average size of 1 to 20 $\mu$m, and the cell separation membrane has a porosity of 5 to 50%.

[16] The vessel for cell isolation, culture and proliferation according to any one of aspects 1 to 15, wherein the fine pores formed in the cell separation membrane are open in a vertical direction and/or in a substantially vertical direction with respect to a membrane surface.

[17] The vessel for cell isolation, culture and proliferation according to any one of aspects 1 to 16, wherein a part or all of the fine pores formed in the cell separation membrane are arranged in a lattice pattern, a substantially lattice pattern, a staggered pattern, a substantially staggered pattern, a spiral pattern, a substantially spiral pattern, a concentric pattern, a substantially concentric pattern, or a combination of the aforementioned patterns.

[18] The vessel for cell isolation, culture and proliferation according to any one of aspects 1 to 17, wherein the cell separation membrane has a structure in which hydrophilic polymers are covalently bonded to a surface of a base material layer made of hydrophobic polymers.

[19] The vessel for cell isolation, culture and proliferation according to any one of aspects 1 to 18,

wherein the cell separation membrane has a structure in which one or more selected from the hydroxyl group, the carboxyl group and a compound structure including said functional groups are covalently bonded to a vicinity of the surface of the base material layer made of hydrophobic polymers, and/or
the cell separation membrane has an insoluble layer in the vicinity of the surface of the base material layer made of hydrophobic polymers, the insoluble layer being insoluble in a good solvent for the hydrophobic polymers.

[20] The vessel for cell isolation, culture and proliferation according to any one of aspects 1 to 19, wherein a surface of the culture surface for primary culture and/or a surface of the culture surface for cell proliferation are modified with molecules that exhibit cell adhesion properties during culture and exhibits cell detachment properties in response to a stimulus.

[21] The vessel for cell isolation, culture and proliferation according to aspect 20, wherein the molecules that exhibit cell detachment properties are temperature responsive polymer molecules having a phase transition temperature, or a lower critical solution temperature, of 15 to 35°C.

[22] The vessel for cell isolation, culture and proliferation according to any one of aspects 1 to 21,

wherein the culture surface for primary culture and/or the culture surface for cell proliferation have a structure in which one or more selected from the hydroxyl group, the carboxyl group, and a compound structure including said functional groups are covalently bonded to a vicinity of a surface of a base material layer made of hydrophobic polymers, and/or
the culture surface for primary culture and/or the culture surface for cell proliferation have an insoluble layer in the vicinity of the surface of the base material layer made of hydrophobic polymers, the insoluble layer being insoluble in a good solvent for the hydrophobic polymers.

[23] The vessel for cell isolation, culture and proliferation according to any one of aspects 1 to 22,

wherein the structural bodies constituting the vessel for cell isolation, culture and proliferation have a structure in which one or more selected from the hydroxyl group, the carboxyl group, and a compound structure including said functional groups are covalently bonded to a vicinity of a surface of a base material layer made of hydrophobic polymers, and/or
the structural bodies constituting the vessel for cell isolation, culture and proliferation have an insoluble layer provided in the vicinity of the surface of the base material layer made of hydrophobic polymers, the insoluble layer being insoluble in a good solvent for the hydrophobic polymers.

[24] The vessel for cell isolation, culture and proliferation according to any one of aspects 1 to 23, wherein the vessel for cell isolation, culture and proliferation is a vessel for actively isolating stem cells and performing their proliferation.

[25] A method for cell isolation, culture and proliferation, using the vessel for cell isolation, culture and proliferation according to any one of aspects 1 to 24.

[26] The method for cell isolation, culture and proliferation according to aspect 25, wherein the target cells of interest include stem cells.

Advantageous Effects of the Invention

The present invention relates to a technique for high-efficiency isolation of cells of interest from a tissue or a cell population and their high-efficiency proliferation that can be stably performed with simple operation and at a low cost. For example, the present invention can provide a technique with which isolation, culture and proliferation of stem cells can be performed at high efficiency.

Brief Description of Drawings

FIG. 1 is an exploded perspective view illustrating the vessel for cell isolation, culture and proliferation produced in Example 1.

FIG. 2 is a perspective view illustrating the entire vessel after the assembly of all the units of the vessel for cell isolation, culture and proliferation produced in Example 1.

FIG. 3 is a longitudinal cross-sectional view illustrating the vessel for cell isolation, culture and proliferation produced in Example 1 in a state in which the main lid portion is removed.

FIG. 4 is a perspective view of the vessel for cell isolation, culture and proliferation produced in Example 1 showing the outline of the operations performed after the cell isolation process in Example 2.

FIG. 5 is a perspective view of the vessel for cell isolation, culture and proliferation produced in Example 1 showing the outline of the operations performed after the primary culture process and in the cell detachment process in Example 2.

FIG. 6 is a perspective view of the vessel for cell isolation, culture and proliferation produced in Example 1 showing the outline of the operations performed before the cell proliferation process in Example 2.

FIG. 7 is a schematic view of one mode of the cell isolation process according to the present invention.

FIG. 8 is a schematic view of one mode of the primary culture process according to the present invention.

FIG. 9 is a schematic view of one mode of the cell detachment process according to the present invention.

FIG. 10 is a schematic view of one mode of the cell proliferation process according to the present invention.

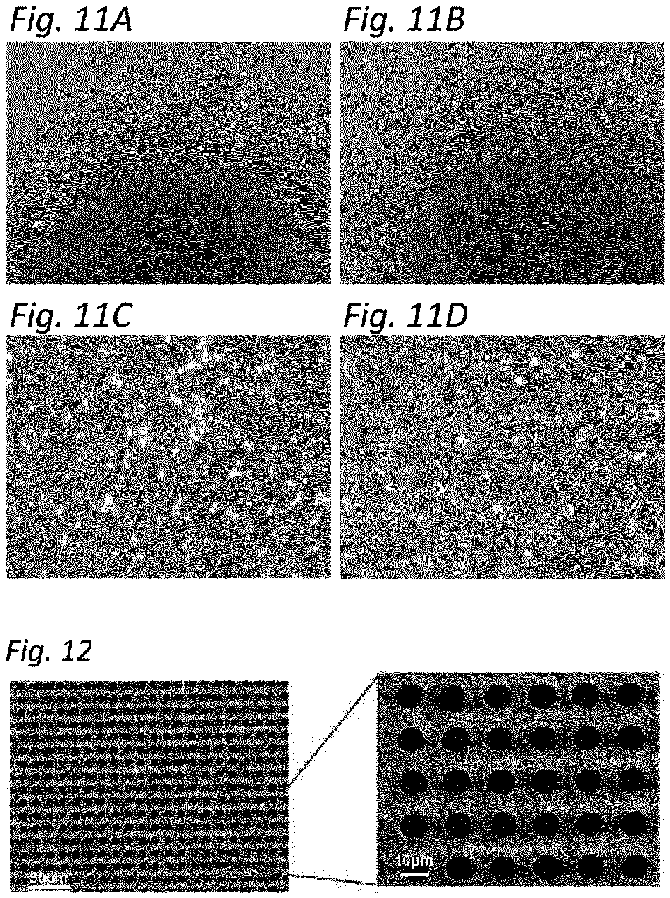

FIG. 11 shows microscopic images of cells in each process of cell separation, culture and proliferation in Example 2. FIG. 11A is a photographic image of the cells on the culture surface for primary culture immediately after the cell isolation process. FIG. 11B is a photographic image of the cells that have been cultured on the culture surface for primary culture after the primary culture process. FIG. 11C is a photographic image of the cells suspended on the culture surface for primary culture after the cell detachment process. FIG. 11D is a photographic image of the cells that have proliferated on the culture surface for cell proliferation after the cell proliferation process.

FIG. 12 is a photographic image of the microstructure of the surface of the cell separation membrane of the present invention taken under a scanning electron microscope (SEM) in the evaluation of the structure of the cell separation membrane in Example 3 (1). The scale bar at the left bottom of the photograph on the left indicates 50 $\mu$m. The scale bar at the left bottom of the photograph on the right indicates 10 $\mu$m.

FIG. 13 is a photographic image of the microstructure of the surface of a PE membrane produced by a conventional method taken under a scanning electron microscope (SEM) in the evaluation of the structure of the cell separation membrane in Example 3 (1). The scale bar at the bottom of the photograph indicates 200 $\mu$m.

FIG. 14 is a graph showing the ratios of the cell non-adhesive area of the cell separation membranes after cell culture in the evaluation of the function of the cell separation membrane in Example 3 (2).

FIG. 15 shows microscopic images of Giemsa-stained surfaces of cell separation membranes after cell culture taken in the evaluation of the function of the cell separation membrane in Example 3 (2). The scale bar at the right bottom of each photograph indicates 200 $\mu$m.

FIG. 16 is a graph showing the cell detachment rates before and after the low-temperature treatment in the assessment of the cell detachment activity of the culture surface for primary culture in Example 4.

FIG. 17 shows microscopic images of the culture surfaces for primary culture taken before and after the low-temperature treatment in the evaluation of the function of the cell separation membrane in Example 4.

FIG. 18 is a schematic view showing one mode of the structure of the vessel for cell isolation, culture and proliferation according to the present invention with which the medium can be suctioned from or injected into the primary culture vessel.

FIG. 19 is a perspective view illustrating the entire vessel after the assembly of all the units of the vessel for cell isolation, culture and proliferation produced in Example 6.

FIG. 20 is a diagram illustrating the structure of the rotation adjustment mechanism provided in the vessel for cell isolation, culture and proliferation produced in Example 6. FIG. 20A is a top perspective view of the ring-shaped structure (click-lock ring) provided with adjustment portions. FIG. 20B is a schematic view showing a state in which the clicking protrusion of the main lid portion is fitted to the adjustment portion on the ring-shaped structure.

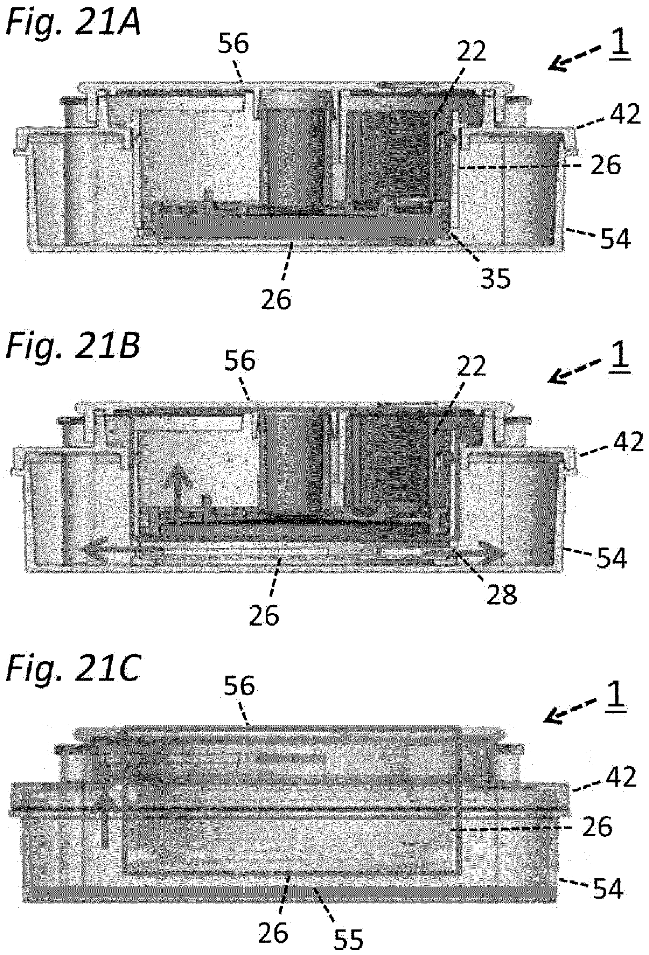

FIG. 21 is a schematic view showing the structures of the vessel for cell isolation, culture and proliferation produced in Example 6 after each process of cell isolation, culture and proliferation. FIG. 21A is a diagram illustrating the structure of the vessel at the time of performing the cell isolation process and the primary culture process. FIG. 21B is a diagram illustrating the structure of the vessel at the time of discharging the cell suspension into the main vessel after the primary culture and cell detachment processes. The upward arrow in the figure indicates the upward movement of the upper structural body of the primary culture vessel (and the member for blocking the releasing structure). The rightward and leftward arrows indicate the movement of the cell suspension from the primary culture vessel to the main vessel. FIG. 21C is a diagram illustrating the structure of the vessel at the time of performing the cell proliferation process. The upward arrow in the figure indicates the upward movement of the lower structural body of the primary culture vessel (the entire primary culture vessel).

FIG. 22 shows microscopic images taken at time intervals showing the stem cells cultured on the culture plate produced by performing plasma treatment on a base material in Example 8.

Description of Embodiments

Hereinafter, embodiments of the present invention will be described in detail but do not limit the scope of the present invention. The reference numerals in the following description refer to those used in the drawings.

[Terminology]

The term "the culture unit for the preceding step" refers to the culture unit used in the step prior to the current process. For example, in one mode, when the cell proliferation unit is not employed, the primary culture unit is the culture unit for the preceding step when viewed from the main vessel unit. In another mode, when the cell proliferation units are employed, the cell proliferation unit used in the prior step is regarded as the culture unit for the preceding step.

The term "the unit having a culture surface for the subsequent step" refers to the culture unit used in the next process. For example, in one mode, when the cell proliferation unit is not employed, the main vessel unit is regarded as the unit having the culture surface for the subsequent step when viewed from the primary culture unit. In another mode, when the cell proliferation units are employed, the cell proliferation unit used in the subsequent step is regarded as the unit having a culture surface for the subsequent step.

The expression of figures, shapes, arrangement, angles or the like modified with the word "substantially" as used herein indicates that some changes or modifications can be added to the figures or the like.

For example, when a word indicating an angle or a direction is modified with the word "substantially" in the present description, the angle or the direction can vary within the range of $\pm$ 10°, preferably $\pm$ 5°, and more preferably $\pm$ 2° with respect to the value of the angle or the direction referred to.

Also, when the figure, shape, arrangement or the like modified with the word "substantially" is circular or the like, it encompasses deformed shapes of a circle such as a shape with its circumference distorted to some extent, an elliptic shape, or an oval shape. When a polygonal shape or the like is referred to, it encompasses deformed shapes of a polygonal shape such as those with round corners, and those with its periphery distorted to some extent. Similarly, when a lattice pattern or the like is referred to, deformation of the shapes formed by its components is permissible.

1. Vessel for Cell Isolation, Culture and Proliferation

The present invention relates to a sealable vessel for cell isolation, culture and proliferation 1, the vessel being a container-like structural body in which isolation, culture and proliferation of target cells of interest can be performed, wherein the vessel comprises a cell separation unit 2, a primary culture unit 3 and a main vessel unit 4. The following table summarizes the units and their main components in the vessel for cell isolation, culture and proliferation 1 according to the present invention.

Though Table 1 summarizes the units and their main components, it does not show the necessary members in the present invention. Therefore, the technical scope of the present invention is not limited to an aspect including all the members described in Table 1.

Table 1

| Main members constituting the vessel for cell isolation, culture and proliferation 1 according to the present invention | |
| --- | --- |
| Cell separation unit 2 | Cell separation membrane 11<br>Membrane holding structure 13 |
| Primary culture unit 3 | Primary culture vessel 21 and 26<br>    - Structures 22 and 23 involved in the connection with the cell separation unit<br>    - Culture surface for primary culture 27<br>    - Releasing structure 28 |

(continued)

| Main members constituting the vessel for cell isolation, culture and proliferation 1 according to the present invention | |
| --- | --- |
| Main vessel unit 4 | Main vessel 41 |
| | - Structures 42 and 43 involved in the storage of the culture units for the preceding steps |
| | - Culture surface for cell proliferation 55 Main lid portion 56 |

[Cell Separation Unit]

The vessel for cell isolation, culture and proliferation 1 according to the present invention is a container-like structural body comprising a cell separation unit 2.

The cell separation unit 2 is a unit for isolating the target cells of interest 101 into the container-like structural body of the primary culture unit 3 through the cell separation membrane 11.

The cell separation unit 2 is a structural body comprising a cell separation membrane 11 having fine pores capable of isolating cells. Specifically, the cell separation unit 2 is a container-like structural body comprising the cell separation membrane provided on at least a part of surfaces of the container-like structural body of the cell separation unit.

In detail, the cell separation unit 2 is a container-like structural body mainly composed of the cell separation membrane 11 having fine pores capable of isolating cells and a membrane holding structure 13, and includes an opening portion 14 that allows the introduction of a liquid and an opening portion 15 that allows the discharge of a liquid through the cell separation membrane.

With the aforementioned structure, the cell separation unit serves as a member that exhibits essential functions in the cell isolation process. Meanwhile, it is permissible to include members other than the cell separation membrane and the membrane holding structure as the members of the cell separation unit as long as they do not interfere with the functions of the vessel for cell isolation, culture and proliferation according to the present invention.

In a preferable mode of the present invention, the cell separation unit is detachably connected to the primary culture unit or the like.

Cell Separation Membrane

The cell separation unit 2 comprises a cell separation membrane 11 as a member that exhibits essential functions in the cell isolation process.

The cell separation membrane 11 is a membrane structure having fine pores that can isolate cells. The fine pores, which are formed so that the target cells to be isolated can pass through them, function as cell-isolation pores.

In the cell separation membrane, it is preferable that pores larger than the fine pores do not exist on the membrane region at which isolation of cells is performed.

When the cell separation membrane 11 of the present invention is produced, it is preferable to employ the imprinting method utilizing a fine die as disclosed in WO2014/171365 in order to obtain fine pores which are the cell-isolation pores 12 having a size, porosity, pore arrangement, an opening angle and the like within preferable ranges as described below.

It is impossible to produce a cell separation membrane of such a structure if a conventional technique is employed in which pores are formed randomly by the irradiation of heavy particle beams or the like.

The size of the fine pores which are the cell-isolation pores 12 can be appropriately selected depending on the size of the target cells. For example, the average size of the fine pores formed in the cell separation membrane (the average inner diameter when the horizontal cross section of the pore is circular) is 1 to 20 $\mu$m, preferably 2 to 15 $\mu$m, and more preferably 3 to 10 $\mu$m. Especially, a preferable size for isolating stem cells is in a range of 3 to 10 $\mu$m.

Any pore shape can be adopted as the shape of the fine pores which are the cell-isolation pores 12, and for example, the horizontal cross section with respect to the membrane surface can be circular, substantially circular, elliptic, quadrangular, or polygonal with five or more apexes. Preferable examples include a circular shape, a substantially circular shape and a hexagonal shape.

The porosity of the cell separation membrane 11 is, for example, 4 to 50%, preferably 5 to 40%, and more preferably 5 to 20%. The higher the porosity, the more efficiently the target cells of interest can pass through the cell separation membrane. As a favorable result, the time required for isolating cells can be shortened.

It is preferable that the fine pores which serve as the cell-isolation pores 12 are regularly arranged on the membrane surface. Specifically, it is preferable that a part of or all of the fine pores are arranged in a lattice pattern, a substantially lattice pattern, a staggered pattern, a substantially staggered pattern, a spiral pattern, a substantially spiral pattern, a concentric circle pattern, a substantially concentric circle pattern, a combination of the aforementioned patterns, or the like. It is preferable that all the fine pores are arranged with aforementioned regularity.

It is preferable that the fine pores which are the cell-isolation pores 12 are opened in a vertical direction and/or a substantially vertical direction with respect to the membrane surface. Here, the substantially vertical direction specifically indicates an angle within $\pm10°$, preferably within $\pm5°$ and more preferably within $2°$ from the angle perpendicular to the membrane surface.

It is not preferable if a conventional membrane on which fine pores are arranged randomly is adopted instead of a membrane having a structure and the like of the cell separation membrane of the present invention, because each cell will have different moving distance, and as a result, the time required for isolating cells will tend to vary. Moreover, such a conventional membrane is not preferable because, if the content of the target cells to be isolated is low or if the cells are vulnerable to damage or the like, the number of cells that can reach the fine pores may decrease drastically.

Furthermore, the conventional membrane is not preferable because, if the randomly formed fine pores overlap with each other, the size of these pores will allow the passage of cells other than those to be isolated, and the cell selectivity will decrease.

It is preferable that the cell separation membrane 11 has a membrane thickness of 0.05 to 100 $\mu$m, preferably 0.5 to 50 $\mu$m, and more preferably 1 to 30 $\mu$m. It is not preferable if the membrane is too thick, because it will take time for the target cells to pass through the cell-isolation pores, and as a result, the time required for isolating cells will become longer. Moreover, it is not preferable because the target cells will be exposed to more stimuli and the like when they pass through the cell-isolation pores. Particularly, when stem cells, which are sensitive to external stimuli, are isolated, the membrane thickness within an appropriate range is desirable in order to obtain cells of good quality.

There is no particular limitation on the outer shape of the cell separation membrane 11 as long as it has a size and shape which can secure a sufficient area for isolating cells. For example, a circular filter shape with an outer diameter of about 2 to 50 mm, preferably 3 to 20 mm, can be adopted.

It is preferable that the base material layer of the cell separation membrane 11 is mainly made of hydrophobic polymers.

Preferable examples of the hydrophobic polymers used as the materials of the base material layer are PE (polyethylene), PET (polyethylene terephthalate), polycarbonate, polysulfone, polypropylene, polyvinylidene fluoride, polyamide and PLA (polylactic acid). Preferably, PE, PET and the like are suitable.

In the cell separation unit 2, a filter structure or filter structures other than the cell separation membrane 11 can be disposed above or below the cell separation membrane or to form layers with the cell separation membrane.

Any membrane, filter or the like can be employed as the filter structure without particular limitation as long as its structure and material do not interfere with the function of the cell separation membrane. Though there is no particular limitation on its material, pore size, thickness and the like, it is desirable that the structure does not greatly affect the migration efficiency, the stimuli responsibility and the like of the target cells.

With regard to the position of the filter structure, when the filter structure is disposed at one side of the cell separation membrane where the cell suspension before the isolation process is introduced, it can be used as a prefilter. When the filter structure is disposed at the primary culture unit side with respect to the cell separation membrane, it can be used as the supporting member of the cell separation membrane. In one mode, the filter structures can be provided on both sides of the cell separation membrane.

In another mode, the filter structure can be integrated with the cell separation membrane to form a composite membrane structure.

Membrane Surface Modification with Hydrophilic Polymers

The cell separation membrane 11 preferably has a structure in which hydrophilic polymers are bonded covalently on the surface of the base material layer.

In one mode, when the surface of the base material layer of the cell separation membrane is modified with hydrophilic polymers, the cell separation membrane can become a membrane member with improved surface properties, exhibiting cell non-adhesive properties. As a result, the number of target cells lost due to adhesion to the cell separation membrane in the cell isolation process can be reduced, and the yield of the target cells can be remarkably improved.

It is preferable that the surface of the base material layer of the cell separation membrane and the hydrophilic polymers are bonded so as to be insoluble in water, as they are brought into contact with a liquid such as culture medium whose main component is water.

As the hydrophilic polymers for improving the surface properties of the base material layer of the cell separation membrane 11 in the present invention, one or more kinds of polymers can be selected from vinylpyrrolidone polymers, ethylene glycol polymers and vinyl alcohol polymers.

Examples of vinylpyrrolidone polymers are polyvinylpyrrolidone, vinylpyrrolidone-vinyl acetate copolymers, vinylpyrrolidone-vinylalcohol copolymers, vinylpyrrolidone-styrene copolymers, vinylpyrrolidone-dimethylaminoethyl methacrylate copolymers, and modified polymers of these polymers. An example of ethylene glycol polymers is polyethylene glycol, and those having an ester group/groups on the side chain/chains are also included. An example of vinyl alcohol polymers is polyvinyl alcohol, and various polymers of different degrees of saponification can be included.

In particular, polyvinylpyrrolidone, polyvinyl alcohol, polyvinylpyrrolidone-polyvinyl acetate copolymers and the like are preferable.

It is preferable that these hydrophilic polymers have high water solubility. Therefore, it is preferable to use polymers whose number average molecular weight is ten thousand to one million, for example, but the molecular weight is not limited to these values as long as the polymers are water soluble.

For example, in a surface modification method for the improvement of the surface properties of the base material layer of the cell separation membrane 11, the base material layer is immersed in a solution containing water-soluble monomers, oligomers or polymers and irradiated with high energy rays to avoid the risks of clogging of the pores, residual of organic solvents and the like (WO2012/058637). Covalent bonding with water-soluble monomers, oligomers or polymers by radicals can also be performed by first irradiating the base material layer with high energy rays and then immersing it in a solution containing water-soluble monomers, oligomers or polymers.

In this covalent bonding method, surface modification is realized by covalently bonding the surface of the base material layer, which is made of hydrophobic monomers, oligomers or polymers, with the hydrophilic polymers.

Examples of the concentration of the water-soluble polymers in the solution used for surface modification with molecules in this covalent bonding method is 10 ppm or more, preferably 100 ppm or more, more preferably 500 ppm or more, and even more preferably 1,000 ppm or more. If the concentration is too high, the fine pores may be clogged. Therefore, it is desirable that the upper limit is preferably 20,000 ppm or less, and more preferably 10,000 ppm or less.

It is desirable to add alcohol to the solution used in the surface modification with molecules when the water absorption of the hydrophobic polymers of the base material layer is 2% or less.

Here, examples of the concentration of alcohol in the solution are 0.05 to 5 wt%, and preferably 0.1 to 1 wt%. From the viewpoint of safety, the alcohol used is preferably ethanol.

Ultraviolet rays, electron beams, gamma rays or the like can be used as the high energy rays to be irradiated. It is preferable to use electron beams or gamma rays to enhance the reaction efficiency.

The irradiation dose is preferably 5 to 35 kGy. In particular, an irradiation dose of about 25 kGy, which is recognized as effective for sterilization, is preferable, as both the membrane surface modification and sterilization can be performed concurrently.

Membrane Surface Modification by Plasma Treatment

It is possible to obtain a cell separation membrane with suitable properties by subjecting the cell separation membrane 11 to plasma treatment and directly hydrophilizing the surface of the hydrophobic base material layer. Examples of the plasma treatment are vacuum plasma treatment and atmospheric pressure plasma treatment, and a preferable example is vacuum plasma treatment.

In this mode of performing plasma treatment, the cell separation membrane can become a membrane member with improved surface properties, exhibiting cell non-adhesive properties. As a result, the adhesive properties of the target cells to the cell separation membrane in the cell isolation process decrease, and the yield of the target cells can be remarkably increased.

The cell separation membrane obtained by plasma treatment in this mode has a structure in which one or more selected from the hydroxyl group, the carboxyl group and a compound structure including said functional groups are covalently bonded to a vicinity of the surface of the base material layer made of hydrophobic polymers, and/or has an insoluble layer in the vicinity of the surface of the base material layer made of hydrophobic polymers, the insoluble layer being insoluble in a good solvent for the hydrophobic polymers. More specifically, the cell separation membrane has a structure in which hydroxyl groups and/or carboxyl groups are covalently bonded.

When plasma treatment is performed by vacuum plasma treatment, it becomes possible to precisely select the functional groups introduced in the vicinity of the surface by choosing the gas to be introduced.

The cell separation membrane obtained in this mode of performing plasma treatment has hydrophilicity as its surface properties, with its contact angle being 70° or less, preferably 60° or less, and more preferably 50° or less.

With regard to the surface properties of the cell separation membrane, there is no limitation on the lower limit of the contact angle because even when the surface is superhydrophilic, the characteristics of inhibiting cell adhesion can be exhibited. In an actual embodiment, the lower limit of the contact angle can be about 5° or more.

When plasma treatment is performed, it is known that deterioration with time usually occurs as hydroxyl groups and carboxyl groups are formed in the vicinity of the surface by surface etching. However, in this mode, the surface of the hydrophobic base material layer is treated by applying voltage at a low energy. Examples of the voltage to be applied are 0.01 kV or more, preferably 0.05 kV or more, more preferably 0.1 kV or more, and even more preferably 0.5 kV or more. The higher the applied voltage, the more the hydrophilization of the surface proceeds and thus is preferable. Although the upper limit of the applied voltage is not particularly limited, its example is 10 kV or less, and preferably 5 kV or less because, the lower the energy, the more the surface etching can be suppressed.

Particularly, by treating under a low voltage, it becomes possible to suitably form in the vicinity of the membrane surface a layer which is not dissolved even by a solvent (e.g. acetone or the like) which usually has a dissolving power. This layer is regarded as a surface structure in which surface etching is suppressed and on which a crosslinked layer is formed, and serves to maintain the effects of the plasma treatment for a long period of time.

In this mode of performing plasma treatment, it becomes possible to produce a cell separation membrane in which the surface of the hydrophobic base material layer is uniformly hydrophilized. Moreover, the cell separation membrane produced in this mode has extremely low occurrence of surface etching compared with a membrane produced by treatment with high energy rays such as gamma-rays. As a result, the cell separation membrane produced can have a crosslinked layer formed in the vicinity of the surface and have less occurrence of deterioration with time.

Plasma treatment can be performed in combination with other membrane treatment techniques. For example, it is possible that, while one surface of the hydrophobic base material layer is subjected to plasma treatment for hydrophilization, the other surface is subjected to treatment with high energy rays or the like.

Membrane Holding Structure

The cell separation unit 2 is a structural body in which at least a part of the surfaces of the vessel forming the membrane holding structure 13 is covered by the cell separation membrane 11.

Here, the membrane holding structure 13 is a member of the container portion of the container-like structural body of the cell separation unit and serves as a supporting member for physically holding the cell separation membrane. Also, the membrane holding structure 13 has a container-like structure which enables a liquid to be introduced into and filled in the cell separation unit when the cell separation membrane is attached, and has a structure with which the liquid filled can pass through the cell separation membrane.

Various forms can be employed for the membrane holding structure as long as the basic structures mentioned above can be realized, and their examples are a tubular shape, a substantially tubular shape, a pipe-like shape, a quadrangular pillar shape, a substantially quadrangular pillar shape, a spherical shape, a substantially spherical shape, a conical shape, a substantially conical shape, a polygonal pyramid shape, a substantially polygonal pyramid shape, a branched shape, a cup-like shape, a substantially cup-like shape, a dish-like shape, a substantially dish-like shape, and a structure obtained by the combination of the aforementioned shapes.

A material having a strength to physically hold the cell separation membrane is preferable as the material of the membrane holding structure 13. Except for the member 17 and the like used for clamping the cell separation membrane, it is preferable that the material has light transmitting properties. Especially, when microscopic observation is carried out without opening the main vessel unit 4, it is preferable that material with light transmitting properties is employed for the components of the membrane holding structure 13 located at positions opposed to or substantially opposed to the culture surfaces provided in the vessel for cell isolation, culture and proliferation 1. Here, the components located at positions opposed to or substantially opposed to the culture surfaces preferably indicate the components on the upper side of the culture surfaces.

Glass can be employed as the material with light transmitting properties, but it is preferable to employ a resin which is colorless, has high transparency and is convenient in handling. For example, materials such as polystyrene, polyethylene terephthalate (PET), polypropylene, polycarbonate, polymethylmethacrylate, polymethylpentene, a transparent ABS resin or the like can be employed.

At least one structure that enables a liquid to be introduced is provided as a part of the membrane holding structure 13. For said at least one structure, any structure can be adopted as long as a liquid can be introduced into the membrane holding structure. For example, a structure such as an opening portion 14 that has an opening is suitable. Although there is no particular limitation on the shape of the opening of the opening portion, a circular shape, a substantially circular shape, an oval shape, or a polygonal shape with four or more sides is preferably used.

The opening portion 14 is preferably designed to be located at a position on the upper side with respect to the surface of the liquid introduced into the membrane holding structure. Specific examples of the position are the side surface or the upper surface of the membrane holding structure, and the upper surface is preferable. When the membrane holding structure has a shape in which the upper surface and the side surface are not distinguished from each other, a position on the upper side of the surface of the liquid introduced is suitable.

Specifically, when the membrane holding structure has a tubular shape, for example, one end of the tubular shape can serve as the opening portion 14.

It is preferable that the membrane holding structure 13 is a container-like structural body having a volume suitable for holding the liquid introduced therein.

It is preferable to determine the volume based on the size of the cell separation membrane. For example, when the outer diameter of the cell separation membrane is 6 mm, the membrane holding structure preferably has a volume suitable for temporarily holding a liquid having a volume of 10 to 2,000 μL, preferably 50 to 1,000 pL, and more preferably about 100 to 500 μL.

The membrane holding structure 13 has, on at least one part thereof, an opening portion 15 that allows the cells to pass through and is covered by the cell separation membrane 11.

It is preferable that opening portion 15 is designed to be formed at a position on the lower side with respect to the surface of the liquid introduced into the membrane holding structure. Specific examples of the position are the side surface or the bottom surface of the membrane holding structure, and the bottom surface is preferable. When the membrane holding structure has a shape in which the bottom surface and the side surface are not distinguished from each other, a position on the lower side of the surface of the liquid introduced is suitable. That is, the cell separation unit 2 according to the present invention preferably has a structure in which the cell separation membrane is provided on the bottom surface of the membrane holding structure.

Specifically, when the membrane holding structure has a tubular shape, for example, one end of the tubular shape can serve as the opening portion.

As the opening portion 15 is covered by the cell separation membrane, target cells to be isolated 101 or a liquid culture medium held in the membrane holding structure can be transferred to the primary culture unit 3 through the cell separation membrane.

There is no particular limitation on the means by which one end of the membrane holding structure 13 physically holds the cell separation membrane 11, and an example thereof is a means to physically clamping the cell separation membrane using two structural members.

A specific example of said means is a ring structural body 17 having a structure to be engaged with the bottom surface portion 16 of the membrane holding structure. Here, the ring structural body can either be a member which is physically coupled with the membrane holding structure or a member formed separately from the membrane holding structure.

The ring structural body 17 is a member having a ring-shaped opening portion 18. Target cells to be isolated 101 or a liquid culture medium which have been held in the membrane holding structure can be transferred to the primary culture unit 3 through this opening portion 18.

When the membrane holding structure 13, the cell separation membrane 11 and the ring structural body 17 are layered, the opening portion 15 and the opening portion 18 preferably communicate with each other through the cell separation membrane.

As a result, a structure in which the cell separation membrane is clamped between the bottom surface portion of the membrane holding structure and the ring structural body can be realized.

When the ring structural body 17 is employed, it is preferable that the bottom surface portion of the membrane holding structure also has a structure to be engaged with or fitted to the ring structural body. There is no particular limitation on the engaging or fitting structure, and the examples thereof are a protrusion structure, a flange structure, and a female structure to be fitted to these structures.

Various shapes can be employed for the membrane holding structure 13 as long as the basic structures mentioned above are realized.

For example, a branching structure can be employed to provide a structure having two or more cell separation membranes. It is also possible to adopt a structure having two or more portions through which cells are introduced or filled. Also, the tubular structure can be formed to have one or more branched tubes or pipes.

It is also possible to bend the tube of the tubular structure. The diameter of the tube can also be changed in a stepwise manner.

Specifically, the membrane holding structure 13 according to the present invention can be a tubular structure. Here, the tubular structure indicates a structure in which the two ends of the tube communicate with each other.

Any shape can be adopted as the horizontal cross section of the tubular structure, examples of which are a circular shape, a substantially circular shape, an oval shape, a quadrangular shape, a substantially quadrangular shape, a polygonal shape and a substantially polygonal shape. Preferably, a membrane holding structure having a cylindrical shape or a substantially cylindrical shape whose horizontal cross section is circular or substantially circular is suitable.

The length of the long axis of the horizontal cross section (the inner diameter when the cross section is circular) can be 4 to 80 mm, and preferably 4 to 24 mm, for example.

When said shape of the tubular structure is employed, one end of the tubular structure can be covered by the cell separation membrane. In this case, the liquid introduced into the tubular structure is transferred to the primary culture unit through the cell separation membrane.

Connecting Portion with the Primary Culture Unit

Although the present invention does not exclude a mode in which the cell separation unit 2 and the primary culture unit 3 are formed integratedly, it is preferable that the cell separation unit can be detachably attached to the primary culture vessel 21 of the primary culture unit. In the mode in which the cell separation unit can be detached, operations such as the medium exchange can be easily performed from the opening portion 23 exposed.

Specifically, the cell separation unit and the primary culture vessel are preferably arranged so that, when a liquid is filled in the primary culture vessel 21, the surface of the cell separation membrane 11 of the cell separation unit and the surface of the liquid filled in the primary culture vessel are in contact with each other.

The state in which "the membrane surface and the liquid surface are in contact with each other" not only includes a state in which the surface of the cell separation membrane and the entire surface of the liquid filled in the primary culture vessel are in contact with each other, but also a state in which the membrane surface and the liquid surface are in contact with each other in a state that the outer side of the membrane holding structure is immersed in the liquid in the primary culture vessel. Also, the two members can be disposed not only in a state in which they are fixed or connected with each other but also in a state in which they are engaged with each other, layered, or the like.

The cell separation unit 2 is preferably a structural body provided with the cell separation membrane 11 at the bottom surface 16 of the membrane holding structure. Specifically, it is preferable that the surface on which the cell separation membrane is formed is a part of the bottom surface of the membrane holding structure.

It is desirable that the surface of the cell separation membrane 11 and the liquid surface of the primary culture unit are in contact with each other without the presence of air bubbles.

Structure for Preventing Air Bubbles

In the present invention, the membrane holding structure 13, at its connecting portion with the primary culture unit 3, preferably has a structure that can prevent the occurrence of air bubbles on the surface of the cell separation membrane when the membrane holding structure is connected to or placed on the primary culture vessel.

Any form can be adopted as the structure for preventing air bubbles, and, for example, the peripheral portion of the bottom surface portion 16 of the opening portion 15 of the membrane holding structure covered by the cell separation membrane can have an upward inclination toward the opening portion 15.

For example, it is desirable that the bottom surface 16 of the membrane holding structure has a shape with an upward inclination toward the opening portion 15. Here, the angle of inclination can either be uniform throughout the entire peripheral portion or varied. From the viewpoint of preventing air bubbles, it is desirable that the angle of inclination is preferably 1 to 10°, and more preferably about 1 to 5°.

Connecting Portion with the Main Lid Portion

The membrane holding structure 13 preferably has a structure that enables to adjust the position of the cell separation membrane 11 of the cell separation unit 2 in response to the operation of the main lid portion 56. As an example of such a structure, the membrane holding structure 13 and the main lid portion are provided to be connectable with each other, and as the main lid portion is rotated, the membrane holding structure is detached or its position is changed, and as a result, the position of the surface of the cell separation membrane can be shifted. For example, it is possible to adopt a structure in which the position of the surface of the cell separation membrane moves upward in response to the rotation of the main lid portion.

As the connecting portion with the main lid portion, the upper portion of the membrane holding structure is preferably provided with a means to be engaged with or fitted to the main lid portion.

[Primary Culture Unit]

The vessel for cell isolation, culture and proliferation 1 according to the present invention is a container-like structural body provided with a primary culture unit 3.

The primary culture unit 3 is a container-like structural body that has a culture surface for primary culture on which cells that have passed through the cell separation membrane can be cultured and a releasing structure through which cells and a liquid that have been held inside the primary culture unit can be freely released toward a unit having a culture surface for a subsequent step. Specifically, (i) the primary culture unit 3 is a container-like structural body; (ii) the primary culture unit has a structure with which the cell separation unit 2 can be disposed in a state in which, when a liquid is filled in the container-like structural body of the primary culture unit, a surface of the cell separation membrane 11 and a surface of the liquid filled are in contact with each other; (iii) the primary culture unit has the culture surface for primary culture 27 on which the cells that have passed through the cell separation membrane can be cultured, the culture surface for primary culture being provided on at least a part of surfaces of the container-like structural body of the primary culture unit; and (iv) the primary culture unit has the releasing structure through which the cells and the liquid that have been held inside the container-like structural body of the primary culture unit can be freely released toward the unit 4 or 5 having the culture surface for the subsequent step, the releasing structure being provided at a surface of the container-like structural body of the primary culture unit.

With this structure, the primary culture unit 3 can function as a container for performing preculture of the cells isolated by the cell separation unit as a preparatory step for the proliferation culture, which is the main culture step. The primary culture unit can include members other than the primary culture vessel, as long as they do not interfere with the functions of the vessel for cell isolation, culture and proliferation 1 according to the present invention.

In the present invention, the primary culture unit is preferably adapted to be freely attached to and detached from the cell separation unit, the cell proliferation unit, the main vessel unit and the like.

Primary Culture Vessel

The primary culture unit 3 is a container-like structural body mainly composed of the primary culture vessel 21. The primary culture vessel 21 has structure and characteristics that secure the above-mentioned functions of the primary culture unit.

The primary culture vessel 21, except for the components of an elastic member 29 such as an O-ring, is preferably made of material with light transmitting properties. Especially, when microscopic observation is carried out without opening the main vessel unit 4, the vessel surface forming the culture surface for primary culture 27 is preferably made of material with light transmitting properties. Preferably, the vessel structures at positions opposed to or substantially opposed to the culture surface for primary culture are also made of material with light transmitting properties. Here, the vessel structures at positions opposed to or substantially opposed to the culture surface for primary culture preferably indicate the vessel structures on the upper side with respect to the culture surface.

Glass can be employed as the material with light transmitting properties, but it is preferable to employ a resin which has high transparency and is convenient in handling. For example, materials such as polystyrene, polyethylene terephthalate (PET), polypropylene, polycarbonate, polymethylmethacrylate, polymethylpentene and a transparent ABS resin can be employed. Especially, a material suitable for the proliferation of the cells to be cultured, such as polystyrene and polyethylene terephthalate (PET), can be appropriately employed for the culture surface.

The primary culture vessel 21 is a container-like structural body capable of holding a liquid. Any shape can be employed as the shape of the container-like portion as long as the above-mentioned functions of the primary culture vessel can be exhibited.

Various shapes and sizes can be adopted for the structure of the container-like portion, as long as the concentration of the cells isolated by the cell separation unit 2 does not become too low and a cell sheet or a cell population can be formed to a certain extent.

Various shapes can be employed for the container-like portion as long as the basic structures mentioned above are realized, and their specific examples are a tubular shape, a substantially tubular shape, a pipe-like shape, a quadrangular pillar shape, a substantially quadrangular pillar shape, a spherical structure, a conical shape, a substantially conical shape, a polygonal pyramid shape, a substantially polygonal pyramid shape, a branched shape, a cup-like shape, a substantially cup-like shape, a dish-like shape, a substantially dish-like shape, and a structure obtained by the combination of the aforementioned shapes.

As the size of the container-like portion, one having an area that can be employed for the culture surface for primary culture 27 described in the paragraphs below is preferable. Though it may depend on the area of the cell separation membrane 11, an area of 10 to 400 $cm^2$ is preferable.

As the volume of the container-like portion, a volume with which a liquid culture medium having a volume suitable for the primary culture process can be held is preferable, and for example, a vessel capable of holding a liquid of 6 to 280 mL is preferable.

Structure for Insertion and Removal of Hollow Tubular Member

It is preferable that the primary culture vessel 21 has a structure 34 which is provided on the wall surface of its container-like structural body and which enables the insertion and removal the hollow tubular member 121.

Here, the structure 34 for insertion and removal of the hollow tubular member of the primary culture vessel is a structure for breaking the contact between the cell separation membrane 11 and the liquid surface in the primary culture vessel without opening the main vessel unit 4.

The structure 34 for insertion and removal of the hollow tubular member of the primary culture vessel does not have to be formed specifically when the upper portion of the primary culture vessel has an opening portion, but it is preferable to form the structure for insertion and removal of the hollow tubular member when the upper portion of the primary culture vessel has a ceiling plate structure.

Here, the hollow tubular member 121 is a member which has a hollow inner portion and through which liquid can be suctioned or injected using an external suction mechanism (plunger, decompression device, or the like). The outer diameter of the hollow tubular member is preferably about 0.2 to 2.2 mm in order to secure airtightness at the structure 60 for insertion and removal of the hollow tubular member of the main vessel unit upon inserting the hollow tubular member into the vessel. Specifically, a syringe needle, a cannula, a tube, a pipe, or the like can be preferably used. The tip portion of the hollow tubular member preferably has a sharp hollow needle shape. It is more preferable to use a syringe needle of 14 to 33 gauge, and even more preferably, 18 to 25 gauge or the like.

The structure 34 for insertion and removal of the hollow tubular member of the primary culture vessel is a structure that enables to exchange the liquid in the primary culture vessel by suction or injection through the hollow tubular member 121. An open structure with a shape and a size that allow the insertion or removal of the hollow tubular member 121 can be employed.

It is not necessary for the structure 34 for insertion and removal of the hollow tubular member of the primary culture vessel to have a structure formed by an elastic member capable of securing airtightness, and a simple open structure can be adopted. Moreover, similar to the structure 60 for insertion and removal of the hollow tubular member of the main vessel unit, a structure mainly composed of an elastic member capable of securing airtightness can also be employed.

It is preferable that the structure 34 for insertion and removal of the hollow tubular member of the primary culture vessel is located at a position which enables to suction the liquid filled in the primary culture unit through the hollow tubular member 121 inserted through the structure 60 for insertion and removal of the hollow tubular member of the main vessel unit. It is provided at the primary culture vessel preferably at its upper portion or its side surface portion located above the surface of the liquid filled.

Specifically, the structure 34 for insertion and removal of the hollow tubular member is preferably located on the line connecting the structure 60 for insertion and removal of the hollow tubular member of the main vessel unit and the surface of the liquid filled in the primary culture unit, so that the hollow tubular member 121 inserted through the main vessel unit 4 can reach the liquid filled in the primary culture unit without penetrating the cell separation membrane 11.

Particularly, the structure 34 for insertion and removal of the hollow tubular member of the primary culture vessel and the structure 60 for insertion and removal of the hollow tubular member of the main vessel unit are preferably located perpendicular to or substantially perpendicular to the surface of the liquid filled in the primary culture unit.

Culture Surface for Primary Culture

The primary culture vessel 21 is a container-like structural body having a culture surface for primary culture 27 on which the cells that have passed through the cell separation membrane 11 can be cultured, the culture surface for primary culture being provided on at least a part of surfaces of the container-like structural body of the primary culture vessel.

The culture surface for primary culture 27 is preferably a structure formed on the bottom surface of the primary culture vessel 21, but, in one mode, the culture surface can be provided on a vessel surface or the like other than the bottom surface, such as the side surface. In another mode, a plurality of the culture surfaces for primary culture can be provided in the primary culture vessel. For example, the bottom surface can be divided or partitioned into two or more areas, so that each of them can be used as the culture surface. In still another mode, the side surface or the like, as well as the bottom surface, can be used as the culture surface for primary culture.

Though the culture surface for primary culture can have a curved surface shape in which the surface is bent or has a curvature, it preferably has a planar shape or a substantially planar shape. It is especially preferable that the culture surface has a flat planar shape.

Various shapes and sizes can be adopted for the structure of the culture surface for primary culture 27, and it is preferable to employ a shape and size with which the concentration of the cells isolated by the cell separation unit 2 does not become too low and a cell sheet or a cell population can be formed to a certain extent.

Specifically, a circular shape, a substantially circular shape, a quadrangular shape, a substantially quadrangular shape, a polygonal shape, a substantially polygonal shape, a polygonal shape with chamfered corners, or the like can be employed as the shape of the culture surface. Preferably, a culture surface having a circular shape or the like is suitable.

As the size of the culture surface, 10 to 400 cm$^2$ is preferable, for example.

The inner surface of the base material of the primary culture vessel 21 itself can be used as the culture surface for primary culture 27. It is also possible to employ a flat plate member provided separately from the surfaces of the vessel. In this case, the flat plate member which serves as the culture surface for primary culture 27 is regarded as a part of the members of the primary culture vessel 21.

The flat plate member which serves as the culture surface for primary culture 27 can be layered on, for example, the bottom surface or the like of the primary culture vessel. Alternatively, the flat plate member can be fitted to the primary culture vessel instead of providing the primary culture vessel with the bottom surface. It is also possible to provide a stepped structure with which a space can be formed between the flat plate member and the bottom surface, so that two or more culture surfaces can be provided.

The shape and size of the culture surface mentioned above can be adopted for the shape and size of the flat plate member.

Surface Modification with Stimuli-Responsive Molecules Exhibiting Cell Detachment Properties

The surface of the culture surface for primary culture 27 is preferably subjected to modification with molecules which have cell adhesion properties during cell culture and exhibit cell detachment properties in response to stimuli. In this mode, the culture surface has improved surface properties, modified with molecules that exhibit cell detachment properties in response to stimuli.

Here, the stimuli responsiveness preferably indicates response to stimuli that can be applied without opening the main vessel unit 4 of the present invention, examples of which are light and temperature. Specifically, modification with molecules which exhibit cell detachment properties in response to stimuli, such as light-responsive molecules or temperature-responsive molecules, is preferable.

For example, temperature-responsive polymers can suitably be used in the present invention as the molecules which have cell adhesion properties during cell culture and exhibit cell detachment properties in response to stimuli. In this mode, the surface is modified with molecules which show hydrophobic tendency during culture and whose characteristics rapidly change to show hydrophilic tendency in response to temperature stimuli.

When temperature-responsive polymers are used as the molecules for surface modification, it is preferable to use polymer molecules whose phase transition temperature, that is, the lower critical solution temperature (LCST), is lower than the culture temperature for the target cells of interest and is within the temperature range suitable for the survival of the cells. Examples of the suitable phase transition temperature are 15 to 35°C, preferably 20 to 35°C, and more preferably 22 to 30°C so as to reduce the damages and stimuli to the cells.

Examples of the constitutional unit of the temperature-responsive polymer are N-n-propylacrylamide (LCST: 21°C), N-n-propylmethacrylamide (LCST: 27°C), N-tetrahydrofurfuryl acrylamide (LCST: about 28°C), N-isopropylacrylamide (LCST: 32°C), N,N-diethylacrylamide (LCST: 32°C), N-ethoxyethyl acrylamide (LCST: about 35°C), N-tetrahydrofurfuryl methacrylamide (LCST: about 35°C), and methyl vinyl ether (LCST: 35°C).

While it is preferable to employ homopolymers of the substances mentioned above as the temperature-responsive polymers in the present invention, copolymers of these substances can also be used as long as the lower critical solution temperature (LCST) is within a predetermined range.

When the surface properties of the culture surface for primary culture 27 are improved by modification with the aforementioned temperature-responsive polymers, the culture surface shows hydrophobic tendency at around 37°C, which is an optimal temperature for cell culture. Then, the nature of the culture surface rapidly changes from hydrophobic to hydrophilic only by performing low-temperature treatment, and the cell detachment properties can be exhibited.

Surface modification treatment on the base material layer of the culture surface for primary culture 27 can be performed by graft copolymerization between the molecules of the base material of the culture surface and the temperature-responsive polymers. Covalent binding to the molecules of the base material can be performed by a covalent binding method using high energy rays. For example, it can be performed following the description of Japanese Patent No. 3641301. It is also possible to perform graft polymerization by conducting plasma treatment (preferably vacuum plasma treatment) and forming radicals on the surface.

Surface Modification by Plasma Treatment

It is possible to provide the culture surface for primary culture 27 with properties suitable as a culture surface by performing plasma treatment and directly hydrophilizing the surface of the hydrophobic base material layer. Examples of the plasma treatment are vacuum plasma treatment and atmospheric pressure plasma treatment, and a preferable example is vacuum plasma treatment.

It is known that plasma treatment usually causes deterioration with time as surface etching occurs and hydroxyl groups and carboxyl groups are formed in the vicinity of the surface. However, when plasma treatment is performed by applying low voltage, the occurrence of surface etching is extremely low, a thick crosslinked layer is formed in the vicinity of the surface, and as a result, a culture surface with remarkably low occurrence of deterioration with time can be obtained. The culture surface with improved surface properties can be obtained, serving as a plate member which promotes cell spreading and exhibits moderate hydrophilicity to an extent that allows cell adhesion. In this mode, in which plasma treatment is performed, it becomes possible to produce a culture surface with the surface of its hydrophobic base material layer uniformly hydrophilized.

The culture surface for primary culture 27 obtained by plasma treatment in this mode has a structure in which one or more selected from the hydroxyl group, the carboxyl group, and a compound structure including said functional groups are covalently bonded to a vicinity of a surface of a base material layer made of hydrophobic polymers, and/or has an insoluble layer in the vicinity of the surface of the base material layer made of hydrophobic polymers, the insoluble layer being insoluble in a good solvent for the hydrophobic polymers. More specifically, the culture surface for primary culture has a structure in which hydroxyl groups and/or carboxyl groups are covalently bonded.

An example of the surface properties of the culture surface for primary culture 27 obtained in this mode, in which plasma treatment is performed, is hydrophilicity, with the contact angle of the culture surface being 70° or less, and preferably 60° or less. Cell adhesion tends to be inhibited when the hydrophilicity of the surface of the culture surface for primary culture 27 is too high, and thus, a contact angle of 10° or more, preferably 20° or more, and more preferably 30° or more is suitable.

In the mode in which plasma treatment is performed, the surface of the hydrophobic base material layer is treated by applying voltage at a low energy. Though the voltage to be applied may vary depending on the polymer materials of the base material layer, examples thereof are 0.01 kV or more, preferably 0.05 kV or more, more preferably 0.1 kV or more, and even more preferably 0.5 kV or more. The upper limit of the applied voltage is, for example, 5 kV or less, preferably 3 kV or less, and more preferably 2 kV or less, because cell adhesion may be affected when the voltage is too high.

Particularly, by treating under low voltage, it becomes possible to suitably form, in the vicinity of the surface of the culture surface for primary culture, a layer which is not dissolved even by a solvent (e.g. acetone or the like) which usually has a dissolving power. This layer is regarded as a surface structure in which surface etching is suppressed and on which a crosslinked layer is formed, and serves to maintain the effects of plasma treatment for a long period of time.

Connection Structure with the Cell Separation Unit

The primary culture vessel 21 is a structural body having a structure that can dispose the cell separation unit 2 in a state that, when a liquid is filled in the container-like structural body of the primary culture vessel, the surface of the cell separation membrane 11 and the surface of the liquid filled are in contact with each other.

Here, the "state in which the membrane surface and the liquid surface are in contact with each other" not only includes a state in which the surface of the cell separation membrane and the entire surface of the liquid filled in the primary culture vessel are in contact with each other, but also a state in which the membrane surface and liquid surface are in contact with each other in a state that the outer side of the membrane holding structure is immersed in the liquid in the primary culture vessel. Also, the two members can be disposed not only in a state in which they are fixed or connected with each other but also in a state in which they are engaged with each other, layered, or the like.

Specifically, in the structure of the vessel's connecting portion with the cell separation unit, an opening portion 23 which communicates with the cell separation membrane 11 of the membrane holding structure is provided at one of the vessel surfaces of the primary culture vessel. At the connecting portion, said opening portion 23 and the opening portion 15 of the cell separation unit are in communication through the cell separation membrane.

Examples of the shape of the opening portion 23 include not only a normal hole-like open structure but also such structures as those in which the peripheral portion of the open structure has a tubular shape.

The size of the opening portion 23 is not particularly limited as long as it allows the smooth transfer of liquid from the cell separation membrane 11, but it is preferably larger than the size of the cell separation membrane 11. Yet, the opening portion 23 is not excluded from having a size smaller than that of the cell separation membrane or a shape formed by the combination of the shapes smaller than the cell separation membrane.

It is more preferable if the opening portion 23 has a shape through which such operations as medium exchange can be performed between processes when the cell separation unit 2 is removed. The suitable longest length of the cross section of the opening portion is, for example, 3 to 50 cm, and preferably about 5 to 30 cm.

The position of the opening portion 23 is not particularly limited, and it is preferably a position on the upper or side surface of the primary culture vessel 21 and at which the liquid medium surface and the cell separation membrane 11 are in contact with each other when the liquid medium is filled. A position on the upper surface of the primary culture vessel is preferable from the viewpoint of improving the cell recovery rate and preventing the occurrence of air bubbles. It should be noted that even a shape in which the upper surface and the side surface are not distinguished from each other can be employed as the vessel shape as long as the upper limit mentioned above can be satisfied.

The peripheral portion of the opening portion 23 preferably has a structure that can be engaged with or fitted to the peripheral portion 16 around the cell separation membrane of the cell separation unit. There is no particular limitation on the engaging or fitting structure, and the examples thereof are a protrusion structure, a flange structure, and a female structure to be fitted to these structures.

The peripheral portion of the opening portion 23 preferably has a medium trapping structure to store the medium that overflows when, for example, the cell separation unit 2 is connected or placed.

When the cell isolation process is performed using the vessel 1 according to the present invention, the surface of the cell separation membrane 11 of the cell separation unit needs to be in contact with the surface of the liquid filled in the primary culture unit. In many cases, when the cell separation unit is placed, the liquid medium is filled to the maximum level in order to prevent the occurrence of air bubbles between the membrane surface and the liquid surface. As an unfavorable result, the medium overflows around the opening portion 23, entering the vessel.

As the medium trapping structure, specifically, it is possible to employ a structure such as a groove structure, a trench structure or the like which is provided around the periphery of the opening portion 23 and can store the overflowing medium. The groove or the trench can be arranged in any shape, such as a circular shape, a substantially circular shape, a polygonal shape, and a chamfered polygonal shape, and it is preferably arranged so as to surround the peripheral portion of the opening portion 23.

The location of the cell separation membrane 11 at the primary culture vessel 21 is a position which allows the formation of a space providing a certain distance between the cell separation membrane and the wall surface on the side opposing to the opening portion 23 (i.e. the culture surface).

It is sufficient if the distance between the cell separation membrane and the culture surface for primary culture can provide an enough space in which the cells that have passed through the cell separation membrane can be cultured in a state they are immersed in the medium. For example, the distance between the surface of the cell separation membrane and the culture surface for primary culture 27 is 1 to 50 mm, and preferably about 2 to 40 mm.

Also, a means for adjusting the position of the cell separation membrane can be provided in the primary culture vessel. In another possible mode, a spacer member or a supporting member is provided additionally and fitted for the adjustment of said position.

In the primary culture process, primary culture of the isolated cells can be performed by filling the liquid medium into the space formed in the vessel.

Releasing Structure

The primary culture vessel 21 is a container-like structural body having a structure at the surface of its container-like structural body which can freely release the cells and liquid retained in the vessel into the unit 4 or 5 having the culture surface for the subsequent step.

The releasing structure 28 can serve to retain the liquid in the vessel when it is closed. When the releasing structure is opened, it allows to release the cells and liquid retained in the vessel outward, that is, into the unit having the culture surface for the subsequent step.

This structure enables to release the liquid medium containing the cells which has been retained in the primary culture vessel during the primary culture process into the structure outside the primary culture vessel.

Any structure can be adopted as the releasing structure 28 without restriction as long as it has a mechanism with which its opening/closing can be freely controlled and it can exhibit the functions mentioned above. For example, it is possible to employ a releasing port structure that can be opened/closed, a releasing window structure that can be opened/closed, a structure that can release the contents inside by detaching or changing the position of the plug member fitted, or a structure that can release the contents inside by detaching or changing the position of the vessel fitted.

The position to form the releasing structure 28 is not particularly limited as long as it is located lower than the liquid surface when a liquid is filled in the primary culture vessel 21. Specific examples are the bottom surface or the side surface of the primary culture vessel.

The number of the releasing structure 28 is not particularly limited, and when a releasing port or a releasing window is employed, two or more releasing structures located at a distance from each other can be provided. With regard to the structure of the releasing structure 28, any shape and size can be employed as long as the liquid medium can be released into the unit 4 or 5 located outside the primary culture vessel.

In one mode of the releasing structure 28, the primary culture vessel 21 is formed as a container-like structural body composed of two or more structures, and, as these structures are detached or their positions are changed, the above-mentioned structure capable of releasing the contents inside can be adjusted to open.

In this mode, the primary culture vessel is provided as a container-like structural body composed of two or more members, having a structure with which the above-mentioned structure capable of releasing the contents inside can be adjusted to open as the members of the container-like structural body are detached or their positions are changed.

In this mode, more specifically, the primary culture vessel is a container-like structural body mainly composed of an upper structural body 22 and a lower structural body 26, and the above-mentioned structure capable of releasing the contents inside can be adjusted to open as the upper structural body 22 and the lower structural body 26 are separated or their positions are changed.

It is also possible to employ an elastic member 29 such as an O-ring sandwiched at a position where the upper structural body 22 and the lower structural body 26 are in contact with each other in order to secure airtightness.

As the mechanism that allows to control the opening/closing of the releasing structure 28, a member 35 for blocking the releasing structure can be employed as the component member, and examples thereof are a fitting member, a filling member, a lid-like member, a stopper member, and a sealing member, which have shapes to block the releasing window. In one mode, a portion of the upper structural body can be provided as the member 35 for blocking the releasing structure in the mechanism for controlling the opening/closing.

When the member 35 for blocking the releasing structure is fitted, the releasing structure 28 closes and thus the liquid is retained in the primary culture vessel. When the blocking member is detached or its position is changed, the releasing structure 28 opens and thus the liquid inside the primary culture vessel is released outside.

In one method for opening the releasing structure 28, the operator opens the main vessel unit 4 and directly touches and handles the primary culture unit. For example, the main lid portion 56 of the main vessel unit is removed, the member 35 for blocking the releasing structure is detached or its position is changed, and consequently, the releasing structure is opened.

In another mode, it is possible to employ an adjustment mechanism which can be operated from outside the main vessel unit without opening the main vessel unit 4 so as to open the releasing structure.

Specifically, in this mode, the primary culture unit 3 can be provided with an adjustment structure in which the structure 28 capable of releasing the contents inside is opened in response to the operation of the main lid portion 56. As an example of such a structure, the member 35 for blocking the releasing structure (or its connecting member) and the main lid portion 56 are provided with structures connectable to each other, and as the main lid portion is rotated, the position of the member for blocking the releasing structure is shifted so that it is detached or its position is changed. Consequently, the releasing structure 28 is opened. For example, it is possible to employ a structure with which the position of the member 35 for blocking the releasing structure is changed as it is lifted upward in response to the rotation of the main lid portion 56 and the releasing structure 28 opens.

At the connecting portion with the main lid portion 56, the upper portion or the connecting member of the member 35 for blocking the releasing structure preferably has a means to engage with or fitted to the main lid portion.

In another mode of the adjustment mechanism, it is possible to employ a structure in which the releasing structure 28 is opened by the operation using magnets or the like. In one example of a possible structure, the member 35 for blocking the releasing structure is made of a magnet or a magnetic material, and as magnetic force is applied from outside, the member 35 for blocking the releasing structure is detached from the releasing structure 28 or its position is changed, thereby opening the releasing structure.

Entire Structure of the Primary Culture Unit

The primary culture unit 3 is a term which practically indicates the same area as the primary culture vessel 21. The primary culture unit can be provided as a structure in which the entire structures, including the container-like portion, the culture surface for primary culture, the structure for releasing the liquid medium and the like, and the structure for connecting with the cell separation unit, are integrated. In another possible mode, these structures are provided as an assemblage of members. The assembled members serve as the members of the primary culture vessel.

The assembled members can be obtained by assembling the members each corresponding to a component structure. In another possible mode, two or three members, each including a plurality of component structures, are assembled.

For example, the primary culture unit can be provided as an assemblage of members that can be divided into the upper structural body 22 and the lower structural body 26. For example, the upper structural body can include the upper portion of the container-like portion, the structure for connecting with the cell separation unit, and the mechanism for controlling the opening/closing of the structure for releasing the liquid medium and the like. The lower structural body can include the side and bottom surfaces of the container-like portion, the culture surface for primary culture, and the window portion of the structure for releasing the liquid medium and the like, for example.

The outer surface of the primary culture vessel preferably has a structure with which, when the primary culture vessel is housed in the container-like structural body to be located outside the primary culture vessel, that is, in the main vessel unit 4 or the cell proliferation unit 5, the primary culture vessel can be engaged with the outer vessel. There is no particular limitation on the engaging or fitting structure, and the examples thereof are a protrusion structure, a flange structure, and a female structure to be fitted to these structures.

In another mode, a spacer member or a supporting member can be additionally provided to realize the engaging or fitting structure.

[Main Vessel Unit]

The vessel for cell isolation, culture and proliferation according to the present invention is a container-like structural body including the main vessel unit.

The main vessel unit 4 is a container-like structural body that has a culture surface for cell proliferation on which cells that have been released from a culture unit for a preceding step can be cultured, an area of the culture surface for cell proliferation being larger than that of a culture surface of the culture unit for the preceding step, the container-like structural body of the main vessel unit having a structure in which the cell separation unit and all of the culture unit/units for preceding step/steps can be stored. Specifically, (i) the main vessel unit 4 is a container-like structural body mainly composed of a main vessel 41 and a main lid portion 56; (ii) the main vessel unit has the culture surface for cell proliferation 55 on which the cells that have been released from the culture unit 3 or 5 for the preceding step can be cultured, the area of the culture surface for cell proliferation being larger than that of the culture surface of the culture unit for the preceding step; (iii) the main vessel unit has a structure with which the cell separation unit 2 and all of the culture unit/units 3 and 5 for the preceding step/steps can be stored in the container-like structural body of the main vessel unit; and (iv) the main lid portion is a lid-like structural body with which the entire vessel can be sealed.

The main vessel unit 4 has a structure capable of maintaining the inner space of the entire vessel according to the present invention in a sealed state. As a result, in the present invention, all the processes from cell isolation, primary culture to cell proliferation and the like can be performed in the same vessel. Moreover, the main vessel unit functions as a housing that can secure the physical strength of the vessel for cell isolation, culture and proliferation 1 according to the present invention.

The main vessel unit 4 is a member that exhibits essential functions in the cell proliferation process. A preferable mode of the cell proliferation process can be achieved by providing a gas exchange port 47 or a medium exchange port 51.

Members of the main vessel unit 4 can include members other than those mentioned above as long as they do not interfere with the functions of the vessel for cell isolation, culture and proliferation according to the present invention.

It is preferable that the main vessel unit 4 can be detachably connected to the primary culture unit, the cell proliferation unit, or the like.

Main Vessel

The main vessel 41 is a container-like structural body capable of retaining a liquid. The main vessel 41 has a structure to secure the above-mentioned functions of the container-like portion of the main vessel unit 4.

Various shapes and sizes can be adopted for the container structure of the main vessel 41, as long as they are shapes and sizes with which the concentration of the cells that have been released from the culture unit for the preceding step does not become too low, and in addition, a cell sheet or a cell population can be formed to a certain extent.

Various shapes can be employed for the container-like portion as long as the basic structures mentioned above are realized, and their specific examples are a tubular shape, a substantially tubular shape, a pipe-like shape, a quadrangular pillar shape, a substantially quadrangular pillar shape, a spherical structure, a conical shape, a substantially conical shape, a polygonal pyramid shape, a substantially polygonal pyramid shape, a branched shape, a cup-like shape, a substantially cup-like shape, a dish-like shape, a substantially dish-like shape, and a structure obtained by the combination of the aforementioned shapes.

The main vessel 41 is preferably made of material with light transmitting properties, except for the members of an elastic member 57 such as an O-ring. Especially, when microscopic observation is carried out without opening the main vessel unit 4, it is preferable that the vessel surface forming the culture surface for cell proliferation 55 is made of material with light transmitting properties. It is preferable that the vessel structures at positions opposed to or substantially opposed to the culture surface for cell proliferation are also made of material with light transmitting properties. Here, the vessel structures at positions opposed to or substantially opposed to the culture surface for cell proliferation preferably indicates the vessel structures on the upper side with respect to the culture surface.

Glass can be employed as the material with light transmitting properties, but it is preferable to employ a resin which has high transparency and is convenient in handling. For example, materials such as polystyrene, polyethylene terephthalate (PET), polypropylene, polycarbonate, polymethylmethacrylate, polymethylpentene, or a transparent ABS resin can be employed. Especially, material suitable for the proliferation of the cells to be cultured, such as polystyrene and polyethylene terephthalate (PET), can be appropriately employed for the culture surface.

With regard to the size of the container-like portion of the main vessel 41, an area that can be employed for the culture surface for cell proliferation 55 described in the paragraphs below is preferable. For example, when usage in an incubator or the like is expected, a vessel having an area of 30 to 1,600 $cm^2$ is preferable, but the size is not limited to this range when the vessel is used in a large-scale experiment or the like.

With regard to the volume of the container-like portion, a volume that can retain a certain amount of liquid medium suitable for cell proliferation is preferable. For example, when usage in an incubator or the like is expected, a vessel capable of retaining about 30 to 1,500 mL of a liquid is preferable, but the volume is not limited to this range when the vessel is used in a large-scale experiment or the like.

Structure for Insertion and Removal of Hollow Tubular Member

The main vessel 41 preferably has, on the wall surface of its container-like structural body, a structure 60 that enables the insertion and removal the hollow tubular member 121.

Here, the structure 60 for insertion and removal of the hollow tubular member of the main vessel is a structure for breaking the contact between the cell separation membrane 11 and the liquid surface in the primary culture vessel without opening the main vessel unit 4.

Here, the hollow tubular member 121 indicates a member which has a hollow inner portion and through which liquid can be suctioned or injected using an external suction mechanism (plunger, decompression device, or the like).

Details are described in the above paragraphs about the primary culture vessel 21.

The structure 60 for insertion and removal of the hollow tubular member of the main vessel is a structure that enables to exchange the liquid in the primary culture vessel by suction or injection through the hollow tubular member 121. For the structure for insertion and removal of the hollow tubular member, it is possible to employ a structure mainly composed of elastic members that can secure airtightness both in a state in which the hollow tubular member is not inserted and in a state in which the hollow tubular member is inserted.

Specifically, the structure for insertion and removal of the hollow tubular member is preferably made of a material with which, when the hollow tubular member 121 is inserted through the structure for insertion and removal of the hollow tubular member, the structure can be in close contact with the outer side of the hollow tubular member located thereon so that airtightness can be secured. Moreover, when the hollow tubular member inserted through the structure for insertion and removal of the hollow tubular member is pulled out, the material preferably enables the inner portion of the through hole to close tightly to secure airtightness.

An example of the member of the structure 60 for insertion and removal of the hollow tubular member is a resin elastic member. Examples thereof are silicone resin, urethane resin, and rubber.

The structure 60 for insertion and removal of the hollow tubular member of the main vessel is preferably located at a position which enables to suction the liquid filled in the primary culture unit through the hollow tubular member inserted through the structure for insertion and removal of the hollow tubular member. It is preferably provided at the upper portion of the main vessel 41 or its side surface portion above the surface of the liquid filled. More preferably, it is provided at the main lid portion 56.

Specifically, the structure for insertion and removal of the hollow tubular member is preferably provided at a position from which the hollow tubular member 121 inserted from outside can reach the liquid filled in the primary culture unit without penetrating the cell separation membrane 11. Particularly, it is preferably located at a position perpendicular to or substantially perpendicular to the surface of the liquid filled in the primary culture unit.

Culture Surface for Cell Proliferation

The main vessel 41 is a container-like structural body having, on at least one surface forming the container-like structural body, a culture surface for cell proliferation 55 on which the cells that have been released from the culture unit 3 or 5 for the preceding step can be cultured.

The culture surface for cell proliferation 55 is preferably a structure formed on the bottom surface of the main vessel 41 but, in one mode, the culture surface can be provided on a vessel surface or the like other than the bottom surface, such as the side surface. In another mode, a plurality of the culture surfaces for cell proliferation can be provided in the main vessel. For example, the bottom surface can be divided or partitioned into two or more areas, and each of them can be used as the culture surface. In still another mode, the side surface or the like, as well as the bottom surface, can be used as the culture surface for cell proliferation.

Though the culture surface for cell proliferation can have a curved surface shape in which the surface is bent or has a curvature, it preferably has a planar shape or a substantially planar shape. It is especially preferable that the culture surface has a flat planar shape.

Various shapes and sizes can be adopted for the structure of the culture surface for cell proliferation 55, and it is preferable to employ shapes and sizes with which the concentration of the cells that have been released from the culture unit 3 or 5 for the preceding step does not become too low, and in addition, a cell sheet or a cell population can be formed to a certain extent.

Specifically, a circular shape, a substantially circular shape, a quadrangular shape, a substantially quadrangular shape, a polygonal shape, a substantially polygonal shape, a polygonal shape with chamfered corners, or the like can be employed as the shapes of the culture surface. Preferably, a culture surface having a quadrangular shape with chamfered corners is suitable.

With regard to the preferable size of the culture surface for cell proliferation 55, when the area of the culture surface of the culture unit 3 or 5 for the preceding step is taken as 1, the area ratio of the culture surface for cell proliferation is in a range of more than 1 to not more than 20, preferably 1.5 to 15, more preferably 2 to 10 and even more preferably 3 to 8. This range is desirable because, when the area ratio is within this range, proliferation culture can be appropriately performed while maintaining the cell characteristics.

It is not preferable if the area ratio is too large, because the number of cells per unit culture area becomes small, intercellular communication becomes difficult, and cell proliferation functions are lowered. Moreover, it is not preferable because there is a possibility that the cell characteristics are changed.

Also, it is not preferable if the area ratio is too small, because the number of cells per unit culture area becomes large, the passage number tends to increase, and thus more operations are required. Moreover, it is not preferable because, during the subculturing process, cell differentiation may be induced due to cell aggregation, and the cell characteristics may be easily changed.

The inner surface of the base material of the main vessel 41 itself can be used as the culture surface for cell proliferation 55. It is also possible to provide the culture surface for cell proliferation 55 separately from the surfaces of the vessel, by employing a flat plate member as the culture surface. In this case, the flat plate member which serves as the culture surface for cell proliferation is regarded as a part of the members of the main vessel.

The flat plate member which serves as the culture surface for cell proliferation 55 can be layered on, for example, the bottom surface or the like of the main vessel. It is also possible to adopt a stepped structure with which a space can be formed between the flat plate member and the bottom surface, so that two or more culture surfaces can be provided.

The shape and size of the culture surface mentioned above can be employed for the shape and size of the flat plate member.

Modification with Stimuli-Responsive Molecules Exhibiting Cell Detachment Properties

The surface of the culture surface for cell proliferation 55 can be subjected to surface modification with molecules which have cell adhesion properties during culture and exhibit cell detachment properties in response to stimuli. In this mode, the culture surface has improved surface properties, modified with molecules that exhibit cell detachment properties in response to stimuli.

The surface modification of the culture surface for cell proliferation can be performed following the method for the surface modification of the culture surface for primary culture 27. Also, in this invention, the surface modification of the culture surface for cell proliferation can be performed by a method different from that employed in the surface modification of the culture surface for primary culture. For example, it is possible that the surface modification of the culture surface for primary culture is performed using temperature-responsive molecules or the like, while the surface modification of the culture surface for cell proliferation is performed using light-responsive molecules or the like.

Surface Modification by Plasma Treatment

The surface of the culture surface for cell proliferation 55 can be provided with properties suitable as a culture surface by performing plasma treatment and directly hydrophilizing the surface of the hydrophobic base material layer.

It is known that plasma treatment usually causes deterioration with time as surface etching occurs and hydroxyl groups and carboxyl groups are formed in the vicinity of the surface. However, when plasma treatment is performed by applying low voltage, the occurrence of surface etching is extremely low, a thick crosslinked layer is formed in the vicinity of the surface, and as a result, a culture surface with remarkably low occurrence of deterioration with time can be obtained. The culture surface with improved surface properties can be obtained, serving as a plate member which promotes cell spreading and exhibits adequate hydrophilicity to an extent that allows cell adhesion. In this mode of performing plasma treatment, it becomes possible to produce a culture surface with the surface of its hydrophobic base material layer uniformly hydrophilized.

The culture surface for cell proliferation 55 obtained by plasma treatment in this mode has a structure in which one or more selected from the hydroxyl group, the carboxyl group, and a compound structure including said functional groups are covalently bonded to a vicinity of a surface of a base material layer made of hydrophobic polymers, and/or has an insoluble layer in the vicinity of the surface of the base material layer made of hydrophobic polymers, the insoluble layer being insoluble in a good solvent for the hydrophobic polymers. More specifically, the culture surface for cell proliferation has a structure in which hydroxyl groups and/or carboxyl groups are covalently bonded.

Plasma treatment on the surface of the culture surface for cell proliferation can be performed by using the surface modification method for the culture surface for primary culture 27.

Particularly, by treating under low voltage, it becomes possible to suitably form in the vicinity of the membrane surface a layer which is not dissolved even by a solvent (e.g. acetone or the like) which usually has a dissolving power. This layer is regarded as a surface structure in which surface etching is suppressed and on which a crosslinked layer is formed, and serves to maintain the effects of plasma treatment for a long period of time.

Structure for Storing Culture Units for Preceding Steps

The main vessel 41 is a structural body having a structure with which the cell separation unit 2 and the culture unit 3 or culture units 3 and 5 for the preceding step/steps can be stored in its container-like structural body.

The main vessel 41 is a structural body provided with an opening portion 43 through which the cell separation unit, the culture units and the like are inserted and taken out. The cell separation unit, the culture unit/units and the like can be stored in the main vessel 41, as they are inserted into the main vessel through the opening portion 43.

An example of the shape of the opening portion 43 is a normal hole-like open structure, and the peripheral portion of the opening structure preferably has a shape suitable for being fitted to the main lid portion. The size of the opening portion 43 is not particularly limited as long as it allows the culture unit 3 or 5 for the preceding step to be inserted or taken out smoothly. Preferably, the size of the opening portion is larger than the size of the outer periphery of the culture unit for the preceding step. More preferably, the opening portion is slightly larger than the size of the outer periphery of the culture unit for the preceding step, and the interior of the opening portion can be physically connected to the culture unit for the preceding step.

As a preferable structure of the opening portion 43, the inner wall of the opening portion 43 has a shape to be engaged with or fitted to the outer periphery of the culture unit for the preceding step, and the opening portion 43 has a structure for fixing the culture unit for the preceding step. There is no particular limitation on the engaging or fitting structure, and examples thereof are a protrusion structure, a flange structure, and a female structure to be fitted to these structures.

In one mode, a spacer member or a supporting member can be provided additionally to realize the engaging or fitting structure.

When the culture units or the like are stored in the main vessel 41, the cell separation unit 2, the culture unit 3 or culture units 3 and 5, and the like are disposed inside the main vessel.

In one mode, when compact storage is achieved in the vessel 1 according to the present invention, the culture units are stored in a state in which the outer wall of the bottom surface of the culture unit 3 or 5 for the preceding step is in contact with the inner surface of the main vessel 41 (specifically, with the culture surface for cell proliferation 55). If desired, a means for adjusting the vertical position of the outer wall of the bottom surface of the culture unit 3 or 5 can be provided in the main vessel so that a certain area can be secured for the culture surface for cell proliferation 55. In another mode, a spacer member or a supporting member can be additionally provided so as to adjust the vertical position of the outer wall of the bottom surface of the culture unit 3 or 5.

In another mode, when said means for adjusting the vertical position is not provided, the culture units can be stored in the main vessel in a state in which the outer wall of the bottom surface of the culture unit 3 or 5 for the preceding step is not in contact with the inner surface (specifically, with the bottom surface) of the main vessel 41 so that a certain area can be secured for the culture surface for cell proliferation 55.

The main vessel 41 is a structural body which has a certain height in the vertical direction to realize the storage of the cell separation unit 2, the culture unit 3 or culture units 3 and 5, and the like.

The height in the vertical direction of the main vessel 41 is not particularly limited as long as the cell separation unit, the culture unit/units, and the like can be stored. Moreover, even when the height in the vertical direction of the main vessel is lower than the height of the culture unit/units and the like, they can be stored in the main vessel by forming the main lid portion 56 in a dome shape or the like.

An example of the height of the main vessel 41 is a height that can secure a distance from the stage or condenser for carrying out microscopic observation.

The opening portion 43 is preferably a structural body with which the main lid portion 56 is fitted and connected to the main vessel 41 so as to cover the opening portion 43. Therefore, the periphery of the opening portion 43 preferably has a structure to be fitted to the main lid portion 56.

The structure 45 of the main vessel to be fitted to the side surface structure of the main lid portion 56 is not particularly limited, and a general structure which can be engaged with a lid, such as a screw structure, a flange structure, and a protrusion structure, can be employed.

Main Lid Portion

The main vessel unit 4 is a structural body having the main lid portion 56. The main lid portion 56 is a lid-like structural body that can bring the entire vessel into a sealed state.

Various shapes and sizes mentioned above can be employed for the structure of the main lid portion 56, and the shapes and sizes are not particularly limited as long as the function of sealing the entire vessel can be exhibited in a state where the cell separation unit 2 and the culture unit 3 or culture units 3 and 5 for the preceding step/steps are stored in the container-like structural body of the main vessel 41.

The main lid portion 56 has a structure capable of being fitted to the opening portion 43 of the main vessel. The structure in which the lid portion is engaged with or fitted to the main vessel is not particularly limited, and it is possible to adopt a general lid structure, such as a structure in which the side surface structure of the main lid portion 56 is fitted to the structure at the opening portion of the main vessel, a structure in which the lid portion is fitted to the opening portion of the main vessel with a screw cap structure, a structure in which a flange structure is employed for the engagement, and a structure in which a protrusion structure is employed for the engagement. An elastic member 57 such as an O-ring is preferably sandwiched at the fitting portion between the main vessel 41 and the main lid portion 56 so as to secure the airtightness of the entire main vessel.

The structure of the upper surface of the main lid portion 56 is not particularly limited as long as the cell separation unit 2 and the culture unit 3 or culture units 3 and 5 for the preceding step/steps can be stored. Any shape can be adopted for the structure, and examples thereof are a flat-plate shape and a dome shape.

The inner portion of the upper surface of the main lid portion is preferably provided with a fitting structure 58 that comes into contact with or engaged with a part or parts of the cell separation unit and/or culture units and fix them.

In one mode, each component unit can be operated in response to the rotation of the main lid portion 56. An example of this mode is the adjustment operation of the releasing structure 28 of the primary culture unit. Another example is the adjustment operation of the vertical position of the outer wall of the bottom surface of the primary culture vessel. Still another example is the adjustment operation of the position of the cell separation membrane 11 of the cell separation unit.

In another mode, a plurality of these adjustment operations can be carried out only by the rotation operation of the main lid portion 56.

The main lid portion 56 preferably has a structure with which the releasing structure 28 of the primary culture unit can be opened in response to the operation of the main lid portion.

An example of this mode is a structure with which the main lid portion 56 and the member 35 for blocking the releasing structure (or its connecting portion) are provided as connectable structures, and as the main lid portion 56 is rotated, the member 35 for blocking the releasing structure is shifted so that it is detached or its position is changed, and the releasing structure 28 is opened. Specifically, the member 35 for blocking the releasing structure or the connecting portion thereof can be detached in the upward direction, or its position can be changed in the upward direction.

In this mode, it is preferable that the structure can secure the airtightness of the main vessel unit 4 even when the main lid portion is rotated.

The main lid portion 56 preferably has a structure in which the vertical position of the outer wall of the bottom surface of the primary culture vessel can be adjusted, as the outer wall of the bottom surface of the primary culture vessel 21 is detached in the upward direction or its position is changed in the upward direction with respect to the culture surface for cell proliferation 55 in response to the operation of the main lid portion. This structure enables to form a gap or a space between the outer wall of the bottom surface of the primary culture vessel 21 and the culture surface for cell proliferation 55 and to increase the area of the culture surface for cell proliferation 55 on which cells can be cultured.

An example of this mode is a structure in which the main lid portion 56 and the primary culture vessel 21 (or its connecting portion) are provided as connectable structures, and as the main lid portion 56 is rotated, the bottom surface of the primary culture vessel is detached in the upward direction, or its position is changed in the upward direction. In this mode, it is preferable that the structure can secure the airtightness of the main vessel unit 4 even when the main lid portion is rotated.

The main lid portion 56 preferably has a structure capable of adjusting the position of the cell separation membrane 11 of the cell separation unit in response to the operation of the main lid portion.

An example of this mode is a structure in which the main lid portion 56 and the membrane holding structure 13 are provided as connectable structures, and as the main lid portion 56 is rotated, the membrane holding structure 13 is detached or its position is changed, and the position of the surface of the cell separation membrane 11 can be shifted. In this mode, it is preferable that the structure can secure the airtightness of the main vessel unit 4 even when the main lid portion is rotated.

The main lid portion 56 is preferably made of material with light transmitting properties, except for the members of the elastic member 57 such as an O-ring. Especially, when microscopic observation is carried out without opening the main vessel unit 4, at least a part of the main lid portion is preferably made of material with light transmitting properties.

Glass can be employed as the material with light transmitting properties, but it is preferable to employ a resin which has high transparency and is convenient in handling. For example, material such as polystyrene, polyethylene terephthalate (PET), polypropylene, polycarbonate, polymethylmethacrylate, polymethylpentene, and a transparent ABS resin can be employed. Especially, material suitable for the proliferation of the cells to be cultured, such as and polystyrene and polyethylene terephthalate (PET), can be appropriately employed for the culture surface.

Rotation Adjustment Mechanism

In one mode of the vessel according to the present invention in which the above-mentioned operation can be performed in response to the rotation of the main lid portion 56, it is possible to adopt a rotation adjustment mechanism 61 with which predetermined rotational positions of the main lid portion 56 can be corresponded to predetermined steps in structural changes of the vessel.

The rotation adjustment mechanism 61 of the vessel according to the present invention is a mechanism including (i) a structural body that is provided in the vicinity of the outer peripheral portion of the structure 45 to be fitted to the main lid portion and can adjust the degree of rotation of the main lid portion to predetermined rotational positions; (ii) a structural body to be fitted to the structural body recited in (i), which is provided on the side surface structure of the main lid portion 56 and with which the operator can recognize the rotational position of the main lid portion.

In one mode of the member of the rotation adjustment mechanism 61, a ring structural body 62 on which adjustment portions 63 to be loosely fitted to the protruding portions 66 of the main lid portion are disposed can be employed. In this mode, the ring structural body 62 and the protruding portions 66 of the main lid portion serve as the members of the rotation adjustment mechanism 61.

In one mode, the ring structural body 62 is disposed in contact with the outer peripheral portion of the structure 45 to be fitted to the main lid portion. In another mode, the ring structural body 62 of the rotation adjustment mechanism 61 can be a structure integrated with the outer peripheral portion of the fitting structure 45 or the opening portion 43 of the main vessel.

In another mode of the member of the rotation adjustment mechanism 61, it is also possible not to employ the ring structural body. For example, the adjustment portions 63 can be directly arranged on the outer peripheral portion of the fitting structure 45 or the opening portion 43 of the main vessel.

An example of the adjustment portion 63 is a structural body which can be loosely fitted to the protruding portions 66 of the main lid portion and with which the engagement can be easily broken by the force applied in the rotation operation. A specific example of the adjustment portion 63 is a boss structure composed of two bosses 64. Preferably, the surface on the opposite side of the boss structure has a recessed or concave structure 65. When such a structure 65 is adopted, the protruding portion 66 of the main lid portion is loosely fitted between the two bosses 64, and when the protruding portion 66 passes over the boss 64 by the pushing force applied in the rotation operation, the operator can feel the change of the pushing force.

Any material can be employed without limitation as the material of the adjustment portion 63 as long as the aforementioned functions can be achieved, and it is preferable to use hard resin having elasticity. For example, materials such as polycarbonate and a transparent ABS resin are preferable. It is particularly preferable to employ materials having light transmitting properties.

General protruding shapes such as a convex shape and a triangular shape can be employed as the shape of the bosses 64 of the adjustment portion 63. Also, in one mode, one of the bosses of the adjustment portion 63 can be provided as a boss for preventing reverse rotation 64b which has a trapezoidal shape and whose surface facing the other boss has a vertical plane so as to prevent reverse rotation.

The adjustment portions 63 can be disposed on the ring structural body 62 or in the vicinity of the opening portion 43 of the main vessel at equal angular intervals with respect to the center of the ring structural body or the center of the opening portion of the main vessel. In one mode, the adjustment portions can be arranged at intervals of 5 to 90 degrees, and preferably about 5 to 45 degrees.

An example of the protruding portion 66 of the main lid portion is a structure that is provided on the side surface structure of the main lid portion 56 at a position capable of being fitted to the adjustment portion 63. Examples of the shape of the protruding portion 66 are protruding shapes such as a pointed shape or a triangular shape. In one mode, one protruding portion 66 is provided at a position capable of being fitted to the adjustment portion. Preferably, two protruding portions are provided at positions point symmetric about the center of the ring structural body 62 or the center of the opening portion 43 of the main vessel. It is also possible to provide protruding portions 66 at the same numbers as the adjustment portions 63 so that they will be arranged at positions corresponding to the adjustment portions 63.

In the mode in which the rotation adjustment mechanism 61 is provided, when the protruding portions 66 moves adjacent to the adjustment portion 63, the operator can recognize that one step of rotation has been performed. For example, in one mode where several predetermined rotational positions are corresponded to certain structural changes of the primary culture unit, the operator can easily recognize the rotational position and its corresponding structural change of the vessel as he rotates the main lid portion.

With the vessel according to the present invention, the operator can easily recognize the degree of rotation of the main lid portion and the corresponding change of the vessel structure by sensing the change of pushing force which occurs as the protruding portion passes over the boss 64 in the rotation operation. It is also possible to provide a structure in which a clicking sound is generated when the protruding portion passes over the boss 64 by the pushing force applied in the rotation operation. It is more preferable to employ a structure in which both the change of the pushing force and the clicking sound can be recognized.

Gas Exchange Port

The main vessel unit 4 is preferably a structure provided with a gas exchange port 47. Gas exchange with the outside air can be performed through the gas exchange port 47 in the cell isolation step, the primary culture step and the proliferation step in the present invention.

The gas exchange port 47 is preferably formed in the upper surface or the upper part of the side surface of the main vessel 41. The upper surface portion is preferable. It can also be formed in the main lid portion 56. When the vessel has a shape in which the upper part of the side surface is not distinguished, or the upper surface and the side surface are not distinguished from each other, it is desirable to provide the gas exchange port at a position on the upper side with respect to the surface of the liquid filled.

The number of the gas exchange port 47 is preferably two or more so as to efficiently carry out the gas exchange.

In this case, some of the gas exchange ports serve as the gas inlet port, while others serve as the gas outlet port.

When gas exchange with the outside air is performed through the gas exchange port, it is desirable that the gas exchange port is provided with a sterilizing filter 49 for ventilation. When the gas exchange port is closed, it is desirable to use a stopper structure such as a cap.

Here, an example of the sterilizing filter 49 is a fine-structure filter which allows the passage of air and prevents the passage of bacteria and the like. A filter having a structure to prevent the passage of bacteria and the like, such as a commercially available disk filter or polytetrafluoroethylene (PTFE) having a membrane with a pore diameter of 0.22 $\mu$m or less, can be used as the sterilizing filter 49. It is preferable to employ a structure in which hollow fiber filters are incorporated into the gas exchange port, as the entire vessel according to the present invention becomes compact and not bulky.

The area of the membrane used as the sterilizing filter 49 can be determined according to the gas capacity of the culture vessel. Typically, it is preferable to select an area that enables to filtrate the air of about 10 times the volume of the vessel within several to 10 minutes. With regard to the type of the membrane used as the sterilizing filter 49, a planar shaped filter, a hollow fiber membrane or the like can be used as desired.

The material of the sterilizing filter 49 can either be hydrophilic or hydrophobic. In a preferable mode, a filter made from a hydrophobic material is used to prevent clogging due to contact with moisture.

With the vessel 1 according to the present invention, the environment of the entire vessel can be adjusted to a culture condition with desired atmosphere by employing a structure in which the gas of desired composition is filled into the vessel through a tube or the like attached to the gas exchange port 47 and then the gas exchange port is closed with a cap or the like. In this case, adjustment of the culture condition can be achieved at a low cost, as the adjustment of the atmospheric composition of the entire incubator is not required.

Medium Exchange Port

The main vessel unit 4 is preferably provided with a medium exchange port 51 through which a medium can be introduced into or discharged from the main vessel 41. Especially, medium exchange can be easily performed through the medium exchange port 51 in the cell proliferation process.

Though the medium exchange port 51 can be formed in the bottom surface or the lower part of the side surface of the main vessel by being provided with a sealing structure such as a cap, the medium exchange port is preferably formed in the upper surface or the upper part of the side surface of the main vessel 41. The upper surface portion is preferable. When the vessel has a shape in which the upper part of the side surface is not distinguished, or the upper surface and the side surface are not distinguished from each other, it is desirable to provide the medium exchange port at a position on the upper side with respect to the surface of the liquid filled.

The number of the medium exchange port 51 is not particularly limited, and it is preferably one or more. When the medium exchange port is closed, it is desirable to use a stopper structure such as a cap. A luer-lock structure, for example, can be incorporated into the stopper structure such as a cap, so that the stopper structure may not be detached easily.

The medium exchange port 51 can be provided with an auxiliary member for facilitating the medium exchange. For example, it is possible to employ a structure provided with a tubular member 52 or the like that can be fitted to the medium exchange port and can be inserted close to the bottom surface of the main vessel 41.

Composition of the Entire Main Vessel Unit

Though the main vessel unit 4 can be provided as a structural body in which all structures, including the main vessel, the main lid portion, the storage structure, the structure for gas exchange, and the structure for medium exchange, are integrated, it is also possible to provide these structures as an assemblage of members.

The assembled members can be obtained by assembling the members each corresponding to a component structure. In another possible mode, two or three members, each including a plurality of component structures, are assembled.

For example, the main vessel can be provided as an assemblage of members that can be divided into the upper structural body 42 and the lower structural body 54. For example, the upper structural body can include the upper portion of the container-like portion and the structure for connection to the main lid portion. The lower structural body can include the side surface, the bottom surface, and the like of the container-like portion, for example.

In one mode, it is also possible to additionally provide a spacer member or a supporting member if desired, as a means to store other vessel units in the main vessel unit.

[Cell Proliferation Unit]

The vessel for cell isolation, culture and proliferation 1 according to the present invention can be provided as a container-like structural body having one or more cell proliferation units 5 as well as the aforementioned units.

In this mode, the vessel for cell isolation, culture and proliferation 1 according to the present invention comprises one or more cell proliferation units with which a plurality of cell proliferation processes can be performed after a primary culture process.

This structure realizes a preferable form of a culture vessel when preculture is performed in several steps, induction is carried out, or proliferation culture is expected to be performed in multiple steps.

Specifically, (i) the cell proliferation unit 5 is a container-like structural body; (ii) the cell proliferation unit comprises a culture surface for cell proliferation on which cells that have been released from a culture unit for a preceding step can be cultured, an area of the culture surface for cell proliferation being larger than that of a culture surface of the culture unit for the preceding step; (iii) the cell proliferation unit has a structure with which the cell separation unit and all of the culture unit/units for the preceding step/steps can be stacked or stored in the container-like structural body of the cell proliferation unit; and (iv) the cell proliferation unit has a releasing structure through which cells and a liquid that have been held inside the container-like structural body of the cell proliferation unit can be freely released toward a unit having a culture surface for a subsequent step, the releasing structure being provided at a surface of the container-like structural body of the cell proliferation unit.

The structures of the main vessel 41 and the primary culture vessel 21 can be applied as the basic structure of the cell proliferation unit 5. Specifically, structures of the main vessel 41 can be adopted to the structures mentioned in (i) to (iii) above. The structure of the primary culture vessel 21 can be adopted to the structure mentioned in (iv) above.

The structure unique to the cell proliferation unit 5 is that it has a structure with which the culture units for the preceding steps or the like can be stacked or stored. Unlike the main vessel, it is not necessary for the container-like portion of the cell proliferation unit 5 to store the culture unit for the preceding step. Therefore, in one mode, the culture unit for the preceding step can be simply stacked in the container-like structural body of the cell proliferation unit 5.

It is preferable that the cell proliferation unit 5 has, on its inner wall, a shape to be fitted to the culture unit for the preceding step and has an engaging or fitting structure capable of fixing the culture unit for the preceding step.

There is no particular limitation on the engaging or fitting structure, and the examples thereof are a protrusion structure, a flange structure, and a female structure to be fitted to these structures. In one mode, a spacer member or a supporting member can be provided additionally to realize the engaging or fitting structure.

The cell proliferation unit 5 serves as a unit having a culture surface for the subsequent step when viewed from the primary culture unit 3 and serves as a preceding culture unit when viewed from the main vessel unit 4. In one mode, two or more cell proliferation units can be stacked or stored in a nested manner.

It is possible to employ a culture surface for cell proliferation of the cell proliferation unit 5 that is provided with characteristics similar to those of the culture surface for cell proliferation 55 of the main vessel or the culture surface for primary culture 27 of the primary culture vessel.

With regard to the preferable size of the culture surface for cell proliferation of the cell proliferation unit 5, when the area of the culture surface of the culture unit for the preceding step is taken as 1, the ratio of the area of the culture surface for cell proliferation is within a range of more than 1 to not more than 20, preferably 1.5 to 15, more preferably 2 to 10, and even more preferably 3 to 8.

## 2. Functions of the Vessel for Cell Isolation, Culture and Proliferation According to the Present Invention

The vessel for cell isolation, culture and proliferation 1 according to the present invention can realize high-efficiency isolation of cells of interest from a tissue or a cell population and their high-efficiency proliferation. The isolation cells of interest and their proliferation culture can be performed at high efficiency with simple operation and at a low cost by using the vessel for cell isolation, culture and proliferation 1 according to the present invention.

[Cell Isolation and Proliferation Performed in One Vessel]

## Main Functions of the Vessel for Cell Isolation. Culture and Proliferation

The vessel for cell isolation, culture and proliferation 1 according to the present invention enables to carry out the isolation and proliferation of the target cells of interest in one vessel with a simple operation, as the series of processes including cell separation, primary culture, cell detachment, and cell proliferation are performed in the same vessel. This series of processes can be performed without requiring such handling steps as the transfer of cells to a different vessel for conducting proliferation culture.

The excellent functions of the vessel for cell isolation, culture and proliferation 1 according to the present invention can only be achieved by the interaction of the special structures and features of its component units.

Specifically, with the vessel for cell isolation, culture and proliferation according to the present invention, a series of processes such as cell separation and various culture processes can be performed while a closed culture environment or a sterile environment in which contamination or the like from outside sources is prevented is maintained in the vessel.

This effect has been achieved with the idea of storing the culture unit/units for the preceding step/steps, such as the primary culture unit, in the main vessel unit and performing the cell isolation process, the primary culture process, and the like inside the same vessel in a sealed state.

Also, the vessel for cell isolation, culture and proliferation according to the present invention has been realized with the idea of providing the vessel portion of the primary culture unit with a structure capable of freely releasing the contents in the vessel portion toward the unit having the culture surface for the subsequent step.

Especially, the vessel for cell isolation, culture and proliferation according to the present invention can be suitably used for the cell isolation, culture and proliferation of stem cells, which need to meet the safety requirements of the Ministerial Ordinance on Standards for Manufacturing Control and Quality Control for Drugs and Quasi-drugs (GMP), including the prevention of mycoplasma and virus infection, contamination from other cell sources, and risk of canceration.

Yield of Target Cells

In the vessel for cell isolation, culture and proliferation 1 according to the present invention, it becomes even more easier to transfer the cells, as a base material whose surface is modified with stimuli-responsive molecules having cell detachment properties is employed as the culture surface of the culture unit. In this mode, it becomes possible to greatly reduce the physical damage at the time of detaching the cells. Especially, for the cells that have required enzyme treatment with trypsin or the like, stimulation applied to cells by enzyme treatment can be reduced.

Physical damage or stimulation by enzyme treatment must be avoided as much as possible in the procedures after the primary culture, which is a preculture step, as the yield of target cells is not sufficient yet. Especially, in isolation and culturing of stem cells or the like, which are highly sensitive to stimuli and whose pluripotency tends to be easily lost, avoidance of stimulation is crucial in order to obtain cells of high quality without negatively affecting the functions and structure of the cells. Therefore, it is especially effective to provide the primary culture unit with a culture surface in which the surface of the base material is modified with stimuli-responsive molecules having cell detachment properties.

In the vessel for cell isolation, culture and proliferation 1 according to the present invention, when the cell separation membrane 11 of the cell separation unit is made of a base material in which the surface of the base material made of hydrophobic polymers is modified with hydrophilic polymers, cell adhesion to the cell separation membrane can be prevented and the isolation of target cells can be performed even more efficiently. Especially, when the target cells are precious stem cells, it is crucial to prevent cell adhesion and improve the yield.

Types of Target Cells

The vessel for cell isolation, culture and proliferation 1 according to the present invention can be applied to the isolation of any cells as long as they can adhere to the culture surface and be cultured. In principle, it can be applied to cells of all the eukaryotic organisms exhibiting adhesion properties. Examples thereof are animal cells, specifically vertebrate cells, and more specifically mammalian cells.

Especially, for reasons mentioned above, the vessel can be suitably used for the isolation and proliferation of stem cells. Particularly, the vessel is preferable in that it can fulfill the safety requirements of GMP, preventing mycoplasma and virus infection, contamination from other cell sources and risk of canceration, and moreover, cell isolation, culture and proliferation can be performed efficiently.

Examples of stem cells include embryonic stem cells, tissue stem cells, mesenchymal stem cells, and iPS cells, but the cells for which the vessel can be used are not limited to these examples.

[A Mode for Avoiding the Opening of the Main Vessel Unit]

The vessel for cell isolation, culture and proliferation 1 according to the present invention preferably has a means and/or structure that allow to avoid the opening of the main vessel unit during the cell culture operation to further reduce the risk of infection and contamination.

Light-Transmitting Member

In a preferable mode, in the vessel for cell isolation, culture and proliferation 1, members at predetermined positions in the vessel for cell isolation, culture and proliferation are made of light-transmitting material, and the culture surfaces inside the vessel can be observed through portions having light-transmitting properties without opening the main vessel unit.

This composition realizes a structural body with which the inside of the vessel can be observed with a microscope or the like without opening the main vessel unit 4 throughout the entire processes including cell isolation, primary culture, cell detachment and cell proliferation. Specifically, direct observation through the vessel can be carried out using a stereo microscope, an inverted microscope, a phase-contrast microscope, or the like. Particularly, in this mode, real-time observation with clear focus images can be suitably performed using a phase-contrast microscope.

With regard to the portions having light-transmitting properties in this mode, it is preferable that the portions through which the culture surfaces inside the vessel are observed are made of material with light-transmitting properties. Specifically, it is preferable that at least a part, and preferably the entire parts, of all the culture surfaces in the vessel for cell isolation, culture and proliferation are made of material with light-transmitting properties. Here, said culture surfaces include the culture surface 27 of the primary culture vessel and the culture surface 55 of the main vessel and, in one mode when the cell proliferation unit 5 is installed, the culture surface of the cell proliferation unit is also included therein.

Moreover, with regard to the portions having light-transmitting properties, it is preferable that a part, and preferably the entire parts, of the vessel structures opposed to or substantially opposed to the culture surface of the main vessel unit 4 are made of material with light-transmitting properties. Here, the components opposed to or substantially opposed to the culture surface preferably refer to the structures on the upper side of the culture surface.

Specifically, it is preferable that at least a part of the main lid portion 56, and preferably the entire part of the main lid portion except for the elastic member and the like, is made of material with light-transmitting properties.

Also, with regard to the portions having light-transmitting properties, it is preferable that, in the culture unit 3 or culture units 3 and 5 and the cell separation unit 2, at least a part of a structure located at a position opposed to or substantially opposed to the culture surface is made of material with light-transmitting properties. It is preferable that the entire parts thereof are made of material with light-transmitting properties.

Especially, the upper surface or the ceiling plate portion of the primary culture unit 3 is preferably made of material with light-transmitting properties.

It is more preferable that practically the entire parts of the members of the vessel for cell isolation, culture and proliferation 1, except for the elastic members, cell separation membranes and the like, are made of material with light-transmitting properties.

Mechanism for Adjusting the Position of the Cell Separation Membrane

In one mode, the vessel for cell isolation, culture and proliferation 1 has a mechanism for adjusting the position of the cell separation membrane to break the contact between the surface of the cell separation membrane 11 and the surface of the liquid inside the primary culture vessel. With this positional adjustment mechanism, transition from the cell isolation process to the primary culture process can be performed without opening the main vessel unit. Here, the positional adjustment refers to the adjustment of the position in the vertical direction or substantially vertical direction with respect to the cell separation membrane.

Specifically, an example of the positional adjustment mechanism is a structure in which the main lid portion 56 and the cell separation unit 2 are provided so as to be connectable to each other, and the position of the cell separation membrane can be adjusted in response to the operation of the main lid portion. More specifically, as the main lid portion is rotated, the cell separation unit 2 is detached from the primary culture unit or its position is changed, and as a result, the position of the surface of the cell separation membrane is shifted. An example is a structure with which the position of the surface of the cell separation membrane 11 can move upward in response to the rotation of the main lid portion.

In this mode, the contact between the cell separation membrane 11 and the surface of the liquid inside the primary culture vessel is broken by the operation from outside the main vessel unit, and transition to the primary culture process can be realized without opening the main vessel unit.

Mechanism for Adjusting the Position of the Surface of the Medium in the Primary Culture Vessel

With the vessel for cell isolation, culture and proliferation 1 according to the present invention, transition from the cell isolation process to the primary culture process can be carried out without opening the main vessel unit 4 as the main vessel or the like is provided with a structure 60 through which a hollow tubular member can be inserted or taken out.

In this mode, the hollow tubular member 121 is inserted into the structure 60 for insertion and removal of the hollow tubular member of the main vessel or the like, and the liquid filled in the primary culture unit is suctioned out through the hollow tubular member inserted. As a result, the surface level of the liquid medium filled in the primary culture unit can be lowered without opening the main vessel unit.

As the surface level of the liquid is lowered, the contact between the cell separation membrane 11 and the surface of the liquid in the primary culture vessel is broken, and transition to the primary culture process can be achieved without opening the main vessel unit.

Also, by using this mechanism, the medium can be exchanged in the middle of the primary culture process without opening the main vessel unit.

Mechanism for Controlling the Releasing Structure of the Primary Culture Vessel and the Like

As the vessel for cell isolation, culture and proliferation is provided with an adjustment mechanism with which the releasing structure 28 of the primary culture vessel or the like can be opened by the operation from outside the main vessel unit, transition to the subsequent culture process can be achieved without opening the main vessel unit.

Here, the means and structure for opening the releasing structure without opening the main vessel unit are already described in the paragraphs about the releasing structure in chapter 1 above.

When the primary culture unit 3 is provided with this adjustment mechanism, transition from the primary culture process to the subsequent cell proliferation process can be achieved without opening the main vessel unit.

Also, in one mode when the cell proliferation unit is provided with this adjustment mechanism, transition to another subsequent cell proliferation process can be achieved without opening the main vessel unit.

Mechanism for Adjusting the Vertical Position of the Outer Bottom Wall of the Primary Culture Vessel

In one mode of the vessel for cell isolation, culture and proliferation 1 according to the present invention, the vessel can become compact as the outer wall of the bottom surface of the primary culture vessel 21 is disposed in contact with the culture surface for cell proliferation 55 of the main vessel when the primary culture vessel is stored in the main vessel.

In this mode, however, when the cell proliferation process is performed, it is preferable that the outer wall of the bottom surface of the primary culture vessel 21 is detached or its position is changed in the upward direction from the culture surface for cell proliferation 55 to form a gap or a space between them and to increase the area of the culture surface for cell proliferation 55 on which cells are cultured. Therefore, in this mode, it is preferable to provide a mechanism with which the vertical position of the outer wall of the bottom surface of the primary culture vessel can be adjusted by the operation from outside the main vessel unit 4.

Here, the means and structure for adjusting the vertical position of said outer wall without opening the main vessel unit are already described in the paragraphs in chapter 1 above.

When the primary culture unit 3 is provided with this adjustment mechanism, transition from the primary culture process to the subsequent cell proliferation process can be achieved without opening the main vessel unit. Also, in one mode when the cell proliferation unit is provided with this adjustment mechanism, transition to another subsequent cell proliferation process can be achieved without opening the main vessel unit.

Surface Modification by Plasma Treatment

When a hydrophobic base material is used for the vessel walls or supporting structures of the structural bodies of the vessel for cell isolation, culture and proliferation 1 according to the present invention, the surface of the hydrophobic base material layer can be hydrophilized by plasma treatment. In this mode of performing plasma treatment, the occurrence of surface etching is low, a crosslinked layer is formed in the vicinity of the surface, and as a result, a vessel surface with low occurrence of deterioration with time can be obtained. Especially, when plasma treatment is performed by applying low voltage, the occurrence of surface etching is extremely low, a thick crosslinked layer is formed in the vicinity of the surface, and as a result, a vessel surface with remarkably low occurrence of deterioration with time can be obtained.

In this mode of performing plasma treatment, it becomes possible to produce a vessel in which a crosslinked layer is formed uniformly in the vicinity of the surface of the hydrophobic base material layer.

Examples of the structural bodies of the vessel for cell isolation, culture and proliferation on which plasma treatment can be performed are the structural body 13 of the cell separation unit, the container-like structural body 21 of the primary culture unit, the container-like structural body 41 of the main vessel unit, the main lid portion 56 of the main vessel unit, and the container-like structural body of the cell proliferation unit 5. Plasma treatment can be performed on at least a part of one or more of these structural bodies. Also, plasma treatment can be performed on the entire parts of one or more of these structural bodies. Furthermore, plasma treatment can be performed on the entire parts of all these structural bodies.

The structural bodies of the vessel for cell isolation, culture and proliferation 1 have a structure in which one or more selected from the hydroxyl group, the carboxyl group, and a compound structure including said functional groups are covalently bonded to a vicinity of a surface of a base material layer made of hydrophobic polymers, and/or have an insoluble layer provided in the vicinity of the surface of the base material layer made of hydrophobic polymers, the insoluble layer being insoluble in a good solvent for the hydrophobic polymers. More specifically, the structural bodies constituting the vessel for cell isolation, culture and proliferation have structures in which hydroxyl groups and/or carboxyl groups are covalently bonded.

Plasma treatment on the surface of these structural bodies can be performed in a manner similar to that of the means for surface modification of the culture surface for primary culture 27. Particularly, by treating under low voltage, it becomes possible to suitably form in the vicinity of the membrane surface a layer which is not dissolved even by a solvent (e.g. acetone or the like) which usually has a dissolving power. This layer is regarded as a surface structure in which the occurrence of surface etching is suppressed and on which a crosslinked layer is formed, and serves to maintain the effects of plasma treatment for a long period of time.

### 3. Processes Constituting the Method for Cell Isolation, Culture and Proliferation

The method for cell isolation, culture and proliferation according to the present invention is a method in which a series of operations as described below, including the cell isolation process, the primary culture process, the cell detachment process, and the cell proliferation process, are performed. The method according to the present invention does not exclude the inclusion of processes other than those expressly named as long as the cell isolation and proliferation according to the present invention can be achieved.

### Cell Isolation Process

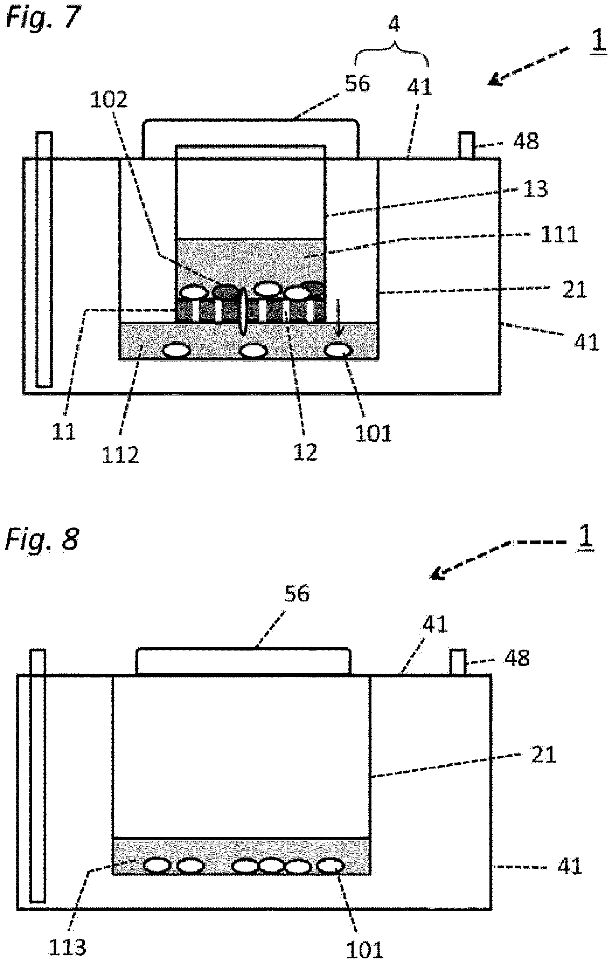

In the method for cell isolation, culture and proliferation according to the present invention, the cell isolation process for selectively isolating the target cells from a culture medium such as a tissue containing various cells is performed. An example of the cell isolation process is shown in FIG. 7.

The cell isolation process is performed as follows: a cell isolation medium 112 is filled in the container-like structural body of the primary culture unit; the cell separation unit 2 is installed so that the surface of the cell separation membrane 11 and the liquid surface of the cell isolation medium 112 are in contact with each other; and the cell suspension containing the cells to be isolated is introduced into the cell separation unit. It is not preferable if there are air bubbles between the surface of the cell separation membrane and the liquid surface of the cell isolation medium, as the yield of cells isolated will be reduced.

The state in which "the membrane surface and the liquid surface are in contact with each other" not only includes a state in which the surface of the cell separation membrane and the entire surface of the liquid filled in the primary culture vessel are in contact with each other, but also a state in which the membrane surface and liquid surface are in contact with each other in a state that the outer side of the membrane holding structure is immersed in the liquid in the primary culture vessel. Also, the arrangement of the two members is not limited to a state in which they are fixed or connected with each other but also a state in which they are engaged with each other, layered, or the like.

With regard to the modes of the cell isolation process according to the present invention, any mode can be employed as long as it can achieve the selective isolation of target cells, and, in order to actively isolate the target cells, it is preferable to employ a means for attracting or activating the movement of the target cells. It is particularly preferable to employ a means for attracting or activating the movement of the target cells by utilizing chemotactic factors.

A liquid medium suitable for the target cells to be isolated 101 can be appropriately employed as the cell isolation medium 112 and the cell suspension medium 111 in the cell isolation process. To which medium the chemotactic factors are added can be decided based on how they are used, as described below.

Specific examples of the modes of utilizing the chemotactic factors in the cell isolation process according to the present invention are as follows.

In one mode of the cell isolation process, (i) a medium containing the chemotactic factors is used as the cell isolation medium to be filled in the primary culture unit. In this mode, when the cell suspension containing the target cells to be isolated is introduced into the cell separation unit, the target cells are selectively attracted by the chemotactic factors in the primary culture unit and move to the primary culture unit through the fine pores of the cell separation membrane.

In another mode of the cell isolation process, (ii) the chemotactic factors are added to the cell suspension before it is introduced into the cell separation unit so that the cell movement activity of the target cells of interest can be activated in advance. Here, the movement activity can be activated in advance by the chemotactic factors as the cell suspension containing the chemotactic factors are cultured for several hours.

In this mode, the movement activity of the target cells is selectively activated by the effects of the chemotactic factors. As a result, the target cells can move to the primary culture unit through the fine pores of the cell separation membrane even when the chemotactic factors are not contained in the cell isolation medium in the primary culture unit.

In this mode, the movement activity of the target cells is activated in advance in the cell suspension before being introduced into the cell separation unit. Therefore, the movement activity of the target cells can be maintained in an activated state even when the medium is washed to remove the chemotactic factors before being introduced into the cell separation unit. That is, in this mode, introduction of the medium into the cell separation unit can be performed in a state in which the chemotactic factors are contained in the medium or in a state in which the chemotactic factors are removed by cleaning the medium.

In a more preferable mode of the cell isolation process, the aforementioned (i) and (ii) are combined - the movement activity of the target cells of interest is activated in advance, and the target cells are attracted by the chemotactic factors in the cell isolation medium in the primary culture unit.

Even more preferably, the movement activity of the target cells of interest is activated in advance, the medium is washed to remove the chemotactic factors, and then, a medium containing chemotactic factors is used as the cell isolation medium to be filled in the primary culture unit. In this mode, the movement of the target cells to the primary culture unit through the fine pores of the cell separation membrane can be achieved more efficiently.

The cell isolation process according to the present invention is performed using chemotactic factors having activity to attract or activate the cells to be isolated so that the target cells of interest can pass through the fine pores of the cell separation membrane.

Chemotactic factors suitable for the target cells of interest can be appropriately used. Preferable examples of chemotactic factors that can be used in the isolation of stem cells are G-CSF, bFGF, SDF-1, TGF-beta, NGF, PDGF, BDNF, GDNF, EGF, VEGF, SCF, MMP3, Slit, GM-CSF, LIF, HGF, SIP, protocatechuic acid, serum, and the like. The concentration of the chemotactic factors can be a concentration with which they can attract the target cells or activate their migration activity, and at the same time, the cell differentiation activity or repelling activity is not exhibited. A preferable example of such concentration is, but not limited to, about 1 to 500 ng/mL.

The concentration of the cells contained in the cell suspension to be introduced into the cell separation unit can be appropriately adjusted depending on the ratio of the target cells contained or the like, and for example, when the membrane area is 0.3 cm$^2$, a concentration of about $3 \times 10^2$ to $3 \times 10^4$ cells / 100 $\mu$L is preferable. It is not preferable if the cell concentration is too high, because clogging or the like is likely to occur.

In the cell isolation process, the size of the fine pores of the cell separation membrane can be selected depending on the type and properties of the target cells to be isolated.

With regard to the culture conditions in the cell isolation process, conditions suitable for the target cells to be isolated can be employed. In one mode, when the main vessel unit has a gas exchange port provided with a sterilizing filter, the cell culture can be carried out in an incubator with a desired atmosphere. It is also possible to carry out the cell culture by injecting a gas of a desired atmosphere into the main vessel unit in advance.

When the progress in the isolation process is assessed, it is desirable to perform microscopic observation through the vessel without opening the lid of the main vessel so as to reduce the risk of infection or contamination.

Primary Culture Process

In the method for cell isolation, culture and proliferation according to the present invention, the target cells isolated in the cell isolation process is subjected to the primary culture process.

The primary culture process corresponds to a preculture step before the proliferation culture and is performed to obtain a cell density at which appropriate intercellular communication is maintained and which is suitable for proliferation culture, the subsequent culture step. FIG. 8 is a schematic view showing an example of the primary culture process.

Though the primary culture process can be performed simultaneously with the cell isolation process by maintaining the contact between the cell separation membrane 11 and the liquid surface of the medium in the primary culture vessel, the primary culture process is preferably performed by breaking the contact between the cell separation membrane and the surface of the medium in the primary culture vessel once it is confirmed that the progress in the cell isolation process has reached a desired level. Preventing the target cells from moving back to the cell separation unit or adhering to the structural body of the cell separation unit can help prevent the loss of cells isolated.

The following modes can be employed as a means for breaking the contact between surface of the cell separation membrane 11 and the surface of the medium in the primary culture vessel.

In one means for breaking the contact, (i) the cell separation unit 2 is physically detached from the primary culture unit 3 or the position of the cell separation unit is changed, and as a result, the contact between the surface of the cell separation membrane and the surface of the medium in the primary culture vessel is broken. It is possible to open the main vessel unit and detach or change the position of the cell separation unit 2 by hand. It is also possible to detach or change the position of the cell separation unit by employing a structure which does not require the opening of the main vessel unit.

In another means for breaking the contact, (ii) the surface level of the medium in the primary culture vessel is lowered, and as a result, the contact between the surface of the cell separation membrane and the surface of the medium in the primary culture vessel is broken. It is possible to lower the surface level of the medium in the primary culture vessel by opening the main vessel unit and performing a suction operation. It is also possible to lower the surface level by employing a structure which does not require the opening of the main vessel unit and performing a suction operation.

The means and structures of aforementioned (i) and (ii) are already described in the paragraphs about the vessel structures in chapters 1 and 2 above.

The primary culture process is performed in the liquid medium filled in the structural body of the primary culture vessel 21. In this process, the isolated cells that have moved to the primary culture unit 3 through the cell separation membrane 11 are cultured on the culture surface for primary culture 27 in the primary culture unit.

A liquid medium suitable for the isolated target cells can be appropriately employed as the primary culture medium 113 for performing the primary culture process. When chemotactic factors are contained in the cell isolation medium, it is preferable that, before proceeding to the primary culture, the cell isolation medium containing the chemotactic factors is removed and changed to a fresh liquid medium that does not contain chemotactic factors, and afterwards, the primary culture is performed.

With regard to the culture conditions in the primary culture process, conditions suitable for the isolated target cells can be employed. In one mode, when the main vessel unit has a gas exchange port provided with a sterilizing filter, the cell culture can be carried out in an incubator with a desired temperature atmosphere. It is also possible to carry out the cell culture by injecting a gas of a desired atmosphere into the main vessel unit in advance. When the progress in the culture process is assessed, it is desirable to perform microscopic observation through the vessel without opening the lid of the main vessel so as to reduce the risk of infection or contamination.

It is preferable to carry out the transition from the primary culture process to the subsequent cell proliferation process when the cultured cells reach 50 to 100% confluence, preferably 60 to 80% confluence, at which an appropriate cell density is maintained. Particularly, confluence of 70% or less is preferable for cells sensitive to stimuli, such as stem cells.

When the cell culture proceeds to a certain extent in the primary culture process, it is preferable to detach the cells from the culture surface for primary culture 27 of the primary culture unit, reseed the cells on the same culture surface to obtain a more uniform state, and continue the primary culture process.

Specifically, in the primary culture process, the cells that have passed through the cell separation membrane tend to exist unevenly, concentrated on the area of the culture surface below the cell separation membrane. Especially, when the target cells isolated are adhesive cells, it is difficult to achieve uniform dispersion of the cells only by permeation or the like. Therefore, it is preferable to detach the cells in the middle of the cell culture process and reseed them in order to avoid the negative effects caused by cells overcrowded in certain areas. This mode is preferable in that intercellular communication can be maintained in a preferable state, cells of high quality can be obtained, and the efficiency of proliferation culture improves.

Here, the cell detachment operation is performed by the method described in the below-mentioned paragraphs about the cell detachment process.

Cell Detachment Process

In the method for cell isolation, culture and proliferation according to the present invention, a process for detaching cells from the culture surface is performed between or after cell culture processes so as to transfer the cells to the outside of the unit in which the cells have been cultured. That is, in the method according to the present invention, cell detachment processes are preferably performed after the cell culture in the primary culture unit 3, after the cell culture in the main vessel unit 4 and, if the cell proliferation unit is installed, after the cell culture in the cell proliferation unit 5.

Any means can be employed as the means for detaching the cells from the culture surface as long as it does not cause safety concerns regarding the survival or the canceration of the cells, and the examples thereof are physical detachment, enzymatic detachment, and chemical detachment. Yet, in order to minimize the damage or effects on the target cells as much as possible, the cell detachment operation is preferably performed using a culture surface made of a base material whose surface is modified with molecules exhibiting cell detachment properties.

Especially, the culture surface for primary culture 27 is preferably made of a base material whose surface is modified with molecules exhibiting cell detachment properties, as the yield of the target cells isolated is not sufficient yet after the primary culture process.

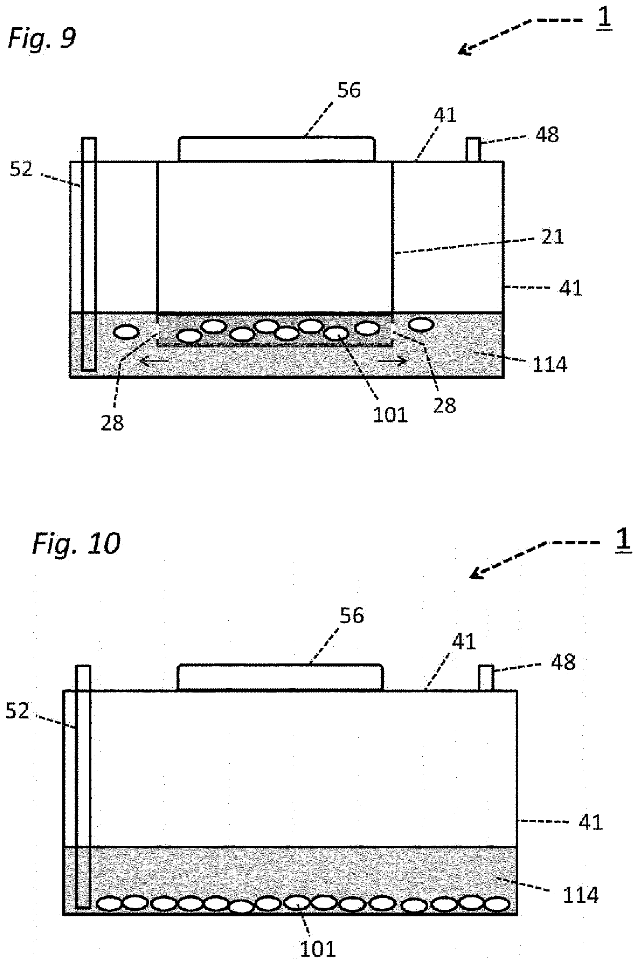

When the progress in the cell detachment process is assessed, it is desirable to perform microscopic observation through the vessel without opening the lid 56 of the main vessel so as to reduce the risk of infection or contamination. FIG. 9 is a schematic view showing an example of the cell detachment process.

The cells that are detached from the culture surface and suspended in the medium in the cell detachment process can be released to the outside of the culture unit as the releasing structure provided at the container-like structural body of the culture unit is adjusted to open. For example, in the primary culture process, the cells suspended in the medium can be released together with the medium to the main vessel unit (or the cell proliferation unit) through the releasing structure 28 of the primary culture vessel.

The means and structures for opening the releasing structure are already described in the paragraphs about the vessel structures in chapters 1 and 2 above.

Cell Proliferation Process

In the method for cell isolation, culture and proliferation according to the present invention, the cell proliferation process is performed after conducting the cell detachment process after the primary culture process. The cell proliferation process corresponds to the main culture step for increasing the yield of target cells. FIG. 10 shows a schematic view of an example of the cell proliferation process.

It is desirable to start the proliferation culture after the cells in the primary culture process have reached a density at which appropriate intercellular communication can be maintained and which is suitable for proliferation culture.

The cell proliferation process is performed in the liquid medium filled in the container-like structural body of the main vessel unit 4 or the cell proliferation unit 5. After the cell detachment process, the cells that have been cultured in the preceding culture process can be transferred only by discharging these cells through the releasing structure of the culture unit for the preceding step into the liquid medium filled in the main vessel unit or the cell proliferation unit.

The cells transferred are cultured on the culture surface of the main vessel unit or the cell proliferation unit.

A liquid medium suitable for the isolated target cells can be appropriately employed as the liquid medium for proliferation culture 114.

The cell proliferation process can be performed in a state in which the culture unit 3 or culture units 3 and 5 for the preceding step/steps are detached from the main vessel unit 4 in order to ensure a sufficient area for cell culture and improve the operability in medium exchange or the like. The cell proliferation process can also be performed in a state in which the culture unit 3 or culture units 3 and 5 for the preceding step/steps are detached or their position is changed in the upward direction. Such a state can be realized by, for example, employing the structure that can be adjusted in response to the rotation of the main lid portion as mentioned in paragraphs above.

With regard to the culture conditions in the cell proliferation process, conditions suitable for the target cells can be employed. In one mode, when the main vessel unit 4 has a gas exchange port 47 provided with a sterilizing filter, the cell culture can be carried out in an incubator with a desired atmosphere. It is also possible to carry out the cell culture by injecting a gas of a desired atmosphere into the main vessel unit in advance.

The proliferation culture can be terminated when the amount of target cells reaches a desired level.

It is desirable to exchange the liquid medium to a fresh one in the middle of the cell proliferation process and continue the cell culture. In this regard, the main vessel unit 4 is preferably provided with a medium exchange port 51.

When the progress in the proliferation process is assessed, it is desirable to perform microscopic observation through the vessel without opening the lid of the main vessel so as to reduce the risk of infection or contamination.

In a basic mode, the cell proliferation process is performed only once using the main vessel unit 4 after the primary culture process.

In another mode, the cell proliferation process can be performed a plurality of times. Specifically, in one mode, for example, a cell proliferation process using the cell proliferation unit 5 is performed after the primary culture process, and then, the final proliferation culture step is performed using the main vessel unit 4. Moreover, the cell proliferation process can be performed three times or more by disposing two or more cell proliferation units 5 in a nested manner.

Also, if desired, the proliferation culture can be performed by employing special culture conditions suitable for desired cell treatment (induction, differentiation, or the like) as part of the culture processes.

Examples

Hereinafter, the present invention will be explained in detail by way of examples, but the scope of the invention is not limited thereto.

The term "G-CSF" as used herein refers to granulocyte-colony stimulating factor, which is a cytokine, and is used as a chemotactic factor for the stem cell. The term "FBS" as used herein refers to fetal bovine serum.

Unless otherwise stated, the operations performed by opening the vessel in the following experiments were carried out in a clean bench in order to maintain sterilized conditions.

[Example 1] Vessel for Cell Isolation, Culture and Proliferation

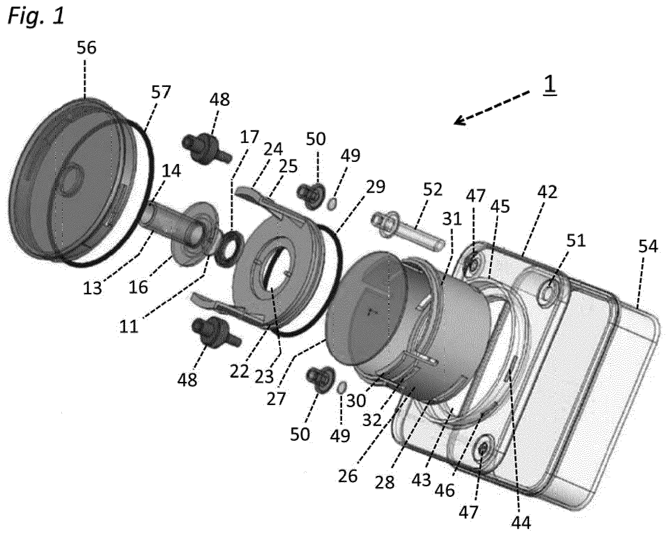

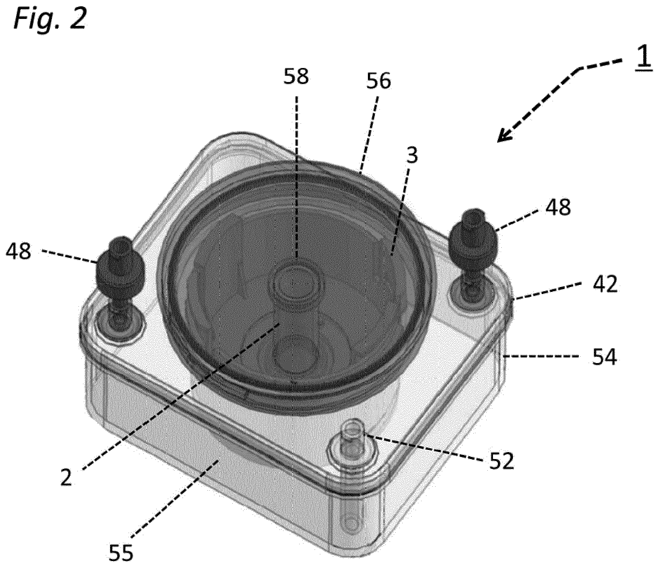

A container-like structural body as shown in the figures was produced as one embodiment of the vessel for cell isolation, culture and proliferation 1 according to the present invention. Hereinafter, details about the vessel for cell isolation, culture and proliferation produced in this example will be explained with reference to the exploded view (FIG. 1), the component drawings (FIGS. 2 to 6) and the like.

(1) Entire Structure

The vessel for cell isolation, culture and proliferation produced in this example is a container-like structural body mainly composed of a cell separation unit 2, a primary culture unit 3, and a main vessel unit 4.

(2) Cell Separation Unit

The cell separation unit 2 in this example is a structural body including a cylindrical structure 13, a cell separation membrane 11 and a ring structural body 17. In this example, the ring structural body 17 serves as an auxiliary member for the cylindrical structure 13, which is a membrane holding structure. The cylindrical structure 13 is a light-transmitting transparent member made of polystyrene and is a cylindrical-shaped structural member having an outer diameter of 12 mm, an inner diameter of 10 mm, and a tube length of 30 mm. A ring structural portion with a ring thickness of 10 mm is formed around the circumference of the bottom surface of the cylindrical structure.

The ring structural body 17 is a holed member with an inner diameter of 10 mm provided with an opening portion 18 at its center.

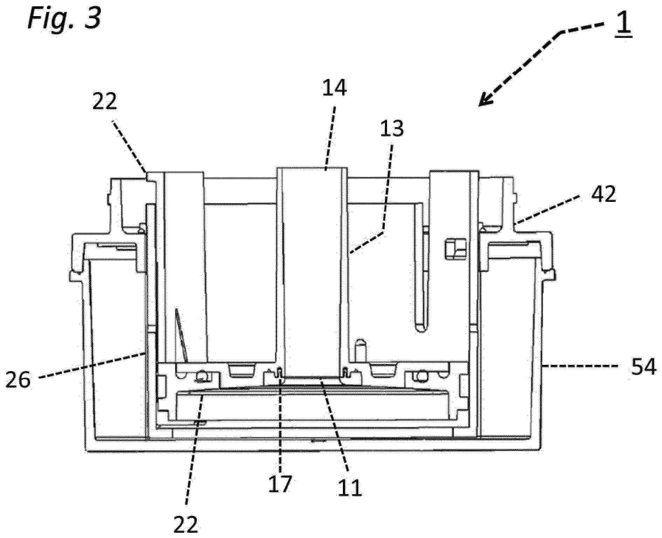

The bottom surface of the cylindrical structure has a structure having a gentle inclination directed upward toward the opening portion so that air bubbles may not occur between the cell separation membrane 11 and the liquid surface of the medium filled in the primary culture unit when they are in contact with each other (FIG. 3).

The cell separation membrane 11 is a membrane structure which has fine pores serving as cell-isolation pores 12, whose base material layer is made of PE, and whose surface has been subjected to molecular modification with hydrophilic polymers. It is a membrane member having a membrane thickness of 20 $\mu$m and an outer diameter of 18 mm.

The treatment for forming fine pores on the membrane was performed by preparing a fine die and conducting the imprinting method, following the description of WO2014/171365. The cell separation membrane 11 in this example has a fine structure in which fine pores having an average diameter of 8 $\mu$m are regularly arranged in a lattice pattern with a porosity of 20%. This pore size is suitable for isolating stem cells.

The cell separation membrane 11 was produced by subjecting the membrane surface to covalent bonding with hydrophilic polymers following the description of WO2012/133803. Specifically, the PE membrane on which pores were formed by the imprinting method was immersed into a solution containing 1,000 ppm of polyvinylpyrrolidone-polyvinyl acetate copolymers and 0.1 wt% of ethanol, sealed and treated with 25 kGy of gamma irradiation. In the cell separation membrane 11 produced in this example, the surface properties of the membrane were improved and cell adhesion to the membrane surface was suppressed. The results of the evaluation test on the cell separation membrane of this example are shown in Example 3.

When the cell separation unit 2 is assembled, the bottom surface 16 of the cylindrical structure and the ring structural body 17 are engaged with and fixed to each other, while the cell separation membrane 11 is sandwiched and held between the bottom surface 16 of the cylindrical structure and the ring structural body 17.

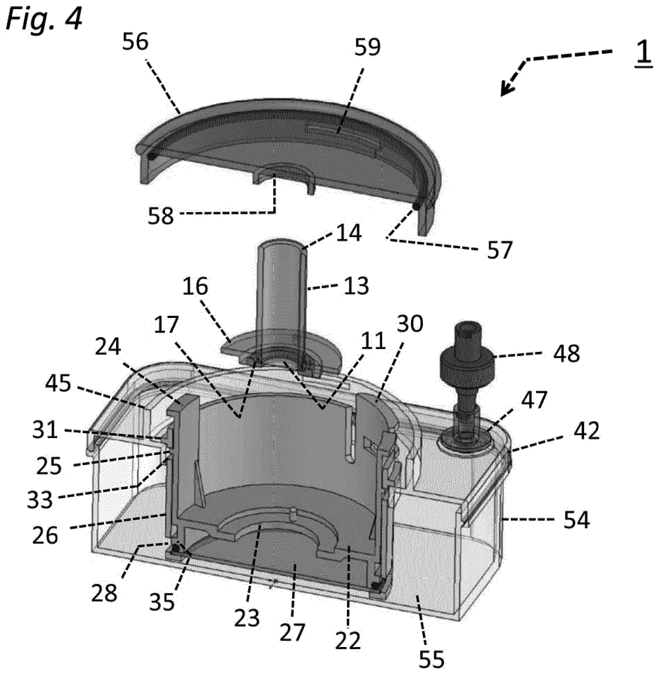

Structurally, the liquid medium and the like introduced into the cylindrical structure from its upper opening portion 14 cannot reach the primary culture unit 3 unless it passes through the cell separation membrane 11 (FIGS. 4 and 7).

In addition to having the structure for engagement with the ring structural body 17, the bottom surface 16 of the cylindrical structure has an engaging structure that enables the cylindrical structure to be layered onto the upper central portion of the primary culture vessel 21.

(3) Primary Culture Unit

The primary culture unit 3 in this example is a structural body mainly composed of a primary culture vessel 21. In this example, the primary culture vessel 21 is a member that can be separated into the upper structural body 22 and the lower structural body 26.

The lower structural body 26 of the primary culture vessel is a light-transmitting member made of polystyrene and is a cylindrical member having an outer diameter of 61 mm (64 mm including the fitting structure), an inner diameter of 58 mm, and a tube length of 36.5 mm (41.5 mm including the grip portion). The bottom surface portion of this cylindrical structure is provided with an inner frame having a width of 3 mm for holding a flat plate member that serves as a culture surface for primary culture 27. Also, slit-shaped releasing windows 28, each having widths of 4 mm in the vertical direction and 20 mm in the horizontal direction, are formed in the lower portion of the side surface of the lower structural body 26. The releasing windows 28 are formed at two positions opposing to each other in the cross-sectional view of the cylinder.

Locking holes 33 to be engaged with the projection structures 25 of the upper structural body 22 are formed in the lower structural body 26 of the primary culture vessel. The locking holes 33 are formed at two positions opposing to each other in the cross-sectional view of the cylinder.

A grip portion 30 is formed on the lower structural body 26 by forming vertical grooves at a 2 cm interval and forming the portion between the grooves to be 5 mm higher than other parts of the cylinder. The grip portions 30 are formed at two positions opposing to each other in the cross-sectional view of the cylinder. In this example, as moderate strength of physical pressure is applied to the grip portions 30, the outer shape of the vessel slightly changes or distorts, and the upper structural body can be easily detached.

A locking hole 32 to be engaged with the projection structure 44 of the main vessel of the main vessel unit is formed in the grip portion 30. The locking holes are formed at two positions opposing to each other in the cross-sectional view of the cylinder.

The upper vessel portion of the upper structural body 22 is a lid-like cylindrical structure having an outer diameter of 57 mm, an inner diameter of 50 mm, an outer height of 9.1 mm and an inner height of 7.1 mm. It is composed of a light-transmitting member made of polystyrene. An O-ring 29, which is an elastic member for securing airtightness of the primary culture vessel, is provided at the bottom portion of the outer periphery of the cylindrical portion. The bottom portion of the outer periphery functions as a blocking member of a releasing structure 35.

The upper part of the upper structural body 22 has a canopy structure which is flat-shaped as a whole, and has an opening portion 23 with an inner diameter of 22.4 mm at the central portion of the structure. A structure that can be engaged with the bottom surface portion 16 of the cylindrical structural body of the cell separation unit is provided on the circumference of the opening portion 23.

A grip portion 24, which has a width of 20 mm and is 29.7 mm higher than the adjacent parts of the cylinder, is formed on the outer edge of the upper part of the upper structural body 22. The grip portions 24 are formed at two positions opposing to each other in the cross-sectional view of the cylinder. As moderate strength of physical pressure is applied to the grip portions 24, the shape of the vessel slightly changes or distorts, and the lower structural body 26 can be easily attached to or detached from the main vessel unit.

A projection structure 25 that can be engaged with the locking hole 33 of the lower structural body 26 is provided at a position located 10.5 mm apart from the upper end of the grip portion 24. The projection structures 25 are formed at two positions opposing to each other in the cross-sectional view of the cylinder.

The flat plate member 27 which serves as the culture surface for primary culture is a plate for cell culture whose surface is modified to exhibit cell detachment properties and is a member constituting the culture surface for primary culture. In this example, the flat plate member 27 is a light-transmitting member made of polystyrene resin and is a circular plate member which has an outer diameter of 57 mm and whose base material has a thickness of 1 mm.

The base material of the flat plate member 27 has a structure whose surface is modified by graft polymerization between the poly(N-isopropylacrylamide), which is a temperature-responsive polymer, and the molecules of the base material. In this example, UpCell (registered trademark) manufactured by CellSeed Inc. was used as the flat plate member for primary culture.

The results of the evaluation test on the cell detachment properties of the flat plate member 27, which is the culture surface for primary culture of this example, are shown in Example 4.

When the primary culture unit 3 is assembled, the flat plate member 27, which is the culture surface for primary culture, is fitted to the inner bottom portion of the lower structural body 26, and the upper structural body 22 is further placed thereon as shown in FIG. 4.

Here, the flat plate member 27, which is the culture surface for primary culture, has a structure sandwiched by the inner frame of the bottom portion of the lower structural body 26 and the side surface of the upper structural body 22, being supported by the O-ring 29, which is an elastic member. Therefore, when the upper structural body 21 is inserted to the lowest position, the releasing structure 28 of the lower structural body 26 is closed, realizing a structure with which the liquid medium or the like inside can be maintained. In this example, the bottom portion of the side surface of the upper structural body serves as the member 35 for blocking the releasing structure.

With this structure of the primary culture unit in this example, the inner portion of the assembled structure of the primary culture vessel 21 can practically have an inner diameter (ø 50 mm) with which cells can be cultured. The bottom surface area on which cells can be cultured in this vessel is practically 19.62 cm$^2$. When the upper structural body 22 is moved upward, the releasing windows 28 open.

(4) Main Vessel Unit

The main vessel unit 4 in this example is a container-like structural body mainly composed of a main vessel 41 and a main lid portion 56.

The main vessel in this example is composed of an upper structural body 42 and a lower structural body 54.

The lower structural body 54 is a container-like member having a substantially cuboid shape and is a light-transmitting member made of polystyrene. The bottom surface inside the lower structural body 54 serves as a culture surface for cell proliferation 55. The inner size of the bottom surface of the lower structural body is 97.5 mm $\times$ 81.5 mm, and the bottom surface area is 78.6 cm$^2$. This area is about 4 times larger than the culture surface area of the primary culture unit. The outer size of the main vessel 41 is 98 mm $\times$ 83 mm. The vessel has an inner height of 30 mm and an outer height of 34.2 mm.

The upper structural body 42 is a member provided with an opening portion 43 at its central portion through which a culture unit can be inserted or taken out. The upper structural body is fitted and fixed to the lower structural body 54 and serves as a member constituting the upper peripheral portion of the main vessel. It is a light-transmitting member made of polystyrene. In this example, the upper structural body is adhered and fixed to the lower structural body 54 so as to secure airtightness.

The opening portion 43 is a circular opening structure having an inner diameter of 61.4 mm, and the inner frame forming the opening portion has protrusions 44 to be engaged with the primary culture unit, which is the culture unit for the preceding step. The protrusions 44 are formed at two positions opposing to each other in the cross-sectional view of the cylinder. A short cylindrical structure extending from the upper portion of the main vessel is formed at the outer edge of the opening portion 43. This short cylindrical structure functions as a fitting structure 45 to be fitted and connected to the main lid portion 56. The outer surface of the fitting structure of the short cylindrical structure has protrusions 46 to be engaged with the main lid portion 56. The protrusions 46 are formed at two positions opposing to each other in the cross-sectional view of the cylinder.

The upper structural body 42 has a medium exchange port 51, and a tube for medium exchange 52, with which the medium for cell proliferation can be exchanged easily, is connected to the medium exchange port. The tube for medium exchange 52 is a pipe-like structure having an inner diameter of 3.6 mm, an outer diameter of 7.6 mm, and a cylindrical length of 40.2 mm, and is fixed so that the lower end of the tube is located 0.1 to 0.2 mm apart from the bottom surface of the main vessel 41. The main vessel unit 4 can be kept airtight as a cap 53 is attached to the tube for medium exchange 52.

The upper structural body 42 has a gas exchange port 47 to which a gas exchange channel 48 having a sterilizing filter 49 is connected. In this example, the gas exchange ports 47 are formed at two positions on the upper structural body 42.

The main lid portion 56 is a lid-like member which can be engaged with the upper structural body 42 of the main vessel. It is a cylindrical lid structure having an outer diameter of 81.2 mm, an inner diameter of 74 mm, an outer height of 10.8 mm and an inner height of 8.8 mm. It is a light-transmitting member made of polystyrene.

The upper surface of the main lid portion 56 has a flat circular plate shape. Locking holes 59 that can be engaged with the protrusions 46 of the fitting structure 45 of the upper structural body are provided on the side surface structure of the main lid portion 56. Further, an O-ring 57 for securing airtightness is provided at the upper inner portion of the side surface structure of the main lid portion 56.

A short cylindrical structure 58 that can be fitted to, be in contact with and fix the upper surface of the cylindrical structure 13 of the cell separation unit is provided at the central portion of the bottom surface of the main lid portion 56.

When the main vessel unit 4 is assembled, the lower structural body of the main vessel 54 and the upper structural body of the main vessel 42 are adhered and fixed to each other in a sealed state as shown in FIG. 2. At the upper structural body, the tube for medium exchange 52 is connected to the medium exchange port 51, and the gas exchange channel 48 provided with a ventilation filter 49 is connected to the gas exchange port 47.

The main lid portion 56 is fitted to the main vessel as the fitting structure 45 of the upper structural body 42 of the main vessel and the O-ring 57 are fitted to each other, and as a result, the entire vessel can be sealed. The main vessel unit can seal the entire vessel in a state in which the cell separation unit 2 and the primary culture unit 3 are stored in the main vessel unit.

[Example 2] Isolation and Proliferation of Stem Cells

The cell isolation process, the primary culture process, the cell detachment process and the cell proliferation process were performed on stem cells using the vessel for cell isolation, culture and proliferation produced in the example above.

(1) Cells and Mediums

Tissue cells and liquid mediums as shown in the table below were used in this example. Gentamicin and amphotericin were used as the antibiotics. The G-CSF was used as the chemotactic factor.
Table 2

| Tissue cells and liquid mediums used in this example | |
|---|---|
| Tissue cells used as the experimental material | $2 \times 10^4$ cells of the dental pulp tissue of the dog canine tooth |

(continued)

| Tissue cells and liquid mediums used in this example | |
| --- | --- |
| Cell suspension medium | DMEM medium containing antibiotics |
| Cell isolation medium | DMEM medium containing antibiotics, 10% of autoscrum, and 100ng/ml of chemotactic factors |
| Primary culture medium | DMEM medium containing antibiotics and 10% of autoserum |
| Medium for cell proliferation | DMEM medium containing antibiotics and 10% of autoserum |

(2) Cell Isolation Process

The cell separation unit 2, the primary culture unit 3 and the main vessel unit 4 were assembled and prepared as shown in FIGS. 1 and 2 by using the vessel for cell isolation, culture and proliferation 1 produced in Example 1, and stem cells were isolated from the dental pulp tissue of the dog canine tooth. FIG. 7 is a schematic diagram explaining the cell isolation process in this example.

As shown in FIG. 4, the cell separation unit 2 and the main lid portion 56 were removed from the vessel for cell isolation, culture and proliferation 1, the cell isolation medium containing the chemotactic factors was introduced into the primary culture vessel through the opening portion 23 of the upper structural body of the primary culture vessel, and air bubbles were expelled from the opening portion 23.

The cell separation unit 2 was placed on the upper side of the opening portion 23 and engaged and fixed thereto, so that the cell separation membrane 11 would be in contact with the liquid surface of the cell isolation medium without the occurrence of air bubbles.

Then, the cell suspension medium in which the dental pulp tissue cells of the dog canine tooth were suspended was introduced from the opening portion 14 on the upper side of the membrane holding structure, the main lid portion 56 was attached, and cells were cultured in an incubator at 37°C at a

$CO_2$ concentration of 5% for 48 hours. The two gas exchange ports 47 were kept open, and the medium exchange port 51 was kept closed.

After this cell isolation process, the cells were observed from the upper side of the vessel using a phase-contrast microscope without opening the main lid portion 56. As a result, about 70 to 280 cells isolated were observed on the culture surface for primary culture 27. Cell adhesion to the cell separation membrane 11 was not observed. FIG. 11A shows a photographic image of the cells after this cell isolation process.

(3) Primary Culture Process

After the cell isolation process described in section (2) above, the primary culture process was performed to increase the number of cells to a predetermined level. FIG. 8 is a schematic view explaining the primary culture process in this example.

After the cell isolation process, the main lid portion 56 was opened, the cell separation unit 2 and the main lid portion 56 were taken out as shown in FIG. 4, and the cell isolation medium containing chemotactic factors was changed to the primary culture medium through the opening portion 23 of the upper structural body of the primary culture vessel. Then, the main lid portion 56 was closed, and the cells were cultured in an incubator at 37°C at a $CO_2$ concentration of 5% until they reached 60 to 70% confluence.

After culturing, the cells were observed from the upper side of the vessel using a phase-contrast microscope without opening the main lid portion 56. It was observed that stem cells were cultured on the culture surface for primary culture 27. It was confirmed that the cells were cultured to an amount sufficient for the subsequent proliferation culture when the ratio of the bottom surface area of the culture surface for primary culture to the bottom surface area of the culture surface for cell proliferation (55; culture surface for secondary culture) of the main vessel in this example was taken into consideration. FIG. 11B shows a photographic image of the cells after the primary culture process.

(4) Cell Detachment Process

After the primary culture process described in section (3) above, the cells cultured were detached from the culture surface for primary culture. FIG. 9 is a schematic diagram explaining the cell detachment process in this example.

After the primary culture process mentioned above, the main lid portion 56 was opened, the culture medium in the primary culture vessel was exchanged through the opening portion 23 of the upper structural body of the primary culture vessel, and the main lid portion 56 was sealed again.

Then, the entire vessel was cooled at 4°C for 60 minutes, so that the temperature would be lower than the phase transition temperature of the surface modification molecules of the culture surface for primary culture 27, and the culture surface was observed from the upper side of the vessel using a phase-contrast microscope to determine the degree of cell detachment.

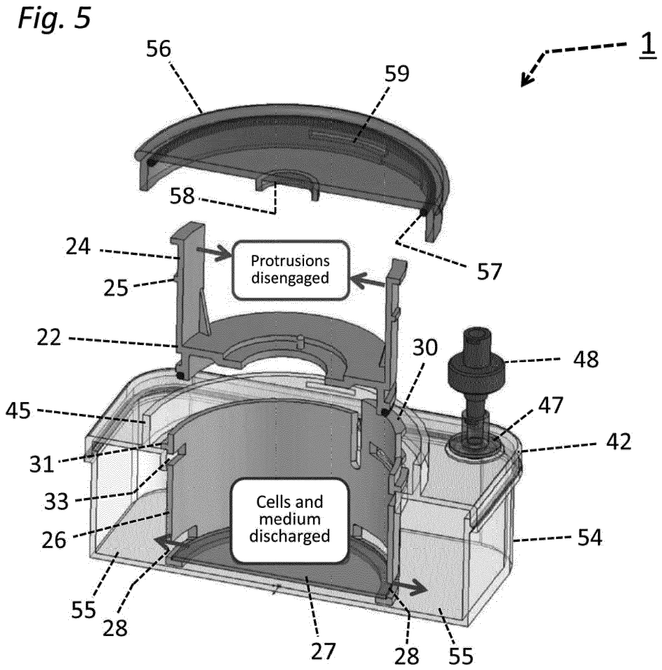

Subsequently, as shown in FIG. 5, the main lid portion 56 was opened, the upper structural body 22 of the primary culture vessel was detached to open the releasing windows 28 on the side surface of the lower structural body of the primary culture vessel, and as a result, the liquid medium in which the detached cells were suspended was released into the main vessel 41. The culture surface for primary culture 27 was rinsed again using a liquid medium so that the rest of the cells would be completely decanted into the main vessel 41.

The ratio of the cells detached from the culture surface for primary culture after this process was 90% or more. FIG. 11C shows a photographic image of the cells after the cell detachment process.

(5) Cell Proliferation Process

After the process described in section (4) above, the secondary culture was performed for cell proliferation. FIG. 10 is a schematic diagram explaining the cell proliferation process in this example.

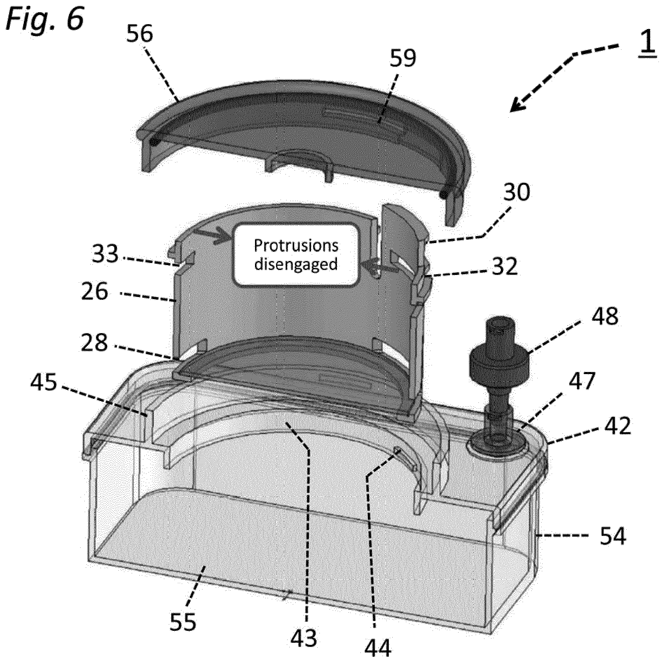

After a series of operations in the cell detachment process, the lower structural body 26 of the primary culture vessel was taken out as shown in FIG. 6, the main lid portion 56 was sealed, and the entire vessel was shaken so that the cells would be dispersed evenly on the bottom surface. The cells were then cultured in an incubator at 37°C at a $CO_2$ concentration of 5%.

After the proliferation culture, the cells were observed from the upper side of the vessel using a phase-contrast microscope without opening the main lid portion 56. It was confirmed that the stem cells sufficiently adhered to the culture surface and proliferated. FIG. 11D shows a photographic image of the cells after the cell proliferation process.

(6) Evaluation of Cell Characteristics

The characteristics of the cells which were isolated and proliferated in the series of operations and processes described in sections (1) to (5) above were evaluated.

Flow cytometric analysis of CD105, G-CSFR and CXCR4, which are stem cell surface markers, was performed using their antibodies. Dental pulp tissue cells of the dog canine tooth which were not subjected to the isolation process in this example were used as a control, and, by the flow cytometric analysis, their cell characteristics were compared with those obtained by the method for cell isolation, culture and proliferation of this invention. The positive rates of the stem cell markers are shown in Table 3.

The cells obtained by the series of operations and processes described in sections (1) to (5) had higher positive rates of stem cell markers compared with the untreated control cells and indicated the characteristics of stem cells.

It was also confirmed that the characteristics of stem cells were not affected even when the cell detachment process using the temperature-responsive polymers was performed after the primary culture process.

Table 3

| Antigen markers | Positive rates of stem cell markers (%) | |
| --- | --- | --- |
| | Cells obtained in the present invention | Control |
| CD105 | 98.8 | 100.0 |
| G-CSFR | 24.7 | 18.0 |
| CXCR4 | 15.0 | 7.2 |

(7) Examples of Applications

While dental pulp tissue cells of the dog canine tooth were used as the experimental material in this example as described in section (1) above, it is thought to be possible to isolate stem cells directly from bone marrow tissue or adipose tissue and let them proliferate using the vessel in this example, when the pore size of the fine pores of the cell separation membrane 11, the characteristics of the chemotactic factors and the description in WO2012/133803 are taken into consideration.

[Example 3] Evaluation of the Cell Separation Membrane

Evaluation was carried out on the cell separation membrane of the cell separation unit of the vessel for cell isolation, culture and proliferation produced in the example above, with regard to its microstructure and ability to suppress cell adhesion.

(1) Evaluation of the Microstructure

The microstructure of the cell separation membrane of the cell separation unit produced in the example above was evaluated.

The surface microstructure of the cell separation membrane produced by the imprinting method in Example 1-(2) was observed by a scanning electron microscope (SEM). FIG. 12 shows the microscopic images of the cell separation membrane.

It was confirmed that the cell separation membrane produced in the example above was a membrane having a microstructure on which fine pores with an average pore size of 8 $\mu$m were arranged with a porosity of 20%. Moreover, since the pores penetrated the membrane surface almost vertically and were regularly arranged in a lattice pattern with the same intervals, the enlargement of the fine pores due to the overlapping of the pores was not observed.

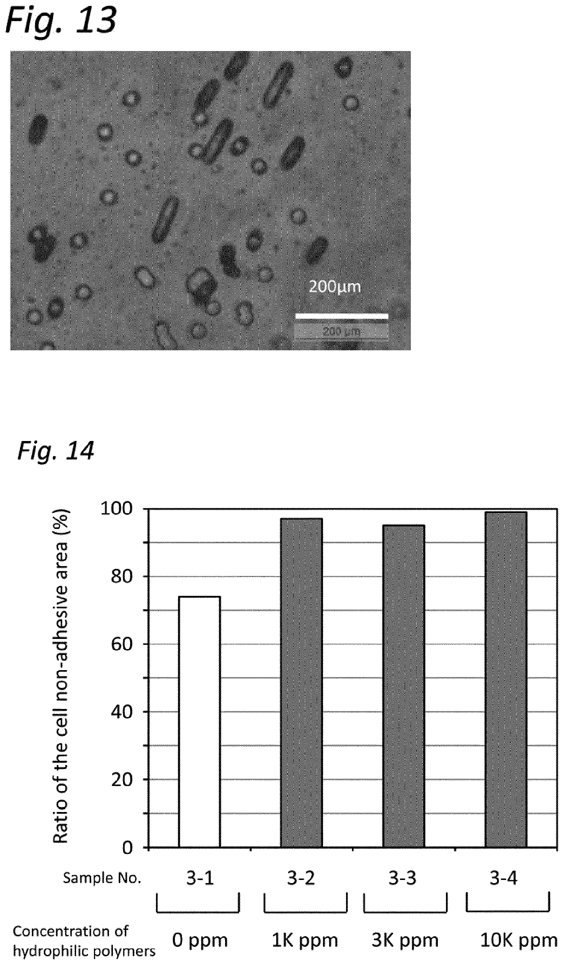

In contrast, when a control membrane which was produced by forming fine pores on PE by a conventional method employing heavy particle beams was observed, the pores were arranged randomly, large pores existed due to the overlapping of the pores, and moreover, where the particles did not penetrate vertically, the pores had elliptic shapes (FIG. 13). This phenomenon became worse as the porosity increased.

As apparent from its microstructure, the cell separation membrane produced in the example above is a membrane which has a remarkable cell-size selectivity and an excellent transmission efficiency. It should also be noted that the pore size is suitable especially for the isolation of stem cells.

(2) Evaluation of the Function of Suppressing Cell Adhesion

The function of suppressing cell adhesion exhibited by the cell separation membrane of the cell separation unit produced in the example above was evaluated.

The difference in the function of suppressing cell adhesion due to the different concentrations of hydrophilic polymers was evaluated on the cell separation membranes produced by the imprinting method in Example 1-(2).

The PE membranes on which pores were formed by the imprinting method in Example 1-(2) were treated with 25 kGy of gamma irradiation in a state in which they were immersed in the solutions containing polyvinylpyrrolidone-polyvinyl acetate copolymers at concentrations shown in Table 4 and 0.1 wt% of ethanol.

The cell separation membranes obtained by the surface modification with hydrophilic polymers were adhered to the polystyrene cell culture inserts for a 24-well plate, and stem cells contained in a cell isolation medium were seeded and cultured in an incubator at a $CO_2$ concentration of 5% for 48 hours.

In this example, the third passage of stem cells of FI65N DPSCs derived from the dental pulp tissue of the dog canine tooth were seeded at $2 \times 10^4$ cells per well. A DMEM medium containing 10% FBS was used as the culture medium.

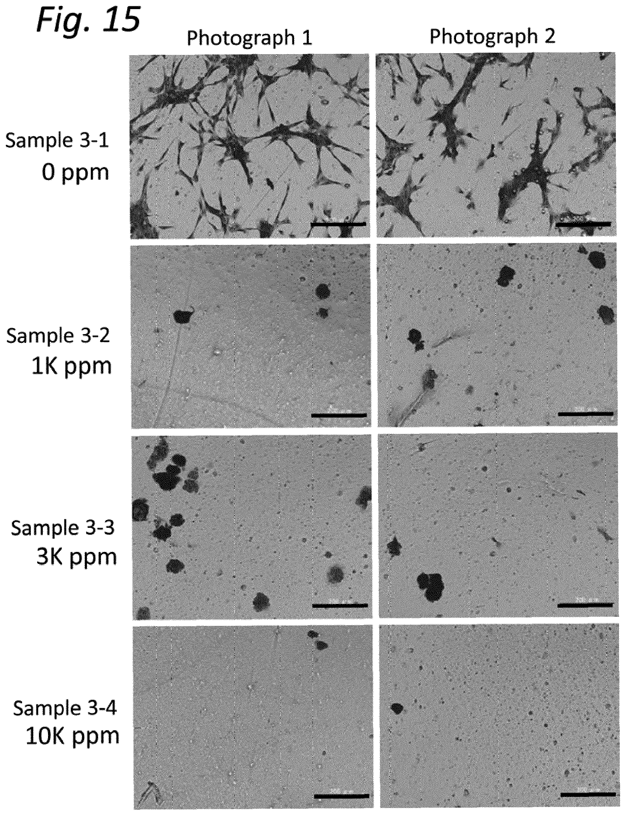

After the culturing, membranes were taken out of the inserts and Giemsa stained, and the number of the cells (nuclei) and the total area of the cells were measured using the image analysis software ImageJ. The ratio of the area (%) of the PE membrane to which the cells did not adhere was calculated by the following formula (1). Table 4 and FIG. 14 show the results. FIG. 15 shows the microscopic images of the cell separation membranes.

Formula (1):

Ratio of the cell non-adhesive area (%)

$$= \text{(Total image area} - \text{Total cell area)} \, / \, \text{Total image area} \times 100$$

The results show that, by conducting the surface treatment on PE membranes using solutions containing hydrophilic polymers at concentrations of 0.1% or more, the characteristics of the PE membrane surface improve drastically, and the function of suppressing cell adhesion to the cell separation membrane is sufficiently exhibited (Samples 3-2 to 3-4).

Table 4

| Samples | Concentration of hydrophilic polymers | Ratio of the cell non-adhesion area of the PE membrane |
|---|---|---|
| 3-1 | 0 ppm | 74 % |
| 3-2 | 1000 ppm | 97 % |
| 3-3 | 3000 ppm | 95 % |
| 3-4 | 10000 ppm | 99 % |

[Example 4] Evaluation on Cell Detachment

The cell detachment activity of the culture surface for primary culture of the primary culture unit of the vessel for cell isolation, culture and proliferation produced in the example above was evaluated.

The plate-like structural body (a plate having cell detachment activity), which is the culture surface for primary culture described in Example 1-(3), was placed on a cell culture dish. This plate-like structural body is a plate whose surface has a structure modified with the temperature-responsive polymer poly(N-isopropylacrylamide).

Stem cells contained in a medium were seeded on the plate-like structural body and cultured in an incubator at a $CO_2$ concentration of 5% for 24 hours. In this example, the fourth passage of stem cells of FI65N DPSCs derived from the dental pulp tissue of the dog canine tooth was seeded at $4 \times 10^4$ cells per plate (ø 35 mm). A DMEM medium containing 10% FBS was used as the culture medium.

After the culturing, the plate was cooled at 4°C for 60 minutes, so that the temperature would be lower than the phase transition temperature of the surface modification molecules on the plate surface. The surface of the plate-like structural body was observed and the number of suspended cells (the number of non-adhesive cells) was counted immediately before the initiation of the low-temperature culture, 30 minutes after the initiation of the low-temperature culture, 60 minutes after the initiation of the low-temperature culture, and after the pipetting operation after the low-temperature treatment.

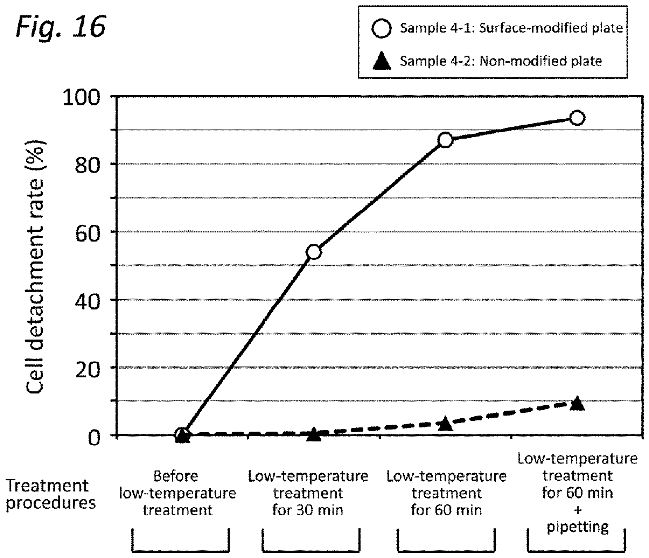

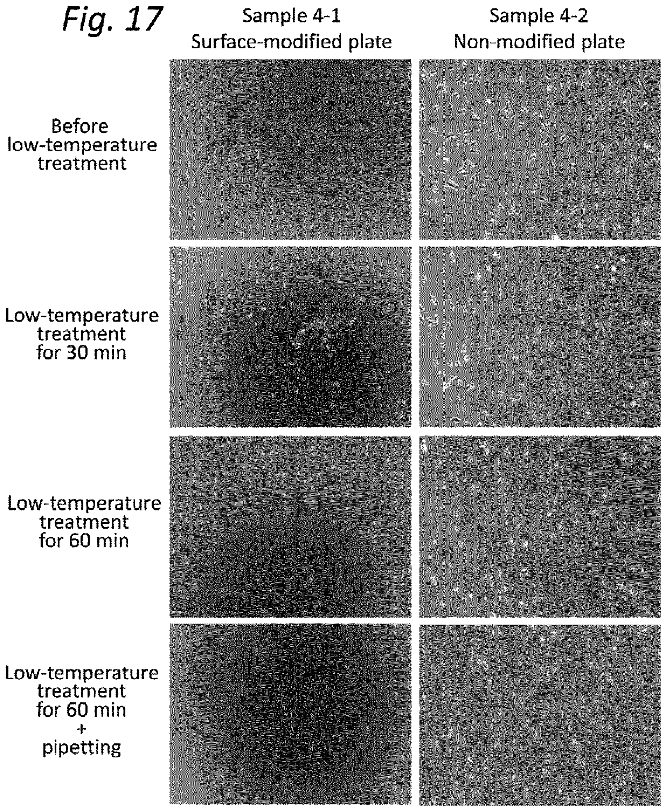

Using the values measured, the cell detachment rate of the plate-like structural body was calculated by the following formula (2). Table 5 and FIG. 16 show the results. FIG. 17 shows the microscopic images of the surfaces of the plate-like structural body.

Formula (2):

$$\text{Cell detachment rate (\%)} = (\text{Number of suspended cells / Number of seeded cells}) \times 100$$

The results showed that, when a plate whose surface was modified with poly(N-isopropylacrylamide) was employed (Sample 4-1), the phase transition occurred on the plate surface by the low-temperature treatment and the surface of the plate-like structural body exhibited cell detachment activity. Specifically, it was shown that about 54% of the cells were detached after the low-temperature treatment for 30 minutes. The detachment effects drastically improved when the low-temperature treatment was performed for 60 minutes, exhibiting a cell detachment rate of 87 %. When physical treatment by pipetting was further performed after the low-temperature treatment for 60 minutes, 93.5 % of the cells were detached.

Table 5

| | Cell detachment rate | |
|---|---|---|
| | Sample 4-1 Surface-modified plate | Sample 4-2 Non-modified plate |
| Before the low-temperature culture | 0.0 % | 0.0 % |
| Low-temperature culture for 30 min | 54.0 % | 0.5 % |
| Low-temperature culture for 60 min | 87.0 % | 3.5 % |

(continued)

| | Cell detachment rate | |
|---|---|---|
| | Sample 4-1 Surface-modified plate | Sample 4-2 Non-modified plate |
| Low-temperature culture for 60 min + pipetting | 93.5 % | 9.5 % |

[Example 5] A Mode of Performing All the Processes without Opening the Main Vessel Unit

The vessel for cell isolation, culture and proliferation produced in the example above was improved, and the cell isolation process, the primary culture process, the cell detachment process, and the cell proliferation process were performed on stem cells without opening the main vessel unit.

(1) Vessel for Cell Isolation, Culture and Proliferation

A vessel for cell isolation, culture and proliferation was prepared in the same manner as described in Example 1 except that modifications were made to some parts of the constituting members. The members modified in this example are as follows.

(i) Modifications to the Composition of the Members

A structural body that had an opening portion of 8 mm diameter on the ceiling plate portion of the upper surface, said opening portion serving as the structure 34 for insertion and removal of the hollow tubular member, was employed as the upper structural body 22 of the primary culture vessel of the primary culture unit. Protrusions connectable with the locking holes of the side surface structure of the main lid portion were provided at the upper ends of the two grip portions 24 of the upper structural body 22.

As the main lid portion 56 of the main vessel unit, a structural body having the structure 60 for insertion and removal of the hollow tubular member, which was formed by embedding a cylindrical silicon resin with an outer diameter of 37 mm and thickness of 0.5 mm at a position located 37 mm apart from the center of the ceiling plate of the upper portion of the main lid portion, was employed. Locking holes engageable with the protrusions of the grip portions of the upper structural body of the primary culture vessel were provided at an inner portion with respect to the side surface structure of the main lid portion 56.

(ii) Modifications to the Assemblage Structure

The vessel for cell isolation, culture and proliferation in this example was assembled in the same manner as described in Example 1 except that modifications were made to some parts of the composition. The modifications made in this example are as follows.

In the vessel in this example, the protrusions of the grip portions 24 of the upper structural body of the primary culture vessel and the locking holes of the main lid portion 56 are engaged with and connected to each other when the primary culture unit 3 is stored in the main vessel unit 4 and the main vessel is sealed by the main lid portion 56.

With this structure, the upper structural body of the primary culture vessel, whose bottom portion of the side surface serves as the member for blocking the releasing structure, is moved upward in response to the rotation of the main lid portion, and as a result, the releasing windows 28 of the lower structural body of the primary culture vessel open. This operation can be performed while the main vessel unit is maintained in a sealed state, as the O-ring 57 provided at the upper inner portion of the main lid portion secures airtightness.

In the vessel in this example, a space in which cells can proliferate can be provided between the culture surface for cell proliferation of the main vessel and the outer wall of the bottom surface of the primary culture vessel in a state in which the primary culture unit is stored in the main vessel unit.

In the vessel in this example, the structure 60 for insertion and removal of the hollow tubular member of the main lid portion and the structure 34 for insertion and removal of the hollow tubular member of the upper structural body of the primary culture vessel are provided at positions overlapping with each other when viewed from the top.

(2) Isolation and Proliferation of Stem Cells

The cell isolation process, the primary culture process, the cell detachment process and the cell proliferation process were performed on stem cells using the vessel for cell isolation, culture and proliferation 1 produced in section (1) above. Upon conducting the experiment in this example, following modifications were made to the procedures in Example 2. For other procedures and experimental conditions, those described in Example 2 were employed.

(i) Exchanging the Medium in the Primary Culture Vessel

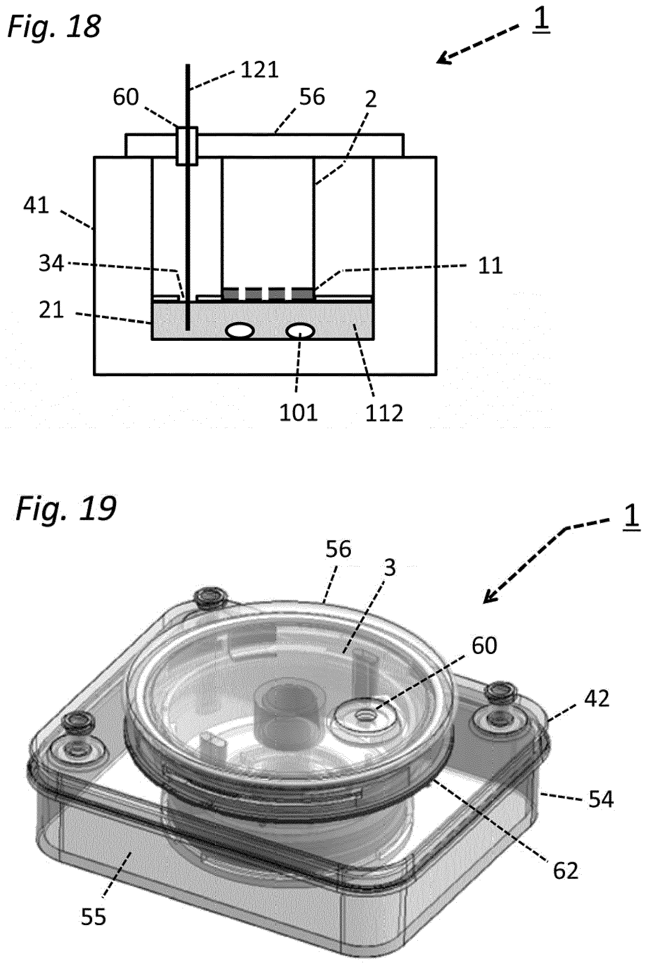

After the cell isolation process, a syringe with a 20G needle of 38 mm length was inserted into the structure 60 for insertion and removal of the hollow tubular member of the main lid portion, and the cell isolation medium in the primary culture vessel 21 was exchanged for the primary culture medium through the structure 34 for insertion and removal of the hollow tubular member of the upper structural body of the primary culture vessel. Here, upon injecting the fresh medium, the primary culture medium was filled so that its liquid surface would be lower than the surface of the cell separation membrane 11. These operations were performed without opening the main vessel unit 4. FIG. 18 is a schematic diagram outlining this operation.

(ii) Reseeding in the Primary Culture Process

When four days passed after the initiation of the primary culture, cell detachment was performed by a cooling operation at 4°C for 60 minutes. Then, the entire vessel was shaken so that the cells would be dispersed evenly in the medium, and the cells were cultured again in an incubator at 37°C at a $CO_2$ concentration of 5% until they reached 60 to 70% confluence. These operations were performed without opening the main vessel unit 4.

(iii) Opening of the Releasing Structure

When the primary culture process was completed and the cell detachment operation was performed, the main lid portion 56 was rotated to move the upper structural body 22 of the primary culture vessel upward, and the releasing windows 28 of the lower structural body of the primary culture vessel were opened. The main vessel was shaken so that all the cells detached and suspended would flow into the main vessel 41. These operations were performed without opening the main vessel unit 4.

(iv) Cell Proliferation Process

The cell proliferation process was performed without opening the main vessel unit 4, in a state in which the primary culture vessel and the like were stored in the main vessel.

(3) Evaluation

The characteristics of the cells which were isolated and proliferated in the series of operations and processes described above were evaluated in the same manner as in Example 2. The results showed that the cells obtained in this example had a higher positive rates of stem cell markers compared with the untreated control cells, indicating that the cells obtained in this example were cells exhibiting the characteristics of stem cells.

In this example, in which the reseeding of the cells was performed in the middle of the primary culture process, the stem cell content was higher compared with Example 2, and the proliferation rate in the cell proliferation process was also improved.

These results show that, by employing the vessel for cell isolation, culture and proliferation of this example, the isolation and proliferation of stem cells can be performed without opening the main vessel unit 4. That is, stem cells can be isolated, cultured and proliferate while the risk of infection or contamination is greatly reduced.

Also, it is shown that the reseeding operation in the middle of the primary culture process is effective in improving the yield of the stem cells.

[Example 6] A Mode with a Rotation Adjustment Mechanism

The vessel for cell isolation, culture and proliferation produced in the example above was improved to produce a vessel for cell isolation, culture and proliferation provided with a rotation adjustment mechanism having improved operability in the rotation of the main lid portion of the main vessel unit.

(1) Vessel for Cell Isolation, Culture and Proliferation

A vessel for cell isolation, culture and proliferation 1 shown in FIG. 19 was prepared in the same manner as described in Example 1 except that modifications were made to some parts of the constituting members. The members modified in this example are as follows.

(i) Modifications to the Composition of the Members

A structural body that had an opening portion of 8 mm diameter on the ceiling plate portion of on the upper surface, said opening portion serving as the structure 34 for insertion and removal of the hollow tubular member, was employed as the upper structural body 22 of the primary culture vessel of the primary culture unit. Protrusions connectable with the locking holes of the side surface structure of the main lid portion were provided at the upper end of the two grip portions 24 of the upper structural body 22. The lower structural body 26 of the primary culture vessel was provided as a structural body having flange structures on the upper portions of its two grip portions 30, and fitting structures connectable with the locking holes on the side surface structure of the main lid portion were provided on the flange structures.

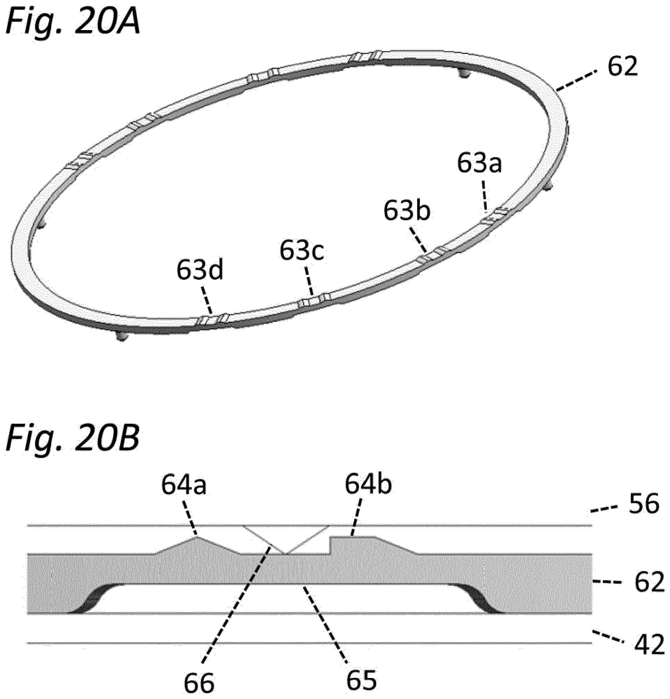

At the upper structural body 42 of the main vessel of the main vessel unit, a click-lock ring 62 was disposed in contact with the outer circumference of the structure (45: short cylindrical structure) to be fitted to the main lid portion (FIG. 19). The click-lock ring 62 is a ring-shaped member made of a hard elastic resin (polycarbonate) and having eight adjustment portions 63 aligned (FIG. 20A).

On the click-lock ring 62, four adjustment portions 63 are aligned at an angular interval of 20 to 35 degrees with respect to the center of the click-lock ring, and another four adjustment portions are provided on their symmetric positions with respect to the center of the ring 62. The upper surface of an adjustment portion 63 has a pair of bosses 64 of 0.2 mm height, and the lower surface of the adjustment portion has a deformable recessed structure 65 which can bend downward when a pushing force is applied from the upper side of the bosses (FIG. 20B). In this mode, one of the two bosses of the adjustment portion 63 is provided as a boss for preventing reverse rotation 64b which has a trapezoidal shape and whose surface facing the other boss has a vertical plane to prevent reverse rotation.

As the main lid portion 56 of the main vessel unit, a structural body provided with a structure 60 for insertion and removal of the hollow tubular member formed by embedding a cylindrical silicon resin with an outer diameter of 37 mm and thickness of 0.5 mm at a position located 37 mm apart from the center of the ceiling plate of the upper portion of the main lid portion was employed. Locking holes engageable with the protrusions of the grip portions 24 of the upper structural body of the primary culture vessel were provided at an inner portion with respect to the side surface structure of the main lid portion 56. Locking holes engageable with the fitting structures on the flanges of the grip portions 30 of the lower structural body of the primary culture vessel were provided at the inner portion with respect to the side surface structure of the main lid portion 56. Clicking protrusions 66 were provided on the lower portion of the side surface structure of the main lid portion 56. A clicking protrusion 66 is a pointed triangular structure whose apex is on the lower side, and two clicking protrusions are formed at symmetric positions with respect to the center of the click-lock ring 62.

(ii) Modifications to the Assemblage Structure

The vessel for cell isolation, culture and proliferation 1 in this example was assembled in the same manner as described in Example 1 except that modifications were made to some parts of the composition. The modifications made in this example are as follows.

In the mode of this example, the vessel has a structure with which the change of the pushing force can be felt and a clicking sound is generated when the clicking protrusions 66 disposed at the lower portion of the side surface structure of the main lid portion 56 pass over the bosses 64 of the adjustment portions 63a by the rotation operation of the main lid portion. As a result, with the vessel according to the present invention, the operator can easily recognize the degree of the rotation of the main lid portion and its corresponding structural change of the vessel.

In the vessel of this example, the protrusions of the grip portions 24 of the upper structural body of the primary culture vessel and the locking holes of the main lid portion 56 are engaged with and connected to each other when the primary culture unit 3 is stored in the main vessel unit 4 and the main vessel is sealed. In this state, the clicking protrusion 66 disposed at the lower portion of the side surface structure of the main lid portion 56 is positioned between the two bosses 64 of the adjustment portion 63a located at the rightmost position among the four adjustment portions provided on the click-lock ring 62 (FIG. 21A).

When the main lid portion is rotated and the clicking protrusion 66 is moved to the adjustment portion 63b located at the second position from the right, the upper structural body of the primary culture vessel, whose bottom portion of the side surface serves as the member 35 for blocking the releasing structure, moves upward in response to the rotation operation and the releasing windows 28 of the lower structural body of the primary culture vessel are opened (FIG. 21B).

Then, by the rotation operation of the upper portion of the main lid portion, the fitting structures on the flanges of the grip portions 30 of the lower structural body of the primary culture vessel and the locking holes of the main lid portion 56 are engaged with and connected to each other. In the vessel of this example, when the clicking protrusion 66 is positioned at the adjustment portion 63a located at the rightmost position or at the adjustment portion 63b located at the second position from the right, the outer wall of the bottom surface of the lower structural body 26 of the primary culture vessel is kept in contact with the culture surface for cell proliferation 55 of the main vessel (FIG. 21B).

In this state, when the clicking protrusion 66 is further moved to the adjustment portion 63c located at the third position from the right by the rotation operation, the bottom surface of the lower structural body 26 of the primary culture vessel moves upward in response to the rotation operation, and a space in which cells can proliferate is formed between the outer wall of the bottom surface of the lower structural body 26 of the primary culture vessel and the culture surface for cell proliferation 55 of the main vessel. As the space is formed in the vessel of this example, the structure of the vessel is changed to enlarge the area of the culture surface for performing the cell proliferation process (FIG. 21C).

In the vessel of this example, the click-lock ring 62 provided with eight adjustment portions 63 (two sets of four adjustment portions) described above and the clicking protrusions 66 are the main members of the rotation adjustment mechanism 61.

The above-mentioned operations on the vessel of this example can be performed while the main vessel unit is maintained in a sealed state because the O-ring 57 provided at the upper inner portion of the main lid portion secures airtightness.

In the vessel of this example, the structure 60 for insertion and removal of the hollow tubular member of the main lid portion and the structure 34 for insertion and removal of the hollow tubular member of the upper structural body of the primary culture vessel are provided at positions overlapping with each other when viewed from the top.

(2) Isolation and Proliferation of Stem Cells

The cell isolation process, the primary culture process, the cell detachment process and the cell proliferation process were performed on stem cells using the vessel for cell isolation, culture and proliferation 1 produced in section (1) above. Upon conducting the experiment in this example, following modifications were made to the procedures in Example 2. For other operations and experimental conditions, those described in Example 2 were employed.

(i) Exchanging the Medium in the Primary Culture Vessel

The primary culture vessel and the like were stored in the vessel for cell isolation, culture and proliferation 1 produced in section (1) above in a state in which the bottom surface of the lower structural body 26 of the primary culture vessel was in contact with the culture surface 55 of the main vessel. In this state, the clicking protrusion 66 is loosely fitted to the adjustment portion 63a located at the rightmost position. After the cell isolation process, a syringe with a 20G needle of 38 mm length was inserted into the structure 60 for insertion and removal of the hollow tubular member of the main lid portion, and the cell isolation medium in the primary culture vessel 21 was exchanged for the primary culture medium through the structure 34 for insertion and removal of the hollow tubular member of the upper structural body of the primary culture vessel. These operations were performed without opening the main vessel unit 4.

(ii) Reseeding in the Primary Culture Process

When four days passed after the initiation of the primary culture, cell detachment was performed by a cooling operation at 4°C for 60 minutes. Then, the entire vessel was shaken so that the cells would be dispersed evenly in the medium, and the cells were cultured again in an incubator at 37°C at a $CO_2$ concentration of 5% until they reached 60 to 70% confluence. These operations were performed without opening the main vessel unit 4.

(iii) Opening of the Releasing Structure

When the primary culture process was completed and the cell detachment operation was performed, the main lid portion 56 was rotated to the right until the clicking protrusion 66 loosely fitted the adjustment portion 63b located at the second position from the right. With this operation, the upper structural body 22 of the primary culture vessel moved upward, and the releasing windows 28 of the lower structural body of the primary culture vessel were opened (FIG. 21B).

Then, the main vessel was shaken so that all the cells detached and suspended would flow into the main vessel 41. These operations were performed without opening the main vessel unit 4.

(iv) Cell Proliferation Process

The main lid portion 56 was further rotated to the right until the clicking protrusion 66 loosely fitted the adjustment portion 63c located at the third position from the right. With this operation, the bottom surface of the lower structural body 26 of the primary culture vessel moved upward, and a space in which cells could proliferate was formed between the outer wall of the bottom surface of the lower structural body 26 of the primary culture vessel and the culture surface for cell proliferation 55 of the main vessel (FIG. 21C). The vessel was shaken lightly so that the cells detached and suspended would be dispersed evenly on the culture surface for cell proliferation 55.

In this example, the cell proliferation process was performed without opening the main vessel unit 4, in a state in which the primary culture vessel and the like were stored in the main vessel.

After the culturing, the main lid portion 56 was further rotated to the right until the clicking protrusion 66 loosely fitted the adjustment portion 63d located at the fourth position from the right. With this operation, the main lid portion, together with the primary culture unit and the cell separation unit which were connected to the main lid portion, were pulled up and the cultured cells in the main vessel were collected.

(3) Evaluation

The characteristics of the cells which were isolated and proliferated in the series of operations and processes described above were evaluated in the same manner as in Example 2. The results showed that the cells obtained in this example had a higher positive rates of stem cell markers compared with the untreated control cells, indicating that the cells obtained in this example were cells exhibiting the characteristics of stem cells.

These results show that, by employing the vessel for cell isolation, culture and proliferation 1 of this example, the isolation and proliferation of stem cells can be performed without opening the main vessel unit 4. That is, stem cells can be isolated, cultured and proliferate while the risk of infection or contamination is greatly reduced.

Moreover, it was confirmed that, upon performing the rotation operation of the main lid portion, the operator could recognize the predetermined rotational position and its corresponding structural change of the vessel, as the main lid portion was provided with a rotation adjustment mechanism 61 corresponded to the structural change of the primary culture unit.

[Example 7] Production of a Cell Separation Membrane Using Plasma Treatment

It was examined whether the hydrophilization of the surface of the hydrophobic base material layer by plasma treatment was possible as one production mode of the cell separation membrane.

(1) Plasma Treatment on the Hydrophobic Base Material

A polyethylene (PE) membrane with a thickness of $20\mu m$, which can be used as a base material layer of the cell separation membrane, was subjected to vacuum plasma treatment at a low energy. The vacuum plasma treatment was conducted at the voltages shown in Table 6 using a bell-jar vacuum plasma apparatus manufactured by Stec Co., Ltd. The contact angles of the surface of the PE membranes measured in this experiment are shown in Table 6.

The results show that vacuum plasma treatment at voltages of 0.5 to 1.75 kV reduces the contact angles of the PE membrane surface, indicating the hydrophilization of the membrane structure. It is shown that the hydrophilicity increases in correlation with the increase in the voltage applied.

Moreover, since one PE membrane had almost the same contact angles on its entire surface, it was indicated that the entire surface of the PE membrane was uniformly hydrophilized.

These results show that a cell separation membrane in which the surface of the hydrophobic base material layer is uniformly hydrophilized can be produced as a PE membrane is subjected to plasma treatment at a low energy. The contact angle of the cell separation membrane produced in this mode did not change even after six months from the production, when compared with a membrane treated at a high voltage. It was confirmed that, in the cell separation membrane produced in this mode, the occurrence of surface etching was extremely low, and the deterioration with time was reduced.

Table 6

|  | Voltage applied (kV) in the vacuum plasma treatment on PE | Contact angle (degrees) |
|---|---|---|
| Control | — (Untreated) | 96.9 |
| Sample 7-1 | 0.50 | 55.1 |
| Sample 7-2 | 0.75 | 44.5 |
| Sample 7-3 | 1.00 | 38.0 |
| Sample 7-4 | 1.25 | 25.1 |

(continued)

|  | Voltage applied (kV) in the vacuum plasma treatment on PE | Contact angle (degrees) |
|---|---|---|
| Sample 7-5 | 1.50 | 19.3 |
| Sample 7-6 | 1.75 | 17.2 |

[Example 8] Plasma Treatment on the Base Material Layer of the Culture Surface

It was examined whether the hydrophilization of the surface of the hydrophobic base material layer by plasma treatment was possible as one production mode of the culture surface for primary culture and the culture surface for cell proliferation.

(1) Plasma Treatment on the Culture Surface

Plate members (ø 35 mm) made of polystyrene (PS), which can be used as the base material layer of the culture surface for primary culture and the culture surface for cell proliferation, were subjected to vacuum plasma treatment at low energies. The vacuum plasma treatment was conducted at a voltage of 0.5 kV or 1.0 kV using a bell-jar vacuum plasma apparatus manufactured by Stec Co., Ltd.

As the contact angle of the surface of the plate member was measured, it was shown that the surface was hydrophilized compared with an untreated PS plate, which was the control (commercially available culture plate: contact angle of 90.5°). The value of the contact angle indicates that the degree of hydrophilization of this plate surface is at an adequate level that does not interfere with the cell adhesion.

When the plate member after plasma treatment was treated with acetone, a residue of a thin undissolved layer was confirmed, indicating the formation of a crosslinked layer on the surface of the hydrophobic base material. However, the plate treated at 1.0 kV had a lower amount of the undissolved layer remaining after the acetone treatment, compared with the plate treated at 0.5 kV. It is thought that, as the voltage applied was higher, etching occurred, the surface properties were less improved, and as a result, the contact angle became larger.

These result show that, in the culture plate produced by this production mode of performing plasma treatment, the occurrence of surface etching was extremely low, and the deterioration with time was reduced. Especially, it is shown that the plate treated at 0.5 kV has an extremely low occurrence of surface etching and a high resistance to deterioration with time.

Table 7

|  | Voltage applied (kV) in the vacuum plasma treatment on PS | Contact angle (degrees) |
|---|---|---|
| Control | — (Untreated) | 90.5 |
| Sample 8-1 | 0.50 | 20.1 |
| Sample 8-2 | 1.00 | 55.8 |

(2) Cell Culture Test

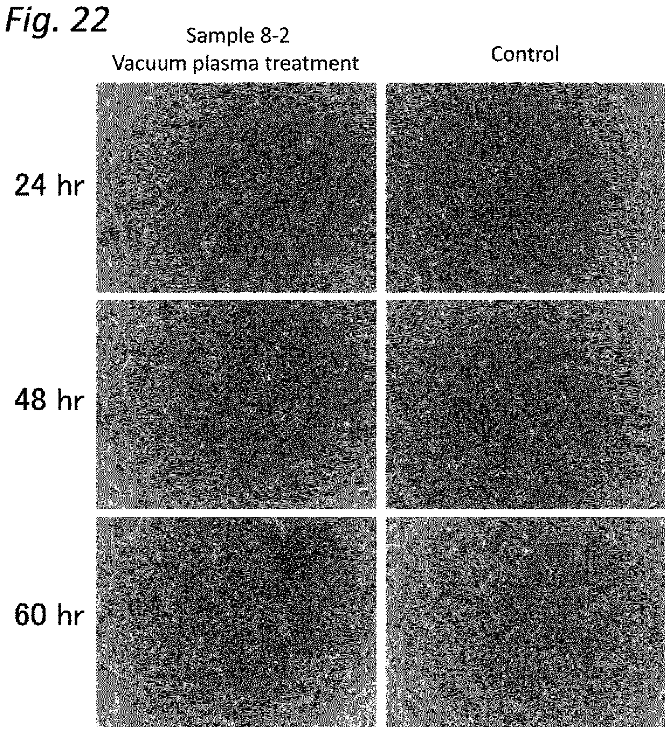

The plate member produced in section (1) above was cut into four equal parts. Stem cells of $1 \times 10^4$ cells per piece of plate were seeded on the surface of the plate piece and cultured in an incubator at a $CO_2$ concentration of 5% for 60 hours, and the cells cultured were observed over time. The fifth passage of VW190 DPSCs derived from the dental pulp tissue of the dog canine tooth was used as the stem cells. A DMEM medium containing 10% FBS was used as the culture medium. The results of observation with the passage of time are shown in FIG. 22 and Table 8.

The results show that, on the plate member whose surface is adequately hydrophilized by the plasma treatment, cells can proliferate to the same extent as on an untreated plate member (commercially available culture plate), which is a control.

These results indicate that the plate member whose surface is appropriately hydrophilized by plasma treatment can be suitably used as the culture surface for primary culture and the culture surface for cell proliferation.

Table 8

| | Voltage applied (kV) in the vacuum plasma treatment on PS | Number of cells after 60 hours |
|---|---|---|
| Control | — (Untreated) | $4.3 \times 10^4$ cells (4.3 times from the initiation of the cell culture) |
| Sample 8-2 | 1.00 | $4.6 \times 10^4$ cells (4.6 times from the initiation of the cell culture) |

List of Reference Numerals

1: Vessel for cell isolation, culture and proliferation
2: Cell separation unit
11: Cell separation membrane
12: Fine pores (cell-isolation pores)
13: Membrane holding structure, tubular structure
14: Opening portion (liquid introducing portion)
15: Opening portion (liquid discharging portion)
16: Bottom surface portion
17: Ring structural body
18: Opening portion
3: Primary culture unit
21: Primary culture vessel
22: Upper structural body (upper structural body of the primary culture vessel)
23: Opening portion (upper opening portion of the primary culture vessel)
24: Grip portion
25: Protrusion (for engagement with the lower structural body)
34: Structure for insertion and removal of the hollow tubular member
35: Member for blocking the releasing structure
26: Lower structural body (lower structural body of the primary culture vessel)
27: Culture surface for primary culture, flat plate member
28: Releasing structure, releasing window
29: Elastic member, O-ring
30: Grip portion
31: Flange structure
32: Locking hole (for engagement with the main vessel)
33: Locking hole (for engagement with the upper structural body)
4: Main vessel unit
41: Main vessel
42: Upper structural body (upper structural body of the main vessel)
43: Opening portion (portion for inserting and taking out the culture units)
44: Protrusion (for engagement with the primary culture vessel)
45: Fitting structure (for fitting to the main lid portion), short cylindrical structure
46: Protrusion (for engagement with the main lid portion)
47: Gas exchange port
48: Gas exchange channel
49: Sterilizing filter for ventilation
50: Adapter
51: Medium exchange port
52: Tube for medium exchange
53: Cap
54: Lower structural body (lower structural body of the main vessel)
55: Culture surface for cell proliferation
56: Main lid portion
57: Elastic member, O-ring

58: Fitting structure (for fitting to the cell separation unit)

59: Locking hole

60: Structure for insertion and removal of the hollow tubular member

61: Rotation adjustment mechanism

62: Click-lock ring, ring structural body provided with adjustment portions

63: Adjustment portion

63a - 63d: Adjustment portion

64: Boss

64a: Boss (triangular shape)

64b: Boss for preventing reverse rotation (trapezoidal shape having a vertical plane)

65: Recessed structure

66: Clicking protrusion

5: Cell proliferation unit

101: Target cells

102: Cells other than target cells

111: Cell suspension medium

112: Cell isolation medium

113: Primary culture medium

114: Medium for proliferation culture

121: Hollow tubular member

**Claims**

1. A sealable vessel for cell isolation, culture and proliferation (1), the vessel (1) being a container-like structural body in which isolation, culture and proliferation of target cells of interest can be performed, wherein

   (A) the vessel (1) comprises a cell separation unit (2), a primary culture unit (3) and a main vessel unit (4);
   (B) the cell separation unit (2) is a structural body comprising a cell separation membrane (11) having fine pores capable of isolating cells by attracting or activating movement of the target cells by utilizing chemotactic factors;
   (C) the primary culture unit (3) is a container-like structural body that has a culture surface for primary culture (27) on which cells that have passed through the cell separation membrane (11) can be cultured and a releasing structure (28) through which cells and a liquid that have been held inside the primary culture unit (3) can be freely released toward a unit having a culture surface for a subsequent step, the culture surface for primary culture (27) having a size of 10 to 400 cm$^2$; and
   (D) the main vessel unit (4) is a container-like structural body that has a culture surface for cell proliferation (55) on which cells that have been released from a culture unit for a preceding step can be cultured, the culture surface for cell proliferation (55) being a culture surface different from the culture surface for primary culture (27) of the primary culture unit (3), an area of the culture surface for cell proliferation (55) being larger than that of a culture surface of the culture unit for the preceding step, the container-like structural body of the main vessel unit (4) having a structure in which the cell separation unit (2) and all of the culture unit/units for preceding step/steps can be stored.

2. The vessel for cell isolation, culture and proliferation (1) according to claim 1, wherein

   (b-1) the cell separation unit (2) recited in (B) is a container-like structural body;
   (b-2) the cell separation unit (2) comprises the cell separation membrane (11) having fine pores capable of isolating cells, the cell separation membrane (11) being provided on at least a part of surfaces of the container-like structural body of the cell separation unit (2);
   (c-1) the primary culture unit (3) recited in (C) is a container-like structural body;
   (c-2) the primary culture unit (3) has a structure with which the cell separation unit (2) can be disposed in a state in which, when a liquid is filled in the container-like structural body of the primary culture unit (3), a surface of the cell separation membrane (11) and a surface of the liquid filled are in contact with each other;
   (c-3) the primary culture unit (3) has the culture surface for primary culture (27) on which the cells that have passed through the cell separation membrane (11) can be cultured, the culture surface for primary culture (27) being provided on at least a part of surfaces of the container-like structural body of the primary culture unit (3);

(c-4) the primary culture unit (3) has the releasing structure (28) through which the cells and the liquid that have been held inside the container-like structural body of the primary culture unit (3) can be freely released toward the unit having the culture surface for the subsequent step, the releasing structure (28) being provided at a surface of the container-like structural body of the primary culture unit (3);

(d-1) the main vessel unit (4) recited in (D) is a container-like structural body mainly composed of a main vessel (41) and a main lid portion (56);

(d-2) the main vessel unit (4) has the culture surface for cell proliferation (55) on which the cells that have been released from the culture unit for the preceding step can be cultured, the area of the culture surface for cell proliferation (55) being larger than that of the culture surface of the culture unit for the preceding step;

(d-3) the main vessel unit (4) has a structure with which the cell separation unit (2) and all of the culture unit/units for the preceding step/steps can be stored in the container-like structural body of the main vessel unit (4); and

(d-4) the main lid portion (56) is a lid-like structural body with which the entire vessel can be sealed.

3. The vessel for cell isolation, culture and proliferation (1) according to claim 1 or 2, wherein the cell separation unit (2) is a unit for isolating the target cells of interest into the container-like structural body of the primary culture unit (3) through the cell separation membrane (11).

4. The vessel for cell isolation, culture and proliferation (1) according to any one of claims 1 to 3, wherein, when an area of the culture surface of the culture unit for the preceding step is taken as 1, a ratio of an area of the culture surface for cell proliferation (55) is within a range of more than 1 to not more than 20.

5. The vessel for cell isolation, culture and proliferation (1) according to any one of claims 1 to 4, wherein, in the vessel for cell isolation, culture and proliferation,

(E)(e-1) at least a part of all the culture surfaces included in the vessel for cell isolation, culture and proliferation (1) and at least a part of vessel structures opposed to or substantially opposed to the culture surface for cell proliferation (55) of the main vessel unit (4) are made of light-transmitting material; and

(e-2) the culture surfaces inside the vessel (1) can be observed through portions having light-transmitting properties without opening the main vessel unit (4).

6. The vessel for cell isolation, culture and proliferation (1) according to any one of claims 1 to 5, wherein the primary culture unit (3) is a container-like structural body composed of two or more members, and has a structure with which the releasing structure (28) can be adjusted to open as the members of the container-like structural body of the primary culture unit (3) are detached or their positions are changed.

7. The vessel for cell isolation, culture and proliferation (1) according to any one of claims 1 to 6, wherein the primary culture unit (3) has a structure with which the releasing structure (28) can be adjusted to open in response to operation from outside the main vessel unit (4).

8. The vessel for cell isolation, culture and proliferation (1) according to any one of claims 2 to 7, wherein the primary culture unit (3) has a structure with which the releasing structure (28) can be adjusted to open in response to operation of the main lid portion.

9. The vessel for cell isolation, culture and proliferation (1) according to any one of claims 2 to 8, wherein, in the vessel for cell isolation, culture and proliferation,

(F)(f-1) a structure (60) through which a hollow tubular member can be inserted into or taken out of the main vessel without opening the main vessel unit (4) is provided at a wall surface of the main vessel or the main lid portion of the main vessel unit (4);

(f-2) the structure (60) is disposed at a position at which the liquid filled in the primary culture unit (3) can be suctioned out through the hollow tubular member inserted; and

(f-3) the structure (60) is mainly composed of an elastic member which can secure airtightness both in a state in which the hollow tubular member is not inserted and in a state in which the hollow tubular member is inserted.

10. The vessel for cell isolation, culture and proliferation (1) according to any one of claims 2 to 9, wherein the cell separation unit (2) has a structure with which a position of the cell separation membrane (11) can be adjusted in response to operation of the main lid portion.

**11.** The vessel for cell isolation, culture and proliferation (1) according to any one of claims 1 to 10, wherein the main vessel unit (4) is provided with a gas exchange port.

**12.** The vessel for cell isolation, culture and proliferation (1) according to any one of claims 1 to 11, wherein the main vessel unit (4) is provided with a medium exchange port through which a medium can be introduced into or discharged from the main vessel.

**13.** The vessel for cell isolation, culture and proliferation (1) according to any one of claims 1 to 12, wherein the cell separation unit (2) is a container-like structural body that is mainly composed of the cell separation membrane (11) and a membrane holding structure (13), and a peripheral portion of a bottom surface of an opening portion of the membrane holding structure (13) covered by the cell separation membrane (11) has a shape having an upward inclination toward the opening portion.

**14.** The vessel for cell isolation, culture and proliferation (1) according to any one of claims 1 to 13, comprising:

(G) one or more cell proliferation units with which a plurality of cell proliferation processes can be performed after a primary culture process;
wherein (g-1) the cell proliferation unit is a container-like structural body;
(g-2) the cell proliferation unit comprises a culture surface for cell proliferation on which cells that have been released from a culture unit for a preceding step can be cultured, an area of the culture surface for cell proliferation being larger than that of a culture surface of the culture unit for the preceding step;
(g-3) the cell proliferation unit has a structure with which the cell separation unit (2) and all of the culture unit/units for the preceding step/steps can be stacked or stored in the container-like structural body of the cell proliferation unit; and
(g-4) the cell proliferation unit has a releasing structure through which cells and a liquid that have been held inside the container-like structural body of the cell proliferation unit can be freely released toward a unit having a culture surface for a subsequent step, the releasing structure being provided at a surface of the container-like structural body of the cell proliferation unit.

**15.** The vessel for cell isolation, culture and proliferation (1) according to any one of claims 1 to 14, wherein the fine pores formed in the cell separation membrane (11) have an average size of 1 to 20 $\mu$m, and the cell separation membrane (11) has a porosity of 5 to 50%.

**16.** The vessel for cell isolation, culture and proliferation (1) according to any one of claims 1 to 15, wherein the fine pores formed in the cell separation membrane (11) are open in a vertical direction and/or in a substantially vertical direction with respect to a membrane surface.

**17.** The vessel for cell isolation, culture and proliferation (1) according to any one of claims 1 to 16, wherein a part or all of the fine pores formed in the cell separation membrane (11) are arranged in a lattice pattern, a substantially lattice pattern, a staggered pattern, a substantially staggered pattern, a spiral pattern, a substantially spiral pattern, a concentric pattern, a substantially concentric pattern, or a combination of the aforementioned patterns.

**18.** The vessel for cell isolation, culture and proliferation (1) according to any one of claims 1 to 17, wherein the cell separation membrane (11) has a structure in which hydrophilic polymers are covalently bonded to a surface of a base material layer made of hydrophobic polymers.

**19.** The vessel for cell isolation, culture and proliferation (1) according to any one of claims 1 to 18,

wherein the cell separation membrane (11) has a structure in which one or more selected from the hydroxyl group, the carboxyl group and a compound structure including said functional groups are covalently bonded to a vicinity of the surface of the base material layer made of hydrophobic polymers; and/or
the cell separation membrane (11) has an insoluble layer in the vicinity of the surface of the base material layer made of hydrophobic polymers, the insoluble layer being insoluble in a good solvent for the hydrophobic polymers.

**20.** The vessel for cell isolation, culture and proliferation (1) according to any one of claims 1 to 19, wherein a surface of the culture surface for primary culture (27) and/or a surface of the culture surface for cell proliferation (55) are modified with molecules that exhibit cell adhesion properties during culture and exhibits cell detachment properties in response to a stimulus.

**21.** The vessel for cell isolation, culture and proliferation (1) according to claim 20, wherein the molecules that exhibit cell detachment properties are temperature responsive polymer molecules having a phase transition temperature, or a lower critical solution temperature, of 15 to 35°C.

**22.** The vessel for cell isolation, culture and proliferation (1) according to any one of claims 1 to 21,

wherein the culture surface for primary culture (27) and/or the culture surface for cell proliferation (55) have a structure in which one or more selected from the hydroxyl group, the carboxyl group, and a compound structure including said functional groups are covalently bonded to a vicinity of a surface of a base material layer made of hydrophobic polymers; and/or
the culture surface for primary culture (27) and/or the culture surface for cell proliferation (55) have an insoluble layer in the vicinity of the surface of the base material layer made of hydrophobic polymers, the insoluble layer being insoluble in a good solvent for the hydrophobic polymers.

**23.** The vessel for cell isolation, culture and proliferation (1) according to any one of claims 1 to 22,

wherein the structural bodies constituting the vessel for cell isolation, culture and proliferation (1) have a structure in which one or more selected from the hydroxyl group, the carboxyl group, and a compound structure including said functional groups are covalently bonded to a vicinity of a surface of a base material layer made of hydrophobic polymers; and/or
the structural bodies constituting the vessel for cell isolation, culture and proliferation (1) have an insoluble layer provided in the vicinity of the surface of the base material layer made of hydrophobic polymers, the insoluble layer being insoluble in a good solvent for the hydrophobic polymers.

**24.** The vessel for cell isolation, culture and proliferation (1) according to any one of claims 1 to 23, wherein the vessel for cell isolation, culture and proliferation is a vessel for actively isolating stem cells and performing their proliferation.

**Patentansprüche**

**1.** Abdichtbares Gefäß zur Zellisolation, Kultivierung und Vermehrung (1), wobei das Gefäß (1) ein behälterartiger Strukturkörper ist, in dem die Isolation, Kultivierung und Vermehrung der Zielzellen von Interesse durchgeführt werden kann,
wobei (A) das Gefäß (1) eine Zellentrenneinheit (2), eine primäre Kultivierungseinheit (3) und eine Hauptgefäßeinheit (4) aufweist:

(B) die Zelltrenneinheit (2) ein Strukturkörper ist, der eine Zelltrennmembran (11) aufweist, die feine Poren hat, die dazu ausgelegt sind Zellen zu isolieren, indem eine Bewegung der Zielzellen durch die Verwendung chemotaktischer Faktoren angeregt oder aktiviert wird;
(C) die Primärkultureinheit (3) ein behälterartiger Strukturkörper ist, der eine Kulturoberfläche für die Primärkultur (27) hat, auf der die Zellen, die sich durch die Zelltrennmembran (11) hindurchbewegt haben, kultiviert werden können, und eine Freisetzungsstruktur (28), durch die Zellen und eine Flüssigkeit, die in der primären Kultureinheit (3) gehalten wurden, frei in Richtung einer Einheit freigesetzt werden können, die eine Kulturoberfläche für einen nachfolgenden Schritt hat, wobei die Kulturoberfläche für die Primärkultur (27) eine Größe von 10 bis 400 cm$^2$ hat; und
(D) die Hauptgefäßeinheit (4) ein behälterartiger Strukturkörper ist, der eine Kulturoberfläche zur Zellvermehrung (55) hat, auf der Zellen, die aus einer Kultureinheit für einen vorhergehenden Schritt freigesetzt wurden, kultiviert werden können, wobei die Kulturoberfläche zur Zellvermehrung (55) eine Kulturoberfläche ist, die sich von der Kulturoberfläche für die Primärkultur (27) der Primärkultureinheit (3) unterscheidet, wobei eine Fläche der Kulturoberfläche zur Zellvermehrung (55) größer als eine Kulturoberfläche der Kultureinheit für den vorhergehenden Schritt ist, wobei der behälterartige Strukturkörper der Hauptgefäßeinheit (4) eine Struktur hat, in der die Zelltrenneinheit (2) und alle von den Kultureinheit(en) für den/die vorhergehenden Schritt(e) aufbewahrt werden können.

**2.** Gefäß zur Zellisolation, Kultivierung und Vermehrung (1) nach Anspruch 1, wobei

(b-1) die Zelltrenneinheit (2), die in (B) angeführt wird, ein behälterartiger Strukturkörper ist;

(b-2) die Zelltrenneinheit (2) die Zelltrennmembran (11) aufweist, die feine Poren hat, die dazu in der Lage sind, Zellen zu isolieren, wobei die Zelltrennmembran (11) auf wenigstens einem Teil der Flächen des behälterartigen Strukturkörpers der Zelltrenneinheit (2) vorgesehen ist;

(c-1) die Primärkultureinheit (3), die in (C) angeführt wird, ein behälterartiger Strukturkörper ist;

(c-2) die Primärkultureinheit (3) eine Struktur hat, bei der die Zelltrenneinheit (2) in einem Zustand angeordnet sein kann, in dem, wenn eine Flüssigkeit in den behälterartigen Strukturkörper der Primärkultureinheit (3) eingefüllt wird, eine Oberfläche der Zelltrennmembran (11) und eine Oberfläche der eingefüllten Flüssigkeit miteinander in Kontakt stehen;

(c-3) die Primärkultureinheit (3) die Kulturoberfläche für die Primärkultur (27) hat, auf der die Zellen, die sich durch die Zelltrennmembran (11) hindurchbewegt haben, kultiviert werden können, wobei die Kulturoberfläche für die Primärkultur (27) auf wenigstens einem Teil der Oberflächen des behälterartigen Strukturkörpers der Primärkultureinheit (3) vorgesehen sind;

(c-4) die Primärkultureinheit (3) die Freisetzungsstruktur (28) hat, durch die die Zellen und die Flüssigkeit, die in dem behälterartigen Strukturkörper der Primärkultureinheit (3) gehalten werden, frei in Richtung der Einheit freigesetzt werden können, die die Kulturoberfläche für den nachfolgenden Schritt hat, wobei die Freisetzungsstruktur (28) an einer Oberfläche des behälterartigen Strukturkörpers der Primärkultureinheit (3) vorgesehen ist;

(d-1) die Hauptgefäßeinheit (4), die in (D) angeführt wurde, ein behälterartiger Strukturkörper ist, der vorwiegend aus einem Hauptgefäß (41) und einem Hauptdeckelbereich (56) zusammengesetzt ist;

(d-2) die Hauptgefäßeinheit (4) die Kulturoberfläche zur Zellvermehrung (55) hat, auf der die Zellen, die aus der Kultureinheit für den vorhergehenden Schritt freigesetzt wurden, kultiviert werden können, wobei die Fläche der Kulturoberfläche zur Zellvermehrung (55) größer ist als die der Kulturoberfläche der Kultureinheit für den vorhergehenden Schritt;

(d-3) die Hauptgefäßeinheit (4) eine Struktur hat, bei der die Zelltrenneinheit (2) und alle von der/den Kultureinheit(en) für den/die vorhergehenden Schritt(e) in dem behälterartigen Strukturkörper der Hauptgefäßeinheit (4) aufbewahrt werden können; und

(d-4) der Hauptdeckelbereich (56) ein deckelartiger Strukturkörper ist, mit dem das gesamte Gefäß abgedichtet werden kann.

3. Gefäß zur Zellisolation, Kultivierung und Vermehrung (1) nach Anspruch 1 oder 2, wobei die Zelltrenneinheit (2) eine Einheit zum Isolieren der Zielzellen von Interesse in dem behälterartigen Strukturkörper der Primärkultureinheit (3) durch die Zelltrennmembran (11) ist.

4. Gefäß zur Zellisolation, Kultivierung und Vermehrung (1) nach einem der Ansprüche 1 bis 3, wobei wenn eine Fläche der Kulturoberfläche der Kultureinheit für den vorhergehenden Schritt als 1 angenommen wird, ein Verhältnis einer Fläche der Kulturoberfläche zur Zellvermehrung (55) in einem Bereich von größer als 1 und nicht größer als 20 liegt.

5. Gefäß zur Zellisolation, Kultivierung und Vermehrung (1) nach einem der Ansprüche 1 bis 4, wobei in dem Gefäß zur Zellisolation, Kultivierung und Vermehrung,

(E) (e-I) wenigstens ein Teil aller Kulturoberflächen, die im Gefäß zur Zellisolation, Kultivierung und Vermehrung (1) enthalten sind, und wenigstens ein Teil der Gefäßstrukturen, die der Kulturoberfläche zur Zellvermehrung (55) der Hauptgefäßeinheit (4) gegenüberliegen oder im Wesentlichen gegenüberliegen, aus einem lichtdurchlässigen Material hergestellt sind; und (e-2) die Kulturoberflächen in dem Gefäß (1) über die Bereiche, die die lichtdurchlässigen Eigenschaften haben, beobachtet werden können, ohne die Hauptgefäßeinheit (4) zu öffnen.

6. Gefäß zur Zellisolation, Kultur und Vermehrung (1) nach einem der Ansprüche 1 bis 5, wobei die Primärkultureinheit (3) ein behälterartiger Strukturkörper ist, der aus zwei oder mehreren Elementen zusammengesetzt ist und eine Struktur hat, mit der die Freisetzungsstruktur (28) angepasst werden kann, um sich zu öffnen, wenn die Elemente des behälterartigen Strukturkörpers der primären Kultureinheit (3) abgelöst werden oder ihre Positionen verändert werden.

7. Gefäß zur Zellisolation, Kultivierung und Vermehrung (1) nach einem der Ansprüche 1 bis 6, wobei die Primärkultureinheit (3) eine Struktur hat, bei der die Freisetzungsstruktur (28) angepasst sein kann, um sich in Reaktion auf die Betätigung der Hauptgefäßeinheit (4) von außen zu öffnen.

8. Gefäß zur Zellisolation, Kultivierung und Vermehrung (1) nach einem der Ansprüche 2 bis 7, wobei die Primärkultureinheit (3) eine Struktur hat, bei der die Freisetzungsstruktur (28) angepasst sein kann, um sich in Reaktion auf die Betätigung des Hauptdeckelbereichs zu öffnen.

9. Gefäß zur Zellisolation, Kultivierung und Vermehrung (1) nach einem der Ansprüche 2 bis 8, wobei in dem Gefäß zur Zellisolation, Kultivierung und Vermehrung,

(F) (f-l) eine Struktur (60), durch die ein hohles rohrförmiges Element in das Hauptgefäß eingesetzt oder herausgenommen werden kann, ohne die Hauptgefäßeinheit (4) zu öffnen, an einer Wandfläche des Hauptgefäßes oder des Hauptdeckelbereichs der Hauptgefäßeinheit (4) vorgesehen ist;
(f-2) die Struktur (60) an einer Position angeordnet ist, an der die in der primären Kultureinheit (3) eingefüllte Flüssigkeit durch das eingesetzte hohle röhrenförmige Element ausgesaugt werden kann; und
(f-3) die Struktur (60) überwiegend aus einem elastischen Element zusammengesetzt ist, das eine Luftdichtigkeit sowohl in einem Zustand, in dem das hohle röhrenförmige Element nicht eingesetzt ist, und in einem Zustand, in dem das hohle röhrenförmige Element eingesetzt ist, sicherstellen kann.

10. Gefäß zur Zellisolation, Kultivierung und Vermehrung (1) nach einem der Ansprüche 2 bis 9, wobei die Zelltrenneinheit (2) eine Struktur hat, bei der eine Position der Zelltrennmembran (11) in Reaktion auf die Betätigung des Hauptdeckelbereichs angepasst werden kann.

11. Gefäß zur Zellisolation, Kultivierung und Vermehrung (1) nach einem der Ansprüche 1 bis 10, wobei die Hauptgefäßeinheit (4) mit einem Gasaustauschanschluss vorgesehen ist.

12. Gefäß zur Zellisolation, Kultivierung und Vermehrung (1) nach einem der Ansprüche 1 bis 11, wobei die Hauptgefäßeinheit (4) mit einem Mediumaustauschanschluss vorgesehen ist, durch den ein Medium in das Hauptgefäß eingeleitet oder daraus ausgegeben werden kann.

13. Gefäß zur Zellisolation, Kultivierung und Vermehrung (1) nach einem der Ansprüche 1 bis 12, wobei die Zelltrenneinheit (2) ein behälterartiger Strukturkörper ist, der überwiegend aus einer Zelltrennmembran (11) und einer Membranhaltestruktur (13) zusammengesetzt ist, und ein Umfangsbereich einer Bodenfläche eines Öffnungsbereichs der Membranhaltestruktur (13), die von der Zelltrennmembran (11) bedeckt ist, eine Form hat, die eine Neigung nach oben in Richtung des Öffnungsbereichs hat.

14. Gefäß zur Zellisolation, Kultivierung und Vermehrung (1) nach einem der Ansprüche 1 bis 13, das aufweist:

(G) eine oder mehrere Zellvermehrungseinheiten, bei denen eine Vielzahl von Zellvermehrungsprozessen nach einem primären Kultivierungsprozess durchgeführt werden können;
wobei (g - 1) die Zellvermehrungseinheit ein behälterartiger Strukturkörper ist; (g-2) die Zellvermehrungseinheit eine Kulturoberfläche zur Zellvermehrung aufweist, auf der die Zellen, die aus der Kultureinheit für einen vorhergehenden Schritt freigesetzt wurden, kultiviert werden können, wobei die Fläche der Kulturoberfläche zur Zellvermehrung größer ist als die der Kulturoberfläche der Kultureinheit für den vorhergehenden Schritt;
(g-3) die Zellvermehrungseinheit eine Struktur hat, bei der die Zelltrenneinheit (2) und alle von der/den Kultureinheit(en) für den/die vorhergehenden Schritt(e) in dem behälterartigen Strukturkörper der Zellvermehrungseinheit gestapelt oder aufbewahrt werden können; und
(g-4) die Zellvermehrungseinheit eine Freisetzungsstruktur hat, durch die Zellen und eine Flüssigkeit, die in dem behälterartigen Strukturkörper der Zellvermehrungseinheit gehalten werden, frei in Richtung einer Einheit freigesetzt werden, die die Kulturoberfläche für den nachfolgenden Schritt hat, wobei die Freisetzungsstruktur an einer Oberfläche des behälterartigen Strukturkörpers der Zellvermehrungseinheit vorgesehen ist.

15. Gefäß zur Zellisolation, Kultivierung und Vermehrung (1) nach einem der Ansprüche 1 bis 14, wobei die feinen Poren, die an der Zelltrennmembran (11) ausgebildet sind, eine durchschnittliche Größe von 1 bis 20 $\mu$m haben und die Zelltrennmembran (11) eine Porosität von 5 bis 50 % hat.

16. Gefäß zur Zellisolation, Kultivierung und Vermehrung (1) nach einem der Ansprüche 1 bis 15, wobei die feinen Poren, die in der Zelltrennmembran (11) ausgebildet sind, in einer vertikalen Richtung und/oder in einer im Wesentlichen vertikalen Richtung bezüglich einer Membranoberfläche offen sind.

17. Gefäß zur Zellisolation, Kultivierung und Vermehrung (1) nach einem der Ansprüche 1 bis 16, wobei ein Teil oder alle der feinen Poren, die in der Zelltrennmembran (11) ausgebildet sind, in einem Gittermuster angeordnet sind, in einem im Wesentlichen Gittermuster, einem versetzten Muster, einem im Wesentlichen versetzten Muster, einem Spiralmuster, einem im Wesentlichen Spiralmuster, einem konzentrischen Muster, einem im Wesentlichen konzentrischen Muster oder einer Kombination aus den oben erwähnten Mustern.

**18.** Gefäß zur Zellisolation, Kultur und Vermehrung (1) nach einem der Ansprüche 1 bis 17, wobei die Zelltrennmembran (11) eine Struktur hat, in der hydrophile Polymere kovalent an eine Oberfläche einer Basismaterialschicht, die aus hydrophoben Polymeren gebildet ist, angebunden sind.

**19.** Gefäß zur Zellisolation, Kultivierung und Vermehrung (1) nach einem der Ansprüche 1 bis 18,

wobei die Zelltrennmembran (11) eine Struktur hat, in der eine oder mehrere ausgewählt aus der Hydroxylgruppe, der Carboxylgruppe und einer Verbindungstruktur, die die funktionalen Gruppen aufweist, kovalent in einer Nähe der Oberfläche der Basismaterialschicht, die aus hydrophoben Polymeren gebildet ist, angebunden sind; und/oder
die Zelltrennmembran (11) eine nicht lösbare Schicht in der Nähe der Oberfläche der Basismaterialschicht hat, die aus hydrophoben Polymeren gebildet ist, wobei die nicht lösbare Schicht in einem guten Lösungsmittel für die hydrophoben Polymeren nicht lösbar ist.

**20.** Gefäß zur Zellisolation, Kultivierung und Vermehrung (1) nach einem der Ansprüche 1 bis 19, wobei eine Oberfläche der Kulturoberfläche für die Primärkultur (27) und/oder einer Oberfläche der Kulturoberfläche zur Zellvermehrung (55) mit Molekülen modifiziert werden, die Zellhaftungseigenschaften während der Kultivierung zeigen und Zellablöseeigenschaften in Reaktion auf einen Stimulus zeigen.

**21.** Gefäß zur Zellisolation, Kultivierung und Vermehrung (1) nach Anspruch 20, wobei die Moleküle, die die Zellablöseeigenschaften zeigen, auf Temperatur reagierende Polymermoleküle sind, die eine Phasenübergangstemperatur oder eine untere kritische Lösungstemperatur von 15 bis 35 °C haben.

**22.** Gefäß zur Zellisolation, Kultivierung und Vermehrung (1) nach einem der Ansprüche 1 bis 21, wobei die Kulturoberfläche für die Primärkultur (27) und/oder die Kulturoberfläche zur Zellvermehrung (55) eine Struktur haben, in der eine oder mehrere ausgewählt aus der Hydroxylgruppe, der Carboxylgruppe und einer Verbindungstruktur, die die funktionalen Gruppen aufweist, kovalent in einer Nähe der Oberfläche einer Basismaterialschicht, die aus hydrophoben Polymeren gebildet ist, angebunden sind; und/oder die Kulturoberfläche für die Primärkultur (27) und/oder die Kulturoberfläche zur Zellvermehrung (55) eine nicht lösbare Schicht in der Nähe der Oberfläche der Basismaterialschicht haben, die aus hydrophoben Polymeren gebildet ist, wobei die nicht lösbare Schicht in einem guten Lösungsmittel für die hydrophoben Polymere nicht lösbar ist.

**23.** Gefäß zur Zellisolation, Kultivierung und Vermehrung (1) nach einem der Ansprüche 1 bis 22,

wobei die Strukturkörper, die das Gefäß für die Zellisolation, Kultivierung und Vermehrung (1) bilden, eine Struktur haben, in der eine oder mehrere ausgewählt aus der Hydroxylgruppe, der Carboxylgruppe und einer Verbindungstruktur, die die funktionalen Gruppen aufweist, kovalent in der Nähe der Oberfläche der Basismaterialschicht, die aus hydrophoben Polymeren gebildet ist, angebunden sind; und/oder
die Strukturkörper, die das Gefäß für die Zellisolation, Kultivierung und Vermehrung (1) bilden, eine nicht lösbare Schicht haben, die in der Nähe der Oberfläche der Basismaterialschicht vorgesehen ist, die aus hydrophoben Polymeren gebildet ist, wobei die nicht lösbare Schicht in einem guten Lösungsmittel für die hydrophoben Polymere nicht lösbar ist.

**24.** Gefäß zur Zellisolation, Kultivierung und Vermehrung (1) nach einem der Ansprüche 1 bis 23, wobei das Gefäß zur Zellisolation, Kultivierung und Vermehrung ein Gefäß ist, um Stammzellen aktiv zu isolieren und deren Vermehrung durchzuführen.

## Revendications

**1.** Cuve pouvant être fermée hermétiquement pour l'isolement, la culture et la prolifération de cellules (1), la cuve (1) étant un corps structurel en forme de récipient dans lequel l'isolement, la culture et la prolifération de cellules cibles souhaitées peuvent être réalisés,
dans laquelle

(A) la cuve (1) comprend une unité de séparation de cellules (2), une unité de culture primaire (3) et une unité de cuve principale (4) ;
(B) l'unité de séparation de cellules (2) est un corps structurel comprenant une membrane de séparation de cellules (11) ayant des pores fins capables d'isoler des cellules en attirant ou en activant le mouvement des cellules cibles en utilisant des facteurs chimiotactiques ;

(C) l'unité de culture primaire (3) est un corps structurel en forme de récipient qui a une surface de culture pour la culture primaire (27) sur laquelle les cellules qui sont passées à travers la membrane de séparation de cellules (11) peuvent être cultivées et une structure de libération (28) à travers laquelle les cellules et un liquide qui ont été retenus à l'intérieur de l'unité de culture primaire (3) peuvent être libérés librement vers une unité ayant une surface de culture pour une étape ultérieure, la surface de culture pour la culture primaire (27) ayant une taille de 10 à 400 cm$^2$ ; et

(D) l'unité de cuve principale (4) est un corps structurel en forme de récipient qui a une surface de culture pour la prolifération de cellules (55) sur laquelle les cellules qui ont été libérées d'une unité de culture pour une étape précédente peuvent être cultivées, la surface de culture pour la prolifération de cellules (55) étant une surface de culture différente de la surface de culture pour la culture primaire (27) de l'unité de culture primaire (3), une zone de la surface de culture pour la prolifération de cellules (55) étant plus grande que celle d'une surface de culture de l'unité de culture pour l'étape précédente, le corps structurel en forme de récipient de l'unité de cuve principale (4) ayant une structure dans laquelle l'unité de séparation de cellules (2) et toute(s) l'unité/les unités de culture pour la/les étape(s) précédente(s) peuvent être stockées.

2. Cuve pour l'isolement, la culture et la prolifération de cellules (1) selon la revendication 1, dans laquelle

(b-1) l'unité de séparation de cellules (2) décrite en (B) est un corps structurel en forme de récipient ;

(b-2) l'unité de séparation de cellules (2) comprend la membrane de séparation de cellules (11) ayant des pores fins capables d'isoler les cellules, la membrane de séparation de cellules (11) étant agencée sur au moins une partie des surfaces du corps structurel en forme de récipient de l'unité de séparation de cellules (2) ;

(c-1) l'unité de culture primaire (3) décrite en (C) est un corps structurel en forme de récipient ;

(c-2) l'unité de culture primaire (3) a une structure avec laquelle l'unité de séparation de cellules (2) peut être agencée dans un état dans lequel, lorsqu'un liquide est rempli dans le corps structurel en forme de récipient de l'unité de culture primaire (3), une surface de la membrane de séparation de cellules (11) et une surface du liquide rempli sont en contact l'une avec l'autre ;

(c-3) l'unité de culture primaire (3) a la surface de culture pour la culture primaire (27) sur laquelle les cellules qui sont passées à travers la membrane de séparation de cellules (11) peuvent être cultivées, la surface de culture pour la culture primaire (27) étant agencée sur au moins une partie des surfaces du corps structurel en forme de récipient de l'unité de culture primaire (3) ;

(c-4) l'unité de culture primaire (3) possède la structure de libération (28) à travers laquelle les cellules et le liquide qui ont été retenus à l'intérieur du corps structurel en forme de récipient de l'unité de culture primaire (3) peuvent être libérés librement vers l'unité ayant la surface de culture pour l'étape suivante, la structure de libération (28) étant agencée à une surface du corps structurel en forme de récipient de l'unité de culture primaire (3) ;

(d-1) l'unité de cuve principale (4) décrite en (D) est un corps structurel en forme de récipient composé principalement d'une cuve principale (41) et d'une partie de couvercle principale (56) ;

(d-2) l'unité de cuve principale (4) a la surface de culture pour la prolifération de cellules (55) sur laquelle les cellules qui ont été libérées de l'unité de culture pour l'étape précédente peuvent être cultivées, la zone de la surface de culture pour la prolifération de cellules (55) étant plus grande que celle de la surface de culture de l'unité de culture pour l'étape précédente ;

(d-3) l'unité de cuve principale (4) a une structure avec laquelle l'unité de séparation de cellules (2) et toute(s) l'unité/les unités de culture pour la/les étape(s) précédente(s) peuvent être stockées dans le corps structurel en forme de récipient de l'unité de cuve principale (4) ; et

(d-4) la partie de couvercle principale (56) est un corps structurel en forme de couvercle avec lequel l'ensemble de la cuve peut être fermé hermétiquement.

3. Cuve pour l'isolement, la culture et la prolifération de cellules (1) selon la revendication 1 ou 2, dans laquelle l'unité de séparation de cellules (2) est une unité pour isoler les cellules cibles souhaitées dans le corps structurel en forme de récipient de l'unité de culture primaire (3) à travers la membrane de séparation de cellules (11).

4. Cuve pour l'isolement, la culture et la prolifération de cellules (1) selon l'une quelconque des revendications 1 à 3, dans laquelle, lorsqu'une zone de la surface de culture de l'unité de culture pour l'étape précédente est considérée égale à 1, un rapport d'une zone de la surface de culture pour la prolifération de cellules (55) est compris dans une plage allant de plus de 1 à au plus 20.

5. Cuve pour l'isolement, la culture et la prolifération de cellules (1) selon l'une quelconque des revendications 1 à 4, dans laquelle, dans la cuve pour l'isolement, la culture et la prolifération de cellules,

(E)(e-1) au moins une partie de toutes les surfaces de culture incluses dans la cuve pour l'isolement, la culture et la prolifération de cellules (1) et au moins une partie des structures de cuve opposées ou sensiblement opposées à la surface de culture pour la prolifération de cellules (55) de l'unité de cuve principale (4) sont constituées d'un matériau transmettant la lumière ; et

(e-2) les surfaces de culture à l'intérieur de la cuve (1) peuvent être observées à travers des parties ayant des propriétés de transmission de la lumière sans ouvrir l'unité de cuve principale (4).

6. Cuve pour l'isolement, la culture et la prolifération de cellules (1) selon l'une quelconque des revendications 1 à 5, dans laquelle l'unité de culture primaire (3) est un corps structurel en forme de récipient composé de deux éléments ou plus, et a une structure avec laquelle la structure de libération (28) peut être ajustée pour s'ouvrir lorsque les éléments du corps structurel en forme de récipient de l'unité de culture primaire (3) sont détachés ou que leurs positions sont modifiées.

7. Cuve pour l'isolement, la culture et la prolifération de cellules (1) selon l'une quelconque des revendications 1 à 6, dans laquelle l'unité de culture primaire (3) a une structure avec laquelle la structure de libération (28) peut être ajustée pour s'ouvrir en réponse à une opération depuis l'extérieur de l'unité de cuve principale (4).

8. Cuve pour l'isolement, la culture et la prolifération de cellules (1) selon l'une quelconque des revendications 2 à 7, dans laquelle l'unité de culture primaire (3) a une structure avec laquelle la structure de libération (28) peut être ajustée pour s'ouvrir en réponse à l'actionnement de la partie de couvercle principale.

9. Cuve pour l'isolement, la culture et la prolifération de cellules (1) selon l'une quelconque des revendications 2 à 8, dans laquelle, dans la cuve pour l'isolement, la culture et la prolifération de cellules,

(F)(f-1) une structure (60) à travers laquelle un élément tubulaire creux peut être inséré dans ou retiré de la cuve principale sans ouvrir l'unité de cuve principale (4) est agencée au niveau d'une surface de paroi de la cuve principale ou de la partie de couvercle principale de l'unité de cuve principale (4) ;

(f-2) la structure (60) est disposée à une position à laquelle le liquide rempli dans l'unité de culture primaire (3) peut être aspiré à travers l'élément tubulaire creux inséré ; et

(f-3) la structure (60) est principalement composée d'un élément élastique qui peut assurer l'étanchéité à l'air à la fois dans un état dans lequel l'élément tubulaire creux n'est pas inséré et dans un état dans lequel l'élément tubulaire creux est inséré.

10. Cuve pour l'isolement, la culture et la prolifération de cellules (1) selon l'une quelconque des revendications 2 à 9, dans laquelle l'unité de séparation de cellules (2) a une structure avec laquelle une position de la membrane de séparation de cellules (11) peut être ajustée en réponse à l'actionnement de la partie de couvercle principale.

11. Cuve pour l'isolement, la culture et la prolifération de cellules (1) selon l'une quelconque des revendications 1 à 10, dans laquelle l'unité de cuve principale (4) est pourvue d'un orifice d'échange de gaz.

12. Cuve pour l'isolement, la culture et la prolifération de cellules (1) selon l'une quelconque des revendications 1 à 11, dans laquelle l'unité de cuve principale (4) est pourvue d'un orifice d'échange de milieu à travers lequel un milieu peut être introduit dans la cuve principale ou évacué de celle-ci.

13. Cuve pour l'isolement, la culture et la prolifération de cellules (1) selon l'une quelconque des revendications 1 à 12, dans laquelle l'unité de séparation de cellules (2) est un corps structurel en forme de récipient qui est principalement composé de la membrane de séparation de cellules (11) et d'une structure de maintien de membrane (13), et une partie périphérique d'une surface inférieure d'une partie d'ouverture de la structure de maintien de membrane (13) recouverte par la membrane de séparation de cellules (11) a une forme ayant une inclinaison vers le haut vers la partie d'ouverture.

14. Cuve pour l'isolement, la culture et la prolifération de cellules (1) selon l'une quelconque des revendications 1 à 13, comprenant :

(G) une ou plusieurs unités de prolifération de cellules avec lesquelles une pluralité de processus de prolifération de cellules peuvent être réalisés après un processus de culture primaire ;

dans laquelle (g-1) l'unité de prolifération de cellules est un corps structurel en forme de récipient ;

(g-2) l'unité de prolifération de cellules comprend une surface de culture pour la prolifération de cellules sur laquelle les cellules qui ont été libérées d'une unité de culture pour une étape précédente peuvent être cultivées, une zone de la surface de culture pour la prolifération de cellules étant plus grande que celle d'une surface de culture de l'unité de culture pour l'étape précédente ;

(g-3) l'unité de prolifération de cellules a une structure avec laquelle l'unité de séparation de cellules (2) et toute(s) l'unité/les unités de culture pour la/les étape(s) précédentes peuvent être empilées ou stockées dans le corps structurel en forme de récipient de l'unité de prolifération de cellules ; et

(g-4) l'unité de prolifération de cellules possède une structure de libération à travers laquelle les cellules et un liquide qui ont été retenus à l'intérieur du corps structurel en forme de récipient de l'unité de prolifération de cellules peuvent être libérés librement vers une unité ayant une surface de culture pour une étape ultérieure, la structure de libération étant agencée à une surface du corps structurel en forme de récipient de l'unité de prolifération de cellules.

15. Cuve pour l'isolement, la culture et la prolifération de cellules (1) selon l'une quelconque des revendications 1 à 14, dans laquelle les pores fins formés dans la membrane de séparation de cellules (11) ont une taille moyenne de 1 à 20 $\mu$m, et la membrane de séparation de cellules (11) a une porosité de 5 à 50 %.

16. Cuve pour l'isolement, la culture et la prolifération de cellules (1) selon l'une quelconque des revendications 1 à 15, dans laquelle les pores fins formés dans la membrane de séparation de cellules (11) sont ouverts dans une direction verticale et/ou dans une direction sensiblement verticale par rapport à une surface de la membrane.

17. Cuve pour l'isolement, la culture et la prolifération de cellules (1) selon l'une quelconque des revendications 1 à 16, dans laquelle une partie ou la totalité des pores fins formés dans la membrane de séparation de cellules (11) sont disposés selon un motif en treillis, un motif sensiblement en treillis, un motif décalé, un motif sensiblement décalé, un motif en spirale, un motif sensiblement en spirale, un motif concentrique, un motif sensiblement concentrique, ou une combinaison des motifs précédents.

18. Cuve pour l'isolement, la culture et la prolifération de cellules (1) selon l'une quelconque des revendications 1 à 17, dans laquelle la membrane de séparation de cellules (11) a une structure dans laquelle des polymères hydrophiles sont liés de manière covalente à une surface d'une couche de matériau de base constituée de polymères hydrophobes.

19. Cuve pour l'isolement, la culture et la prolifération de cellules (1) selon l'une quelconque des revendications 1 à 18,

dans laquelle la membrane de séparation de cellules (11) a une structure dans laquelle un ou plusieurs groupes choisis parmi le groupe hydroxyle, le groupe carboxyle et une structure composée comprenant lesdits groupes fonctionnels sont liés de manière covalente à un voisinage de la surface de la couche de matériau de base constituée de polymères hydrophobes ; et/ou

la membrane de séparation de cellules (11) présente une couche insoluble au voisinage de la surface de la couche de matériau de base constituée de polymères hydrophobes, la couche insoluble étant insoluble dans un bon solvant pour les polymères hydrophobes.

20. Cuve pour l'isolement, la culture et la prolifération de cellules (1) selon l'une quelconque des revendications 1 à 19, dans laquelle une surface de la surface de culture pour la culture primaire (27) et/ou une surface de la surface de culture pour la prolifération de cellules (55) sont modifiées avec des molécules qui présentent des propriétés d'adhésion des cellules pendant la culture et présentent des propriétés de détachement des cellules en réponse à un stimulus.

21. Cuve pour l'isolement, la culture et la prolifération de cellules (1) selon la revendication 20, dans laquelle les molécules qui présentent des propriétés de détachement des cellules sont des molécules polymères sensibles à la température ayant une température de transition de phase, ou une température critique inférieure de solution, de 15 à 35 °C.

22. Cuve pour l'isolement, la culture et la prolifération de cellules (1) selon l'une quelconque des revendications 1 à 21,

dans laquelle la surface de culture pour la culture primaire (27) et/ou la surface de culture pour la prolifération de cellules (55) ont une structure dans laquelle un ou plusieurs groupes choisis parmi le groupe hydroxyle, le groupe carboxyle et une structure composée comprenant lesdits groupes fonctionnels sont liés de manière covalente à un voisinage d'une surface d'une couche de matériau de base constituée de polymères hydrophobes ; et/ou
la surface de culture pour la culture primaire (27) et/ou la surface de culture pour la prolifération de cellules (55) ont une couche insoluble au voisinage de la surface de la couche de matériau de base constituée de polymères hydrophobes, la couche insoluble étant insoluble dans un bon solvant pour les polymères hydrophobes.

23. Cuve pour l'isolement, la culture et la prolifération de cellules (1) selon l'une quelconque des revendications 1 à 22,

dans laquelle les corps structurels constituant la cuve pour l'isolement, la culture et la prolifération de cellules (1) ont une structure dans laquelle un ou plusieurs groupes choisis parmi le groupe hydroxyle, le groupe carboxyle et une structure composée comprenant lesdits groupes fonctionnels sont liés de manière covalente à un voisinage d'une surface d'une couche de matériau de base constituée de polymères hydrophobes ; et/ou
les corps structurels constituant la cuve pour l'isolement, la culture et la prolifération de cellules (1) ont une couche insoluble agencée au voisinage de la surface de la couche de matériau de base constituée de polymères hydrophobes, la couche insoluble étant insoluble dans un bon solvant pour les polymères hydrophobes.

24. Cuve pour l'isolement, la culture et la prolifération de cellules (1) selon l'une quelconque des revendications 1 à 23, dans laquelle la cuve pour l'isolement, la culture et la prolifération de cellules est une cuve pour isoler activement des cellules souches et réaliser leur prolifération.

Fig. 1

*Fig. 2*

Fig. 3

Fig. 4

*Fig. 5*

Fig. 6

56

59

1

30

33

32

Protrusions
disengaged

26

28

48

45

47

42

55

54

43

44

**Fig. 7**

**Fig. 8**

**Fig. 9**

**Fig. 10**

*Fig. 11A*

*Fig. 11B*

*Fig. 11C*

*Fig. 11D*

*Fig. 12*

## Fig. 13

## Fig. 14

# Fig. 15

*Fig. 16*

*Fig. 17*

Sample 4-1
Surface-modified plate

Sample 4-2
Non-modified plate

Before
low-temperature
treatment

Low-temperature
treatment
for 30 min

Low-temperature
treatment
for 60 min

Low-temperature
treatment
for 60 min
+
pipetting

Fig. 18

Fig. 19

*Fig. 20A*

62

63a

63b

63c

63d

*Fig. 20B*

64a

64b

56

62

42

66

65

Fig. 21A

Fig. 21B

Fig. 21C

*Fig. 22*

Sample 8-2
Vacuum plasma treatment

Control

24 hr

48 hr

60 hr

## REFERENCES CITED IN THE DESCRIPTION

**Patent documents cited in the description**

- WO 2012133803 A
- US 2014178992 A1
- US 2012100110 A1
- WO 2015121302 A1

- WO 2012133803 A1
- WO 2014171365 A
- WO 2012058637 A
- JP 3641301 B

**Non-patent literature cited in the description**

- **HORIBE et al.** Isolation of a Stable Subpopulation of Mobilized Dental Pulp Stem Cells (MDPSCs) with High Proliferation, Migration, and Regeneration Potential Is Independent of Age. *PLoS ONE,* 2014, vol. 9 (5

- **MURAKAMI et al.** The use of granulocyte-colony stimulating factor induced mobilization for isolation of dental pulp stem cells with high regenerative potential. *Biomaterials,* December 2013, vol. 34 (36), 9036-9047
- **HIGUCHI et al.** A hybrid-membrane migration method to isolate high-purity adipose-derived stem cells from fat tissues. *Sci Rep,* 2015, vol. 5, 10217